# EUROPEAN PATENT SPECIFICATION

(11) **EP 1 678 497 B1**
(45) Date of publication and mention of the grant of the patent: **11.05.2011**
(21) Application number: 04796496.0
(22) Date of filing: 26.10.2004
(51) Int. Cl.: G01N 33/50, C12Q 1/00, C12Q 1/68, C12N 15/11, C12N 15/00, C07K 14/435

(54) **METHODS AND COMPOSITIONS FOR MODULATING APOPTOSIS**
VERFAHREN UND VERBINDUNGEN ZUR MODULIERUNG VON APOPTOSIS
PROCEDES ET COMPOSITIONS POUR LA MODULATION DE L'APOPTOSE

(30) Priority: 27.10.2003 US 514661 P
(43) Date of publication of application: 12.07.2006
(73) Proprietor: UNIVERSITY OF SOUTHERN CALIFORNIA, Los Angeles, CA 90007-4344 (US)
(72) Inventor: LEE, Amy, S., San Marino, CA 91108 (US)
(74) Representative: Bates, Philip Ian
(86) International application number: PCT/US2004/035575
(87) International publication number: WO 2005/045428

(56) References cited:
- EP-A- 0 927 757
- RAO R V ET AL: "Coupling endoplasmic reticulum stress to the cell death program: role of the ER chaperone GRP78" FEBS LETTERS, ELSEVIER SCIENCE PUBLISHERS, AMSTERDAM, NL, vol. 514, no. 2-3, 13 March 2002 (2002-03-13), pages 122-128, XP004347683 ISSN: 0014-5793
- JAMORA C ET AL: "INHIBITION OF TUMOR PROGRESSION BY SUPPRESSION OF STRESS PROTEIN GRP78/BIP INDUCTION IN FIBROSARCOMA B/C10ME" PROCEEDINGS OF THE NATIONAL ACADEMY OF SCIENCES OF USA, NATIONAL ACADEMY OF SCIENCE. WASHINGTON, US, vol. 93, July 1996 (1996-07), pages 7690-7694, XP002066166 ISSN: 0027-8424
- SUGAWARA S ET AL: "SUPPRESSION OF STRESS PROTEIN GRP78 INDUCTION IN TUMOR B0C10ME ELIMINATES RESISTANCE TO CELL MEDIATED CYTOTOXICITY" CANCER RESEARCH, AMERICAN ASSOCIATION FOR CANCER RESEARCH, BALTIMORE, MD, US, vol. 53, 15 December 1993 (1993-12-15), pages 6001-6005, XP002066167 ISSN: 0008-5472
- DATABASE BIOSIS [Online] BIOSCIENCES INFORMATION SERVICE, PHILADELPHIA, PA, US; August 2003 (2003-08), BANDO YOSHIO ET AL: "GRP94 (94 kDa glucose-regulated protein) suppresses ischemic neuronal cell death against ischemia/reperfusion injury." XP002325342 Database accession no. PREV200300503335 & EUROPEAN JOURNAL OF NEUROSCIENCE, vol. 18, no. 4, August 2003 (2003-08), pages 829-840, ISSN: 0953-816X

### SEQUENCE LISTING

<110> University of Southern California
<120> METHODS AND COMPOSITIONS FOR MODULATING APOPTOSIS
<130> 06666-174WO1
<150> US 60/514,661
   <151> 2003-10-27
<160> 17
<170> FastSEQ for Windows version 4.0
<210> 1
   <211> 3925
   <212> DNA
   <213> Homo sapiens
<220>
   <221> CDS
   <222> (205) ... (2166)
<400> 1
   acagcacaga cagattgacc tattggggtg tttcgcgagt gtgagaggga agcgccgcgg     60
   cctgtatttc tagacctgcc cttcgcctgg ttcgtggcgc cttgtgaccc cgggcccctg    120
   ccgcctgcaa gtcggaaatt gcgctgtgct cctgtgctac ggcctgtggc tggactgcct    180
   gctgctgccc aactggctgg caag atg aag ctc tcc ctg gtg gcc gcg atg       231
                              Met Lys Leu Ser Leu Val Ala Ala Met
                               1               5
   ctg ctg ctg ctc agc gcg gcg cgg gcc gag gag gag gac aag aag gag      279
   Leu Leu Leu Leu Ser Ala Ala Arg Ala Glu Glu Glu Asp Lys Lys Glu
    10                  15                  20                  25
   gac gtg ggc acg gtg gtc ggc atc gac ctg ggg acc acc tac tcc tgc      327
   Asp Val Gly Thr Val Val Gly Ile Asp Leu Gly Thr Thr Tyr Ser Cys
                    30                  35                  40
   gtc ggc gtg ttc aag aac ggc cgc gtg gag atc atc gcc aac gat cag      375
   Val Gly Val Phe Lys Asn Gly Arg Val Glu Ile Ile Ala Asn Asp Gln
                45                  50                  55
   ggc aac cgc atc acg ccg tcc tat gtc gcc ttc act cct gaa ggg gaa      423
   Gly Asn Arg Ile Thr Pro Ser Tyr Val Ala Phe Thr Pro Glu Gly Glu
            60                  65                  70
   cgt ctg att ggc gat gcc gcc aag aac cag ctc acc tcc aac ccc gag      471
   Arg Leu Ile Gly Asp Ala Ala Lys Asn Gln Leu Thr Ser Asn Pro Glu
        75                  80                  85
   aac acg gtc ttt gac gcc aag cgg ctc atc ggc cgc acg tgg aat gac      519
   Asn Thr Val Phe Asp Ala Lys Arg Leu Ile Gly Arg Thr Trp Asn Asp
    90                  95                 100                 105
   ccg tct gtg cag cag gac atc aag ttc ttg ccg ttc aag gtg gtt gaa      567
   Pro Ser Val Gln Gln Asp Ile Lys Phe Leu Pro Phe Lys Val Val Glu
                   110                 115                 120
   aag aaa act aaa cca tac att caa gtt gat att gga ggt ggg caa aca      615
   Lys Lys Thr Lys Pro Tyr Ile Gln Val Asp Ile Gly Gly Gly Gln Thr
               125                 130                 135
   aag aca ttt gct cct gaa gaa att tct gcc atg gtt ctc act aaa atg      663
   Lys Thr Phe Ala Pro Glu Glu Ile Ser Ala Met Val Leu Thr Lys Met
           140                 145                 150
   aaa gaa acc gct gag gct tat ttg gga aag aag gtt acc cat gca gtt      711
   Lys Glu Thr Ala Glu Ala Tyr Leu Gly Lys Lys Val Thr His Ala Val
       155                 160                 165
   gtt act gta cca gcc tat ttt aat gat gcc caa cgc caa gca acc aaa      759
   Val Thr Val Pro Ala Tyr Phe Asn Asp Ala Gln Arg Gln Ala Thr Lys
   170                 175                 180                 185
   gac gct gga act att gct ggc cta aat gtt atg agg atc atc aac gag      807
   Asp Ala Gly Thr Ile Ala Gly Leu Asn Val Met Arg Ile Ile Asn Glu
                   190                 195                 200
   cct acg gca gct gct att gct tat ggc ctg gat aag agg gag ggg gag      855
   Pro Thr Ala Ala Ala Ile Ala Tyr Gly Leu Asp Lys Arg Glu Gly Glu
               205                 210                 215
   aag aac atc ctg gtg ttt gac ctg ggt ggc gga acc ttc gat gtg tct      903
   Lys Asn Ile Leu Val Phe Asp Leu Gly Gly Gly Thr Phe Asp Val Ser
           220                 225                 230
   ctt ctc acc att gac aat ggt gtc ttc gaa gtt gtg gcc act aat gga      951
   Leu Leu Thr Ile Asp Asn Gly Val Phe Glu Val Val Ala Thr Asn Gly
       235                 240                 245
   gat act cat ctg ggt gga gaa gac ttt gac cag cgt gtc atg gaa cac      999
   Asp Thr His Leu Gly Gly Glu Asp Phe Asp Gln Arg Val Met Glu His
   250                 255                 260                 265
   ttc atc aaa ctg tac aaa aag aag acg ggc aaa gat gtc agg aaa gac     1047
   Phe Ile Lys Leu Tyr Lys Lys Lys Thr Gly Lys Asp Val Arg Lys Asp
                   270                 275                 280
   aat aga gct gtg cag aaa ctc cgg cgc gag gta gaa aag gcc aaa cgg     1095
   Asn Arg Ala Val Gln Lys Leu Arg Arg Glu Val Glu Lys Ala Lys Arg
               285                 290                 295
   gcc ctg tct tct cag cat caa gca aga att gaa att gag tcc ttc tat     1143
   Ala Leu Ser Ser Gln His Gln Ala Arg Ile Glu Ile Glu Ser Phe Tyr
           300                 305                 310
   gaa gga gaa gac ttt tct gag acc ctg act cgg gcc aaa ttt gaa gag     1191
   Glu Gly Glu Asp Phe Ser Glu Thr Leu Thr Arg Ala Lys Phe Glu Glu
       315                 320                 325
   ctc aac atg gat ctg ttc cgg tct act atg aag ccc gtc cag aaa gtg     1239
   Leu Asn Met Asp Leu Phe Arg Ser Thr Met Lys Pro Val Gln Lys Val
   330                 335                 340                 345
   ttg gaa gat tct gat ttg aag aag tct gat att gat gaa att gtt ctt     1287
   Leu Glu Asp Ser Asp Leu Lys Lys Ser Asp Ile Asp Glu Ile Val Leu
                   350                 355                 360
   gtt ggt ggc tcg act cga att cca aag att cag caa ctg gtt aaa gag      1335
   Val Gly Gly Ser Thr Arg Ile Pro Lys Ile Gln Gln Leu Val Lys Glu
               365                 370                 375
   ttc ttc aat ggc aag gaa cca tcc cgt ggc ata aac cca gat gaa gct      1383
   Phe Phe Asn Gly Lys Glu Pro Ser Arg Gly Ile Asn Pro Asp Glu Ala
           380                 385                 390
   gta gcg tat ggt gct gct gtc cag gct ggt gtg ctc tct ggt gat caa      1431
   Val Ala Tyr Gly Ala Ala Val Gln Ala Gly Val Leu Ser Gly Asp Gln
       395                 400                 405
   gat aca ggt gac ctg gta ctg ctt gat gta tgt ccc ctt aca ctt ggt      1479
   Asp Thr Gly Asp Leu Val Leu Leu Asp Val Cys Pro Leu Thr Leu Gly
   410                 415                 420                 425
   att gaa act gtg gga ggt gtc atg acc aaa ctg att cca agg aac aca      1527
   Ile Glu Thr Val Gly Gly Val Met Thr Lys Leu Ile Pro Arg Asn Thr
                   430                 435                 440
   gtg gtg cct acc aag aag tct cag atc ttt tct aca gct tct gat aat      1575
   Val Val Pro Thr Lys Lys Ser Gln Ile Phe Ser Thr Ala Ser Asp Asn
               445                 450                 455
   caa cca act gtt aca atc aag gtc tat gaa ggt gaa aga ccc ctg aca      1623
   Gln Pro Thr Val Thr Ile Lys Val Tyr Glu Gly Glu Arg Pro Leu Thr
           460                 465                 470
   aaa gac aat cat ctt ctg ggt aca ttt gat ctg act gga att cct cct      1671
   Lys Asp Asn His Leu Leu Gly Thr Phe Asp Leu Thr Gly Ile Pro Pro
       475                 480                 485
   gct cct cgt ggg gtc cca cag att gaa gtc acc ttt gag ata gat gtg      1719
   Ala Pro Arg Gly Val Pro Gln Ile Glu Val Thr Phe Glu Ile Asp Val
   490                 495                 500                 505
   aat ggt att ctt cga gtg aca gct gaa gac aag ggt aca ggg aac aaa      1767
   Asn Gly Ile Leu Arg Val Thr Ala Glu Asp Lys Gly Thr Gly Asn Lys
                   510                 515                 520
   aat aag atc aca atc acc aat gac cag aat cgc ctg aca cct gaa gaa      1815
   Asn Lys Ile Thr Ile Thr Asn Asp Gln Asn Arg Leu Thr Pro Glu Glu
               525                 530                 535
   atc gaa agg atg gtt aat gat gct gag aag ttt gct gag gaa gac aaa      1863
   Ile Glu Arg Met Val Asn Asp Ala Glu Lys Phe Ala Glu Glu Asp Lys
           540                 545                 550
   aag ctc aag gag cgc att gat act aga aat gag ttg gaa agc tat gcc      1911
   Lys Leu Lys Glu Arg Ile Asp Thr Arg Asn Glu Leu Glu Ser Tyr Ala
       555                 560                 565
   tat tct cta aag aat cag att gga gat aaa gaa aag ctg gga ggt aaa      1959
   Tyr Ser Leu Lys Asn Gln Ile Gly Asp Lys Glu Lys Leu Gly Gly Lys
   570                 575                 580                 585
   ctt tcc tct gaa gat aag gag acc atg gaa aaa gct gta gaa gaa aag      2007
   Leu Ser Ser Glu Asp Lys Glu Thr Met Glu Lys Ala Val Glu Glu Lys
                   590                 595                 600
   att gaa tgg ctg gaa agc cac caa gat gct gac att gaa gac ttc aaa      2055
   Ile Glu Trp Leu Glu Ser His Gln Asp Ala Asp Ile Glu Asp Phe Lys
               605                 610                 615
   gct aag aag aag gaa ctg gaa gaa att gtt caa cca att atc agc aaa      2103
   Ala Lys Lys Lys Glu Leu Glu Glu Ile Val Gln Pro Ile Ile Ser Lys
           620                 625                 630
   ctc tat gga agt gca ggc cct ccc cca act ggt gaa gag gat aca gca      2151
   Leu Tyr Gly Ser Ala Gly Pro Pro Pro Thr Gly Glu Glu Asp Thr Ala
       635                 640                 645
   gaa aaa gat gag ttg tagacactga tctgctagtg ctgtaatatt gtaaatactg      2206
   Glu Lys Asp Glu Leu
   650
   gactcaggaa cttttgttag gaaaaaattg aaagaactta agtctcgaat gtaattggaa    2266
   tcttcacctc agagtggagt tgaaactgct atagcctaag cggctgttta ctgcttttca    2326
   ttagcagttg ctcacatgtc tttgggtggg ggggagaaga agaattggcc atcttaaaaa    2386
   gcaggtaaaa aacctgggtt agggtgtgtg ttcaccttca aaatgttcta tttaacaact    2446
   gggtcatgtg catctggtgt aggaagtttt ttctaccata agtgacacca ataaatgttt    2506
   gttatttaca ctggtctaat gtttgtgaga agcttctaat tagatcaatt acttatttta    2566
   ggaaatttaa gactagatac tcgtgtgtgg ggtgagggga gggagtattt ggtatgttgg    2626
   gataaggaaa cacttctatt taatgcttcc agggattttt tttttttttt tttaaccctc     2686
   ctgggcccaa gtgatccttc cacctcagtc tcccagctaa ttgagaccac aggcttgtta    2746
   ccaccatgct cggcttttgc attaatctaa gaaaagggga gagaagttaa tccacatctt    2806
   tactcaggca aggggcattt cacagtgccc aagagtgggg ttttcttgaa catacttggt    2866
   ttcctatttc cccttatctt tctaaaactg cctttctggt ggcttttttt aaaattatta    2926
   ctaatgatgc ttttatagct gcttggattc tctgagaaat gatggggagt gagtgatcac    2986
   tggtattaac tttatacact tggatttcat ttgtaacttt aggatgtaaa ggtatattgt    3046
   gaaccctagc tgtgtcagaa tctccatccc tgaaatttct cattagtggt actggggtgg    3106
   gatcttggat ggtgacattg aaactacact aaatcccctc actatgaatg ggttgttaaa    3166
   ggcaatggtt tgtgtcaaaa ctggtttagg attacttaga ttgtgttcct gaagaaaaga    3226
   gtccaggtaa atggtatgat caataaagga caggctggtg ctaacataaa atccaatatt    3286
   gtaatcctag cactttggga ggccaaggcg ggtggatcac aaggtcaaga gatagagacc    3346
   atctttgcca acatggtgaa actccatctc tactgaaaat acaaaaatta gctgggcgtg    3406
   gtagtgcaag ctgaaggctg aggcaggaga atcactcgaa cccgggaggc agaggttgca    3466
   gtgagccgag atcacaccac tgtactccag cccggcactc cagcctggcg acaagagtga    3526
   gactccacct caaaaaaaaa aaaaagaatc caatactgcc caaggatagg tattttatag    3586
   atgggcaact ggctgaaagg ttaattctct agggctagta gaactggatc ccaacaccaa    3646
   actcttaatt agacctaggc ctcagctgca ctgcccgaaa agcatttggg cagaccctga    3706
   gcagaatact ggtctcaggc caagcccaat acagccatta aagatgacct acagtgctgt    3766
   gtaccctggg gcaatagggt taaatggtag ttagcaacta gggctagtct tcccttacct    3826
   caaaggctct cactaccgtg gaccacctag tctgtaactc tttctgagga gctgttactg    3886
   aatattaaaa agatagactt caaaaaaaaa aaaaaaaaa                           3925
<210> 2
   <211> 654
   <212> PRT
   <213> Homo sapiens
<400> 2
   Met Lys Leu Ser Leu Val Ala Ala Met Leu Leu Leu Leu Ser Ala Ala
    1               5                  10                  15
   Arg Ala Glu Glu Glu Asp Lys Lys Glu Asp Val Gly Thr Val Val Gly
               20                  25                  30
   Ile Asp Leu Gly Thr Thr Tyr Ser Cys Val Gly Val Phe Lys Asn Gly
           35                  40                  45
   Arg Val Glu Ile Ile Ala Asn Asp Gln Gly Asn Arg Ile Thr Pro Ser
       50                  55                  60
   Tyr Val Ala Phe Thr Pro Glu Gly Glu Arg Leu Ile Gly Asp Ala Ala
   65                  70                  75                  80
   Lys Asn Gln Leu Thr Ser Asn Pro Glu Asn Thr Val Phe Asp Ala Lys
                   85                  90                  95
   Arg Leu Ile Gly Arg Thr Trp Asn Asp Pro Ser Val Gln Gln Asp Ile
               100                 105                 110
   Lys Phe Leu Pro Phe Lys Val Val Glu Lys Lys Thr Lys Pro Tyr Ile
           115                 120                 125
   Gln Val Asp Ile Gly Gly Gly Gln Thr Lys Thr Phe Ala Pro Glu Glu
       130                 135                 140
   Ile Ser Ala Met Val Leu Thr Lys Met Lys Glu Thr Ala Glu Ala Tyr
   145                 150                 155                 160
   Leu Gly Lys Lys Val Thr His Ala Val Val Thr Val Pro Ala Tyr Phe
                   165                 170                 175
   Asn Asp Ala Gln Arg Gln Ala Thr Lys Asp Ala Gly Thr Ile Ala Gly
               180                 185                 190
   Leu Asn Val Met Arg Ile Ile Asn Glu Pro Thr Ala Ala Ala Ile Ala
           195                 200                 205
   Tyr Gly Leu Asp Lys Arg Glu Gly Glu Lys Asn Ile Leu Val Phe Asp
       210                 215                 220
   Leu Gly Gly Gly Thr Phe Asp Val Ser Leu Leu Thr Ile Asp Asn Gly
   225                 230                 235                 240
   Val Phe Glu Val Val Ala Thr Asn Gly Asp Thr His Leu Gly Gly Glu
                   245                 250                 255
   Asp Phe Asp Gln Arg Val Met Glu His Phe Ile Lys Leu Tyr Lys Lys
               260                 265                 270
   Lys Thr Gly Lys Asp Val Arg Lys Asp Asn Arg Ala Val Gln Lys Leu
           275                 280                 285
   Arg Arg Glu Val Glu Lys Ala Lys Arg Ala Leu Ser Ser Gln His Gln
       290                 295                 300
   Ala Arg Ile Glu Ile Glu Ser Phe Tyr Glu Gly Glu Asp Phe Ser Glu
   305                 310                 315                 320
   Thr Leu Thr Arg Ala Lys Phe Glu Glu Leu Asn Met Asp Leu Phe Arg
                   325                 330                 335
   Ser Thr Met Lys Pro Val Gln Lys Val Leu Glu Asp Ser Asp Leu Lys
               340                 345                 350
   Lys Ser Asp Ile Asp Glu Ile Val Leu Val Gly Gly Ser Thr Arg Ile
           355                 360                 365
   Pro Lys Ile Gln Gln Leu Val Lys Glu Phe Phe Asn Gly Lys Glu Pro
       370                 375                 380
   Ser Arg Gly Ile Asn Pro Asp Glu Ala Val Ala Tyr Gly Ala Ala Val
   385                 390                 395                 400
   Gln Ala Gly Val Leu Ser Gly Asp Gln Asp Thr Gly Asp Leu Val Leu
                   405                 410                 415
   Leu Asp Val Cys Pro Leu Thr Leu Gly Ile Glu Thr Val Gly Gly Val
               420                 425                 430
   Met Thr Lys Leu Ile Pro Arg Asn Thr Val Val Pro Thr Lys Lys Ser
           435                 440                 445
   Gln Ile Phe Ser Thr Ala Ser Asp Asn Gln Pro Thr Val Thr Ile Lys
       450                 455                 460
   Val Tyr Glu Gly Glu Arg Pro Leu Thr Lys Asp Asn His Leu Leu Gly
   465                 470                 475                 480
   Thr Phe Asp Leu Thr Gly Ile Pro Pro Ala Pro Arg Gly Val Pro Gln
                   485                 490                 495
   Ile Glu Val Thr Phe Glu Ile Asp Val Asn Gly Ile Leu Arg Val Thr
               500                 505                 510
   Ala Glu Asp Lys Gly Thr Gly Asn Lys Asn Lys Ile Thr Ile Thr Asn
           515                 520                 525
   Asp Gln Asn Arg Leu Thr Pro Glu Glu Ile Glu Arg Met Val Asn Asp
       530                 535                 540
   Ala Glu Lys Phe Ala Glu Glu Asp Lys Lys Leu Lys Glu Arg Ile Asp
   545                 550                 555                 560
   Thr Arg Asn Glu Leu Glu Ser Tyr Ala Tyr Ser Leu Lys Asn Gln Ile
                   565                     570                     575
   Gly Asp Lys Glu Lys Leu Gly Gly Lys Leu Ser Ser Glu Asp Lys Glu
               580                     585                     590
   Thr Met Glu Lys Ala Val Glu Glu Lys Ile Glu Trp Leu Glu Ser His
           595                     600                     605
   Gln Asp Ala Asp Ile Glu Asp Phe Lys Ala Lys Lys Lys Glu Leu Glu
       610                     615                     620
   Glu Ile Val Gln Pro Ile Ile Ser Lys Leu Tyr Gly Ser Ala Gly Pro
   625                     630                     635                     640
   Pro Pro Thr Gly Glu Glu Asp Thr Ala Glu Lys Asp Glu Leu
                   645                     650
<210> 3
   <211> 3925
   <212> DNA
   <213> Homo sapiens
<400> 3
   tttttttttt ttttttttga agtctatctt tttaatattc agtaacagct cctcagaaag       60
   agttacagac taggtggtcc acggtagtga gagcctttga ggtaagggaa gactagccct      120
   agttgctaac taccatttaa ccctattgcc ccagggtaca cagcactgta ggtcatcttt      180
   aatggctgta ttgggcttgg cctgagacca gtattctgct cagggtctgc ccaaatgctt      240
   ttcgggcagt gcagctgagg cctaggtcta attaagagtt tggtgttggg atccagttct      300
   actagcccta gagaattaac ctttcagcca gttgcccatc tataaaatac ctatccttgg      360
   gcagtattgg attctttttt ttttttttga ggtggagtct cactcttgtc gccaggctgg      420
   agtgccgggc tggagtacag tggtgtgatc tcggctcact gcaacctctg cctcccgggt      480
   tcgagtgatt ctcctgcctc agccttcagc ttgcactacc acgcccagct aatttttgta      540
   ttttcagtag agatggagtt tcaccatgtt ggcaaagatg gtctctatct cttgaccttg      600
   tgatccaccc gccttggcct cccaaagtgc taggattaca atattggatt ttatgttagc      660
   accagcctgt cctttattga tcataccatt tacctggact cttttcttca ggaacacaat      720
   ctaagtaatc ctaaaccagt tttgacacaa accattgcct ttaacaaccc attcatagtg      780
   aggggattta gtgtagtttc aatgtcacca tccaagatcc caccccagta ccactaatga      840
   gaaatttcag ggatggagat tctgacacag ctagggttca caatatacct ttacatccta      900
   aagttacaaa tgaaatccaa gtgtataaag ttaataccag tgatcactca ctccccatca      960
   tttctcagag aatccaagca gctataaaag catcattagt aataatttta aaaaaagcca     1020
   ccagaaaggc agttttagaa agataagggg aaataggaaa ccaagtatgt tcaagaaaac     1080
   cccactcttg ggcactgtga aatgcccctt gcctgagtaa agatgtggat taacttctct     1140
   cccctttctt tagattaatg caaaagccga gcatggtggt aacaagcctg tggtctcaat     1200
   tagctgggag actgaggtgg aaggatcact tgggcccagg agggttaaaa aaaaaaaaaa     1260
   aaaatccctg gaagcattaa atagaagtgt ttccttatcc caacatacca aatactccct     1320
   cccctcaccc cacacacgag tatctagtct taaatttcct aaaataagta attgatctaa     1380
   ttagaagctt ctcacaaaca ttagaccagt gtaaataaca aacatttatt ggtgtcactt     1440
   atggtagaaa aaacttccta caccagatgc acatgaccca gttgttaaat agaacatttt     1500
   gaaggtgaac acacacccta acccaggttt tttacctgct ttttaagatg gccaattctt     1560
   cttctccccc ccacccaaag acatgtgagc aactgctaat gaaaagcagt aaacagccgc     1620
   ttaggctata gcagtttcaa ctccactctg aggtgaagat tccaattaca ttcgagactt     1680
   aagttctttc aattttttcc taacaaaagt tcctgagtcc agtatttaca atattacagc    1740
   actagcagat cagtgtctac aactcatctt tttctgctgt atcctcttca ccagttgggg     1800
   gagggcctgc acttccatag agtttgctga taattggttg aacaatttct tccagttcct     1860
   tcttcttagc tttgaagtct tcaatgtcag catcttggtg gctttccagc cattcaatct     1920
   tttcttctac agctttttcc atggtctcct tatcttcaga ggaaagttta cctcccagct     1980
   tttctttatc tccaatctga ttctttagag aataggcata gctttccaac tcatttctag     2040
   tatcaatgcg ctccttgagc tttttgtctt cctcagcaaa cttctcagca tcattaacca     2100
   tcctttcgat ttcttcaggt gtcaggcgat tctggtcatt ggtgattgtg atcttatttt     2160
   tgttccctgt acccttgtct tcagctgtca ctcgaagaat accattcaca tctatctcaa     2220
   aggtgacttc aatctgtggg accccacgag gagcaggagg aattccagtc agatcaaatg     2280
   tacccagaag atgattgtct tttgtcaggg gtctttcacc ttcatagacc ttgattgtaa     2340
   cagttggttg attatcagaa gctgtagaaa agatctgaga cttcttggta ggcaccactg     2400
   tgttccttgg aatcagtttg gtcatgacac ctcccacagt ttcaatacca agtgtaaggg     2460
   gacatacatc aagcagtacc aggtcacctg tatcttgatc accagagagc acaccagcct     2520
   ggacagcagc accatacgct acagcttcat ctgggtttat gccacgggat ggttccttgc     2580
   cattgaagaa ctctttaacc agttgctgaa tctttggaat tcgagtcgag ccaccaacaa    2640
   gaacaatttc atcaatatca gacttcttca aatcagaatc ttccaacact ttctggacgg    2700
   gcttcatagt agaccggaac agatccatgt tgagctcttc aaatttggcc cgagtcaggg    2760
   tctcagaaaa gtcttctcct tcatagaagg actcaatttc aattcttgct tgatgctgag    2820
   aagacagggc ccgtttggcc ttttctacct cgcgccggag tttctgcaca gctctattgt    2880
   ctttcctgac atctttgccc gtcttctttt tgtacagttt gatgaagtgt tccatgacac    2940
   gctggtcaaa gtcttctcca cccagatgag tatctccatt agtggccaca acttcgaaga    3000
   caccattgtc aatggtgaga agagacacat cgaaggttcc gccacccagg tcaaacacca    3060
   ggatgttctt ctccccctcc ctcttatcca ggccataagc aatagcagct gccgtaggct    3120
   cgttgatgat cctcataaca tttaggccag caatagttcc agcgtctttg gttgcttggc    3180
   gttgggcatc attaaaatag gctggtacag taacaactgc atgggtaacc ttctttccca    3240
   aataagcctc agcggtttct ttcattttag tgagaaccat ggcagaaatt tcttcaggag    3300
   caaatgtctt tgtttgccca cctccaatat caacttgaat gtatggttta gttttctttt    3360
   caaccacctt gaacggcaag aacttgatgt cctgctgcac agacgggtca ttccacgtgc    3420
   ggccgatgag ccgcttggcg tcaaagaccg tgttctcggg gttggaggtg agctggttct    3480
   tggcggcatc gccaatcaga cgttcccctt caggagtgaa ggcgacatag gacggcgtga    3540
   tgcggttgcc ctgatcgttg gcgatgatct ccacgcggcc gttcttgaac acgccgacgc    3600
   aggagtaggt ggtccccagg tcgatgccga ccaccgtgcc cacgtcctcc ttcttgtcct    3660
   cctcctcggc ccgcgccgcg ctgagcagca gcagcatcgc ggccaccagg gagagcttca    3720
   tcttgccagc cagttgggca gcagcaggca gtccagccac aggccgtagc acaggagcac    3780
   agcgcaattt ccgacttgca ggcggcaggg gcccggggtc acaaggcgcc acgaaccagg    3840
   cgaagggcag gtctagaaat acaggccgcg gcgcttccct ctcacactcg cgaaacaccc    3900
   caataggtca atctgtctgt gctgt                                          3925
<210> 4
   <211> 21 <212> DNA
   <213> Homo sapiens
<400> 4
   aaggttaccc atgcagttgt t 21
<210> 5
   <211> 2074
   <212> DNA
   <213> Homo sapiens
<220>
   <221> CDS
   <222> (90)...(1604)
<400> 5
   caagcagcgg gttagtggtc gcgcgcccga cctccgcagt cccagccgag ccgcgaccct      60
   tccggccgtc cccaccccac ctcgccgcc atg cgc ctc cgc cgc cta gcg ctg      113
                                   Met Arg Leu Arg Arg Leu Ala Leu
                                    1               5
   ttc ccg ggt gtg gcg ctg ctt ctt gcc gcg gcc cgc ctc gcc gct gcc      161
   Phe Pro Gly Val Ala Leu Leu Leu Ala Ala Ala Arg Leu Ala Ala Ala
        10                  15                  20
   tcc gac gtg cta gaa ctc acg gac gac aac ttc gag agt cgc atc tcc      209
   Ser Asp Val Leu Glu Leu Thr Asp Asp Asn Phe Glu Ser Arg Ile Ser
    25                  30                  35                  40
   gac acg ggc tct gcg ggc ctc atg ctc gtc gag ttc ttc gcc ccc tgg      257
   Asp Thr Gly Ser Ala Gly Leu Met Leu Val Glu Phe Phe Ala Pro Trp
                    45                  50                  55
   tgt gga cac tgc aag aga ctt gca cct gag tat gaa gct gca gct acc      305
   Cys Gly His Cys Lys Arg Leu Ala Pro Glu Tyr Glu Ala Ala Ala Thr
                   60                  65                  70
   aga tta aaa gga ata gtc cca tta gca aag gtt gat tgc act gcc aac      353
   Arg Leu Lys Gly Ile Val Pro Leu Ala Lys Val Asp Cys Thr Ala Asn
           75                  80                  85
   act aac acc tgt aat aaa tat gga gtc agt gga tat cca acc ctg aag      401
   Thr Asn Thr Cys Asn Lys Tyr Gly Val Ser Gly Tyr Pro Thr Leu Lys
       90                  95                  100
   ata ttt aga gat ggt gaa gaa gca ggt gct tat gat gga cct agg act      449
   Ile Phe Arg Asp Gly Glu Glu Ala Gly Ala Tyr Asp Gly Pro Arg Thr
   105                 110                 115                 120
   gct gat gga att gtc agc cac ttg aag aag cag gca gga cca gct tca      497
   Ala Asp Gly Ile Val Ser His Leu Lys Lys Gln Ala Gly Pro Ala Ser
                   125                 130                 135
   gtg cct ctc agg act gag gaa gaa ttt aag aaa ttc att agt gat aaa      545
   Val Pro Leu Arg Thr Glu Glu Glu Phe Lys Lys Phe Ile Ser Asp Lys
               140                 145                 150
   gat gcc tct ata gta ggt ttt ttc gat gat tca ttc agt gag gct cac      593
   Asp Ala Ser Ile Val Gly Phe Phe Asp Asp Ser Phe Ser Glu Ala His
           155                 160                 165
   tcc gag ttc cta aaa gca gcc agc aac ttg agg gat aac tac cga ttt      641
   Ser Glu Phe Leu Lys Ala Ala Ser Asn Leu Arg Asp Asn Tyr Arg Phe
       170                 175                 180
   gca cat acg aat gtt gag tct ctg gtg aac gag tat gat gat aat gga      689
   Ala His Thr Asn Val Glu Ser Leu Val Asn Glu Tyr Asp Asp Asn Gly
   185                 190                 195                 200
   gag ggt atc atc tta ttt cgt cct tca cat ctc act aac aag ttt gag      737
   Glu Gly Ile Ile Leu Phe Arg Pro Ser His Leu Thr Asn Lys Phe Glu
                   205                 210                 215
   gac aag act gtg gca tat aca gag caa aaa atg acc agt ggc aaa att      785
   Asp Lys Thr Val Ala Tyr Thr Glu Gln Lys Met Thr Ser Gly Lys Ile
               220                 225                 230
   aaa aag ttt atc cag gaa aac att ttt ggt atc tgc cct cac atg aca      833
   Lys Lys Phe Ile Gln Glu Asn Ile Phe Gly Ile Cys Pro His Met Thr
           235                 240                 245
   gaa gac aat aaa gat ttg ata cag ggc aag gac tta ctt att gct tac      881
   Glu Asp Asn Lys Asp Leu Ile Gln Gly Lys Asp Leu Leu Ile Ala Tyr
       250                 255                 260
   tat gat gtg gac tat gaa aag aac gct aaa ggt tcc aac tac tgg aga      929
   Tyr Asp Val Asp Tyr Glu Lys Asn Ala Lys Gly Ser Asn Tyr Trp Arg
   265                 270                 275                 280
   aac agg gta atg atg gtg gca aag aaa ttc ctg gat gct ggg cac aaa      977
   Asn Arg Val Met Met Val Ala Lys Lys Phe Leu Asp Ala Gly His Lys
                   285                 290                 295
   ctc aac ttt gct gta gct agc cgc aaa acc ttt agc cat gaa ctt tct     1025
   Leu Asn Phe Ala Val Ala Ser Arg Lys Thr Phe Ser His Glu Leu Ser
               300                 305                 310
   gat ttt ggc ttg gag agc act gct gga gag att cct gtt gtt gct atc      1073
   Asp Phe Gly Leu Glu Ser Thr Ala Gly Glu Ile Pro Val Val Ala Ile
           315                 320                 325
   aga act gct aaa gga gag aag ttt gtc atg cag gag gag ttc tcg cgt      1121
   Arg Thr Ala Lys Gly Glu Lys Phe Val Met Gln Glu Glu Phe Ser Arg
       330                 335                 340
   gat ggg aag gct ctg gag agg ttc ctg cag gat tac ttt gat ggc aat      1169
   Asp Gly Lys Ala Leu Glu Arg Phe Leu Gln Asp Tyr Phe Asp Gly Asn
   345                 350                 355                 360
   ctg aag aga tac ctg aag tct gaa cct atc cca gag agc aat gat ggg      1217
   Leu Lys Arg Tyr Leu Lys Ser Glu Pro Ile Pro Glu Ser Asn Asp Gly
                   365                 370                 375
   cct gtg aag gta gtg gta gca gag aat ttt gat gaa ata gtg aat aat      1265
   Pro Val Lys Val Val Val Ala Glu Asn Phe Asp Glu Ile Val Asn Asn
               380                 385                 390
   gaa aat aaa gat gtg ctg att gaa ttt tat gcc cct tgg tgt ggt cac      1313
   Glu Asn Lys Asp Val Leu Ile Glu Phe Tyr Ala Pro Trp Cys Gly His
           395                 400                 405
   tgt aag aac ctg gag ccc aag tat aaa gaa ctt ggc gag aag ctc agc      1361
   Cys Lys Asn Leu Glu Pro Lys Tyr Lys Glu Leu Gly Glu Lys Leu Ser
       410                 415                 420
   aaa gac cca aat atc gtc ata gcc aag atg gat gcc aca gcc aat gat      1409
   Lys Asp Pro Asn Ile Val Ile Ala Lys Met Asp Ala Thr Ala Asn Asp
   425                 430                 435                 440
   gtg cct tct cca tat gaa gtc aga ggt ttt cct acc ata tac ttc tct      1457
   Val Pro Ser Pro Tyr Glu Val Arg Gly Phe Pro Thr Ile Tyr Phe Ser
                   445                 450                 455
   cca gcc aac aag aag cta aat cca aag aaa tat gaa ggt ggc cgt gaa      1505
   Pro Ala Asn Lys Lys Leu Asn Pro Lys Lys Tyr Glu Gly Gly Arg Glu
               460                 465                 470
   tta agt gat ttt att agc tat cta caa aga gaa gct aca aac ccc cct      1553
   Leu Ser Asp Phe Ile Ser Tyr Leu Gln Arg Glu Ala Thr Asn Pro Pro
           475                 480                 485
   gta att caa gaa gaa aaa ccc aag aag aag aag aag gca cag gag gat      1601
   Val Ile Gln Glu Glu Lys Pro Lys Lys Lys Lys Lys Ala Gln Glu Asp
       490                 495                 500
   ctc taaagcagta gccaaacacc actttgtaaa aggactcttc catcagagat           1654
   Leu
   505
   gggaaaacca ttggggagga ctaggaccca tatgggaatt attacctctc agggccgaga   1714
   ggacagaatg gatataatct gaatcctgtt aaattttctc taaactgttt cttagctgca   1774
   ctgtttatgg aaataccagg accagtttat gtttgtggtt ttgggaaaaa ttatttgtgt   1834
   tgggggaaat gttgtggggg tggggttgag ttgggggtat tttctaattt tttttgtaca   1894
   tttggaacag tgacaataaa tgagacccct ttaaactgtc aaaaaaaaaa aaaaaaaaaa   1954
   aaaaaaaaaa aaaaaaaaaa aaaaaaaaaa aaaaaaaaaa aaaaaaaaaa aaaaaaaaaa   2014
   aaaaaaaaaa aaaaaaaaaa aaaaaaaaaa aaaaaaaaaa aaaaaaaaaa aaaaaaaaaa   2074
<210> 6
   <211> 505
   <212> PRT
   <213> Homo sapiens
<400> 6
   Met Arg Leu Arg Arg Leu Ala Leu Phe Pro Gly Val Ala Leu Leu Leu
    1               5                  10                  15
   Ala Ala Ala Arg Leu Ala Ala Ala Ser Asp Val Leu Glu Leu Thr Asp
               20                  25                  30
   Asp Asn Phe Glu Ser Arg Ile Ser Asp Thr Gly Ser Ala Gly Leu Met
           35                  40                  45
   Leu Val Glu Phe Phe Ala Pro Trp Cys Gly His Cys Lys Arg Leu Ala
       50                  55                  60
   Pro Glu Tyr Glu Ala Ala Ala Thr Arg Leu Lys Gly Ile Val Pro Leu
   65                  70                  75                  80
   Ala Lys Val Asp Cys Thr Ala Asn Thr Asn Thr Cys Asn Lys Tyr Gly
                   85                  90                  95
   Val Ser Gly Tyr Pro Thr Leu Lys Ile Phe Arg Asp Gly Glu Glu Ala
               100                 105                 110
   Gly Ala Tyr Asp Gly Pro Arg Thr Ala Asp Gly Ile Val Ser His Leu
           115                 120                 125
   Lys Lys Gln Ala Gly Pro Ala Ser Val Pro Leu Arg Thr Glu Glu Glu
       130                 135                 140
   Phe Lys Lys Phe Ile Ser Asp Lys Asp Ala Ser Ile Val Gly Phe Phe
   145                 150                 155                 160
   Asp Asp Ser Phe Ser Glu Ala His Ser Glu Phe Leu Lys Ala Ala Ser
                   165                 170                 175
   Asn Leu Arg Asp Asn Tyr Arg Phe Ala His Thr Asn Val Glu Ser Leu
               180                 185                 190
   Val Asn Glu Tyr Asp Asp Asn Gly Glu Gly Ile Ile Leu Phe Arg Pro
           195                 200                 205
   Ser His Leu Thr Asn Lys Phe Glu Asp Lys Thr Val Ala Tyr Thr Glu
       210                 215                 220
   Gln Lys Met Thr Ser Gly Lys Ile Lys Lys Phe Ile Gln Glu Asn Ile
   225                 230                 235                 240
   Phe Gly Ile Cys Pro His Met Thr Glu Asp Asn Lys Asp Leu Ile Gln
                   245                 250                 255
   Gly Lys Asp Leu Leu Ile Ala Tyr Tyr Asp Val Asp Tyr Glu Lys Asn
               260                 265                 270
   Ala Lys Gly Ser Asn Tyr Trp Arg Asn Arg Val Met Met Val Ala Lys
           275                 280                 285
   Lys Phe Leu Asp Ala Gly His Lys Leu Asn Phe Ala Val Ala Ser Arg
       290                 295                 300
   Lys Thr Phe Ser His Glu Leu Ser Asp Phe Gly Leu Glu Ser Thr Ala
   305                 310                 315                 320
   Gly Glu Ile Pro Val Val Ala Ile Arg Thr Ala Lys Gly Glu Lys Phe
                   325                 330                 335
   Val Met Gln Glu Glu Phe Ser Arg Asp Gly Lys Ala Leu Glu Arg Phe
               340                 345                 350
   Leu Gln Asp Tyr Phe Asp Gly Asn Leu Lys Arg Tyr Leu Lys Ser Glu
           355                 360                 365
   Pro Ile Pro Glu Ser Asn Asp Gly Pro Val Lys Val Val Val Ala Glu
       370                 375                 380
   Asn Phe Asp Glu Ile Val Asn Asn Glu Asn Lys Asp Val Leu Ile Glu
   385                 390                 395                 400
   Phe Tyr Ala Pro Trp Cys Gly His Cys Lys Asn Leu Glu Pro Lys Tyr
                   405                 410                 415
   Lys Glu Leu Gly Glu Lys Leu Ser Lys Asp Pro Asn Ile Val Ile Ala
               420                 425                 430
   Lys Met Asp Ala Thr Ala Asn Asp Val Pro Ser Pro Tyr Glu Val Arg
           435                     440                     445
   Gly Phe Pro Thr Ile Tyr Phe Ser Pro Ala Asn Lys Lys Leu Asn Pro
       450                     455                     460
   Lys Lys Tyr Glu Gly Gly Arg Glu Leu Ser Asp Phe Ile Ser Tyr Leu
   465                 470                     475                 480
   Gln Arg Glu Ala Thr Asn Pro Pro Val Ile Gln Glu Glu Lys Pro Lys
                   485                 490                     495
   Lys Lys Lys Lys Ala Gln Glu Asp Leu
               500                 505
<210> 7
   <211> 1899
   <212> DNA
   <213> Homo sapiens
<220>
   <221> CDS
   <222> (69)...(1319)
<400> 7
   gtccgtactg cagagccgct gccggagggt cgttttaaag ggccgcgttg ccgccccctc      60
   ggcccgcc atg ctg cta tcc gtg ccg ctg ctg ctc ggc ctc ctc ggc ctg       110
            Met Leu Leu Ser Val Pro Leu Leu Leu Gly Leu Leu Gly Leu
             1               5                  10
   gcc gtc gcc gag ccc gcc gtc tac ttc aag gag cag ttt ctg gac gga        158
   Ala Val Ala Glu Pro Ala Val Tyr Phe Lys Glu Gln Phe Leu Asp Gly
    15                  20                  25                  30
   gac ggg tgg act tcc cgc tgg atc gaa tcc aaa cac aag tca gat ttt        206
   Asp Gly Trp Thr Ser Arg Trp Ile Glu Ser Lys His Lys Ser Asp Phe
                    35                  40                  45
   ggc aaa ttc gtt ctc agt tcc ggc aag ttc tac ggt gac gag gag aaa        254
   Gly Lys Phe Val Leu Ser Ser Gly Lys Phe Tyr Gly Asp Glu Glu Lys
                50                  55                  60
   gat aaa ggt ttg cag aca agc cag gat gca cgc ttt tat gct ctg tcg        302
   Asp Lys Gly Leu Gln Thr Ser Gln Asp Ala Arg Phe Tyr Ala Leu Ser
            65                  70                  75
   gcc agt ttc gag cct ttc agc aac aaa ggc cag acg ctg gtg gtg cag        350
   Ala Ser Phe Glu Pro Phe Ser Asn Lys Gly Gln Thr Leu Val Val Gln
        80                  85                  90
   ttc acg gtg aaa cat gag cag aac atc gac tgt ggg ggc ggc tat gtg        398
   Phe Thr Val Lys His Glu Gln Asn Ile Asp Cys Gly Gly Gly Tyr Val
    95                 100                 105                 110
   aag ctg ttt cct aat agt ttg gac cag aca gac atg cac gga gac tca        446
   Lys Leu Phe Pro Asn Ser Leu Asp Gln Thr Asp Met His Gly Asp Ser
                   115                 120                 125
   gaa tac aac atc atg ttt ggt ccc gac atc tgt ggc cct ggc acc aag        494
   Glu Tyr Asn Ile Met Phe Gly Pro Asp Ile Cys Gly Pro Gly Thr Lys
               130                 135                 140
   aag gtt cat gtc atc ttc aac tac aag ggc aag aac gtg ctg atc aac        542
   Lys Val His Val Ile Phe Asn Tyr Lys Gly Lys Asn Val Leu Ile Asn
           145                 150                 155
   aag gac atc cgt tgc aag gat gat gag ttt aca cac ctg tac aca ctg      590
   Lys Asp Ile Arg Cys Lys Asp Asp Glu Phe Thr His Leu Tyr Thr Leu
       160                 165                 170
   att gtg cgg cca gac aac acc tat gag gtg aag att gac aac agc cag      638
   Ile Val Arg Pro Asp Asn Thr Tyr Glu Val Lys Ile Asp Asn Ser Gln
   175                 180                 185                 190
   gtg gag tcc ggc tcc ttg gaa gac gat tgg gac ttc ctg cca ccc aag      686
   Val Glu Ser Gly Ser Leu Glu Asp Asp Trp Asp Phe Leu Pro Pro Lys
                   195                 200                 205
   aag ata aag gat cct gat gct tca aaa ccg gaa gac tgg gat gag cgg      734
   Lys Ile Lys Asp Pro Asp Ala Ser Lys Pro Glu Asp Trp Asp Glu Arg
               210                 215                 220
   gcc aag atc gat gat ccc aca gac tcc aag cct gag gac tgg gac aag      782
   Ala Lys Ile Asp Asp Pro Thr Asp Ser Lys Pro Glu Asp Trp Asp Lys
           225                 230                 235
   ccc gag cat atc cct gac cct gat gct aag aag ccc gag gac tgg gat      830
   Pro Glu His Ile Pro Asp Pro Asp Ala Lys Lys Pro Glu Asp Trp Asp
       240                 245                 250
   gaa gag atg gac gga gag tgg gaa ccc cca gtg att cag aac cct gag      878
   Glu Glu Met Asp Gly Glu Trp Glu Pro Pro Val Ile Gln Asn Pro Glu
   255                 260                 265                 270
   tac aag ggt gag tgg aag ccc cgg cag atc gac aac cca gat tac aag      926
   Tyr Lys Gly Glu Trp Lys Pro Arg Gln Ile Asp Asn Pro Asp Tyr Lys
                   275                 280                 285
   ggc act tgg atc cac cca gaa att gac aac ccc gag tat tct ccc gat      974
   Gly Thr Trp Ile His Pro Glu Ile Asp Asn Pro Glu Tyr Ser Pro Asp
               290                 295                 300
   ccc agt atc tat gcc tat gat aac ttt ggc gtg ctg ggc ctg gac ctc     1022
   Pro Ser Ile Tyr Ala Tyr Asp Asn Phe Gly Val Leu Gly Leu Asp Leu
           305                 310                 315
   tgg cag gtc aag tct ggc acc atc ttt gac aac ttc ctc atc acc aac     1070
   Trp Gln Val Lys Ser Gly Thr Ile Phe Asp Asn Phe Leu Ile Thr Asn
       320                 325                 330
   gat gag gca tac gct gag gag ttt ggc aac gag acg tgg ggc gta aca     1118
   Asp Glu Ala Tyr Ala Glu Glu Phe Gly Asn Glu Thr Trp Gly Val Thr
   335                 340                 345                 350
   aag gca gca gag aaa caa atg aag gac aaa cag gac gag gag cag agg     1166
   Lys Ala Ala Glu Lys Gln Met Lys Asp Lys Gln Asp Glu Glu Gln Arg
                   355                 360                 365
   ctt aag gag gag gaa gaa gac aag aaa cgc aaa gag gag gag gag gca     1214
   Leu Lys Glu Glu Glu Glu Asp Lys Lys Arg Lys Glu Glu Glu Glu Ala
               370                 375                 380
   gag gac aag gag gat gat gag gac aaa gat gag gat gag gag gat gag     1262
   Glu Asp Lys Glu Asp Asp Glu Asp Lys Asp Glu Asp Glu Glu Asp Glu
           385                 390                 395
   gag gac aag gag gaa gat gag gag gaa gat gtc ccc ggc cag gcc aag     1310
   Glu Asp Lys Glu Glu Asp Glu Glu Glu Asp Val Pro Gly Gln Ala Lys
       400                 405                 410
   gac gag ctg tagagaggcc tgcctccagg gctggactga ggcctgagcg         1359
   Asp Glu Leu
   415
   ctcctgccgc agagcttgcc gcgccaaata atgtctctgt gagactcgag aactttcatt   1419
   tttttccagg ctggttcgga tttggggtgg attttggttt tgttcccctc ctccactctc   1479
   ccccaccccc tccccgccct tttttttttt ttttttaaac tggtatttta tcctttgatt   1539
   ctccttcagc cctcacccct ggttctcatc tttcttgatc aacatctttt cttgcctctg   1599
   tgccccttct ctcatctctt agctcccctc caacctgggg ggcagtggtg tggagaagcc   1659
   acaggcctga gatttcatct gctctccttc ctggagccca gaggagggca gcagaagggg   1719
   gtggtgtctc caacccccca gcactgagga agaacggggc tcttctcatt tcacccctcc   1779
   ctttctcccc tgcccccagg actgggccac ttctgggtgg ggcagtgggt cccagattgg   1839
   ctcacactga gaatgtaaga actacaaaca aaatttctat taaattaaat tttgtgtctc   1899
<210> 8
   <211> 417
   <212> PRT
   <213> Homo sapiens
<400> 8
   Met Leu Leu Ser Val Pro Leu Leu Leu Gly Leu Leu Gly Leu Ala Val
    1               5                  10                  15
   Ala Glu Pro Ala Val Tyr Phe Lys Glu Gln Phe Leu Asp Gly Asp Gly
               20                  25                  30
   Trp Thr Ser Arg Trp Ile Glu Ser Lys His Lys Ser Asp Phe Gly Lys
           35                  40                  45
   Phe Val Leu Ser Ser Gly Lys Phe Tyr Gly Asp Glu Glu Lys Asp Lys
       50                  55                  60
   Gly Leu Gln Thr Ser Gln Asp Ala Arg Phe Tyr Ala Leu Ser Ala Ser
   65                  70                  75                  80
   Phe Glu Pro Phe Ser Asn Lys Gly Gln Thr Leu Val Val Gln Phe Thr
                   85                  90                  95
   Val Lys His Glu Gln Asn Ile Asp Cys Gly Gly Gly Tyr Val Lys Leu
               100                 105                 110
   Phe Pro Asn Ser Leu Asp Gln Thr Asp Met His Gly Asp Ser Glu Tyr
           115                 120                 125
   Asn Ile Met Phe Gly Pro Asp Ile Cys Gly Pro Gly Thr Lys Lys Val
       130                 135                 140
   His Val Ile Phe Asn Tyr Lys Gly Lys Asn Val Leu Ile Asn Lys Asp
   145                 150                 155                 160
   Ile Arg Cys Lys Asp Asp Glu Phe Thr His Leu Tyr Thr Leu Ile Val
                   165                 170                 175
   Arg Pro Asp Asn Thr Tyr Glu Val Lys Ile Asp Asn Ser Gln Val Glu
               180                 185                 190
   Ser Gly Ser Leu Glu Asp Asp Trp Asp Phe Leu Pro Pro Lys Lys Ile
           195                 200                 205
   Lys Asp Pro Asp Ala Ser Lys Pro Glu Asp Trp Asp Glu Arg Ala Lys
       210                 215                 220
   Ile Asp Asp Pro Thr Asp Ser Lys Pro Glu Asp Trp Asp Lys Pro Glu
   225                 230                 235                 240
   His Ile Pro Asp Pro Asp Ala Lys Lys Pro Glu Asp Trp Asp Glu Glu
                   245                 250                 255
   Met Asp Gly Glu Trp Glu Pro Pro Val Ile Gln Asn Pro Glu Tyr Lys
               260                 265                 270
   Gly Glu Trp Lys Pro Arg Gln Ile Asp Asn Pro Asp Tyr Lys Gly Thr
           275                 280                 285
   Trp Ile His Pro Glu Ile Asp Asn Pro Glu Tyr Ser Pro Asp Pro Ser
       290                 295                 300
   Ile Tyr Ala Tyr Asp Asn Phe Gly Val Leu Gly Leu Asp Leu Trp Gln
   305                 310                 315                 320
   Val Lys Ser Gly Thr Ile Phe Asp Asn Phe Leu Ile Thr Asn Asp Glu
                   325                 330                 335
   Ala Tyr Ala Glu Glu Phe Gly Asn Glu Thr Trp Gly Val Thr Lys Ala
               340                 345                 350
   Ala Glu Lys Gln Met Lys Asp Lys Gln Asp Glu Glu Gln Arg Leu Lys
           355                 360                 365
   Glu Glu Glu Glu Asp Lys Lys Arg Lys Glu Glu Glu Glu Ala Glu Asp
       370                 375                 380
   Lys Glu Asp Asp Glu Asp Lys Asp Glu Asp Glu Glu Asp Glu Glu Asp
   385                 390                 395                 400
   Lys Glu Glu Asp Glu Glu Glu Asp Val Pro Gly Gln Ala Lys Asp Glu
                   405                 410                 415
   Leu
<210> 9
   <211> 1288
   <212> DNA
   <213> Homo sapiens
<220>
   <221> CDS
   <222> (64)...(1215)
<400> 9
   agaggcgcag agagagctgg gagctaaggg gtggcggcga ccggaagcgc agtgcacacc     60
   ccc atg gcc cgg gct ttg gtc cag ttc tgg gcc ata tgc atg ctg cga      108
       Met Ala Arg Ala Leu Val Gln Phe Trp Ala Ile Cys Met Leu Arg
        1               5                  10                  15
   gtg gcg ctg gct acc gtc tat ttc caa gag gaa ttt cta gac gga gag      156
   Val Ala Leu Ala Thr Val Tyr Phe Gln Glu Glu Phe Leu Asp Gly Glu
                   20                  25                  30
   cat tgg aga aac cga tgg ttg cag tcc acc aat gac tcc cga ttt ggg      204
   His Trp Arg Asn Arg Trp Leu Gln Ser Thr Asn Asp Ser Arg Phe Gly
               35                  40                  45
   cat ttt aga ctt tcg tcg ggc aag ttt tat ggt cat aaa gag aaa gat      252
   His Phe Arg Leu Ser Ser Gly Lys Phe Tyr Gly His Lys Glu Lys Asp
           50                  55                  60
   aaa ggt ctg caa acc act cag aat ggc cga ttc tat gcc atc tct gca      300
   Lys Gly Leu Gln Thr Thr Gln Asn Gly Arg Phe Tyr Ala Ile Ser Ala
       65                  70                  75
   cgc ttc aaa ccg ttc agc aat aaa ggg aaa act ctg gtt att cag tac      348
   Arg Phe Lys Pro Phe Ser Asn Lys Gly Lys Thr Leu Val Ile Gln Tyr
   80                  85                  90                  95
   aca gta aaa cat gag cag aag atg gac tgt gga ggg ggc tac att aag      396
   Thr Val Lys His Glu Gln Lys Met Asp Cys Gly Gly Gly Tyr Ile Lys
                   100                 105                 110
   gtc ttt cct gca gac att gac cag aag aac ctg aat gga aaa tcg caa      444
   Val Phe Pro Ala Asp Ile Asp Gln Lys Asn Leu Asn Gly Lys Ser Gln
               115                 120                 125
   tac tat att atg ttt gga ccc gat att tgt gga ttt gat atc aag aaa      492
   Tyr Tyr Ile Met Phe Gly Pro Asp Ile Cys Gly Phe Asp Ile Lys Lys
           130                 135                 140
   gtt cat gtt att tta cat ttc aag aat aag tat cac gaa aac aag aaa      540
   Val His Val Ile Leu His Phe Lys Asn Lys Tyr His Glu Asn Lys Lys
       145                 150                 155
   ctg atc agg tgt aag gtt gat ggc ttc aca cac ctg tac act cta att      588
   Leu Ile Arg Cys Lys Val Asp Gly Phe Thr His Leu Tyr Thr Leu Ile
   160                 165                 170                 175
   tta aga cca gat ctt tct tat gat gtg aaa att gat ggt cag tca att      636
   Leu Arg Pro Asp Leu Ser Tyr Asp Val Lys Ile Asp Gly Gln Ser Ile
                   180                 185                 190
   gaa tcc ggc agc ata gag tac gac tgg aac tta aca tca ctc aag aag      684
   Glu Ser Gly Ser Ile Glu Tyr Asp Trp Asn Leu Thr Ser Leu Lys Lys
               195                 200                 205
   gaa acg tcc ccg gca gaa tcg aag gat tgg gaa cag act aaa gac aac      732
   Glu Thr Ser Pro Ala Glu Ser Lys Asp Trp Glu Gln Thr Lys Asp Asn
           210                 215                 220
   aaa gcc cag gac tgg gag aag cat ttt ctg gac gcc agc acc agc aag      780
   Lys Ala Gln Asp Trp Glu Lys His Phe Leu Asp Ala Ser Thr Ser Lys
       225                 230                 235
   cag agc gac tgg aac ggt gac ctg gat ggg gac tgg cca gcg ccg atg      828
   Gln Ser Asp Trp Asn Gly Asp Leu Asp Gly Asp Trp Pro Ala Pro Met
   240                 245                 250                 255
   ctc cag aag ccc ccg tac cag gat ggc ctg aaa cca gaa ggt att cat      876
   Leu Gln Lys Pro Pro Tyr Gln Asp Gly Leu Lys Pro Glu Gly Ile His
                   260                 265                 270
   aaa gac gtc tgg ctc cac cgt aag atg aag aat acc gac tat ttg acg      924
   Lys Asp Val Trp Leu His Arg Lys Met Lys Asn Thr Asp Tyr Leu Thr
               275                 280                 285
   cag tat gac ctc tca gaa ttt gag aac att ggt gcc att ggc ctg gag      972
   Gln Tyr Asp Leu Ser Glu Phe Glu Asn Ile Gly Ala Ile Gly Leu Glu
           290                 295                 300
   ctt tgg cag gtg aga tct gga acc att ttt gat aac ttt ctg atc aca     1020
   Leu Trp Gln Val Arg Ser Gly Thr Ile Phe Asp Asn Phe Leu Ile Thr
       305                 310                 315
   gat gat gaa gag tat gca gat aat ttt ggc aag gcc acc tgg ggc gaa     1068
   Asp Asp Glu Glu Tyr Ala Asp Asn Phe Gly Lys Ala Thr Trp Gly Glu
   320                 325                 330                 335
   acc aag ggt cca gaa agg gag atg gat gcc ata cag gcc aag gag gaa     1116
   Thr Lys Gly Pro Glu Arg Glu Met Asp Ala Ile Gln Ala Lys Glu Glu
                   340                 345                 350
   atg aag aag gcc cgc gag gaa gag gag gaa gag ctg ctg tcg gga aaa     1164
   Met Lys Lys Ala Arg Glu Glu Glu Glu Glu Glu Leu Leu Ser Gly Lys
               355                 360                 365
   att aac agg cac gaa cat tac ttc aat caa ttt cac aga agg aat gaa     1212
   Ile Asn Arg His Glu His Tyr Phe Asn Gln Phe His Arg Arg Asn Glu
   370 375 380
   ctt tagtgatccc cattggatat aaggatgact ggtaaaatct cattgctact 1265
   Leu
   ttaatctaaa aaaaaaaaaa aaa 1288
<210> 10
   <211> 384
   <212> PRT
   <213> Homo sapiens
<400> 10
   Met Ala Arg Ala Leu Val Gln Phe Trp Ala Ile Cys Met Leu Arg Val
    1               5                  10                  15
   Ala Leu Ala Thr Val Tyr Phe Gln Glu Glu Phe Leu Asp Gly Glu His
               20                  25                  30
   Trp Arg Asn Arg Trp Leu Gln Ser Thr Asn Asp Ser Arg Phe Gly His
           35                  40                  45
   Phe Arg Leu Ser Ser Gly Lys Phe Tyr Gly His Lys Glu Lys Asp Lys
       50                  55                  60
   Gly Leu Gln Thr Thr Gln Asn Gly Arg Phe Tyr Ala Ile Ser Ala Arg
   65                  70                  75                  80
   Phe Lys Pro Phe Ser Asn Lys Gly Lys Thr Leu Val Ile Gln Tyr Thr
                   85                  90                  95
   Val Lys His Glu Gln Lys Met Asp Cys Gly Gly Gly Tyr Ile Lys Val
               100                 105                 110
   Phe Pro Ala Asp Ile Asp Gln Lys Asn Leu Asn Gly Lys Ser Gln Tyr
           115                 120                 125
   Tyr Ile Met Phe Gly Pro Asp Ile Cys Gly Phe Asp Ile Lys Lys Val
       130                 135                 140
   His Val Ile Leu His Phe Lys Asn Lys Tyr His Glu Asn Lys Lys Leu
   145                 150                 155                 160
   Ile Arg Cys Lys Val Asp Gly Phe Thr His Leu Tyr Thr Leu Ile Leu
                   165                 170                 175
   Arg Pro Asp Leu Ser Tyr Asp Val Lys Ile Asp Gly Gln Ser Ile Glu
               180                 185                 190
   Ser Gly Ser Ile Glu Tyr Asp Trp Asn Leu Thr Ser Leu Lys Lys Glu
           195                 200                 205
   Thr Ser Pro Ala Glu Ser Lys Asp Trp Glu Gln Thr Lys Asp Asn Lys
       210                 215                 220
   Ala Gln Asp Trp Glu Lys His Phe Leu Asp Ala Ser Thr Ser Lys Gln
   225                 230                 235                 240
   Ser Asp Trp Asn Gly Asp Leu Asp Gly Asp Trp Pro Ala Pro Met Leu
                   245                 250                 255
   Gln Lys Pro Pro Tyr Gln Asp Gly Leu Lys Pro Glu Gly Ile His Lys
               260                 265                 270
   Asp Val Trp Leu His Arg Lys Met Lys Asn Thr Asp Tyr Leu Thr Gln
           275                 280                 285
   Tyr Asp Leu Ser Glu Phe Glu Asn Ile Gly Ala Ile Gly Leu Glu Leu
       290                 295                 300
   Trp Gln Val Arg Ser Gly Thr Ile Phe Asp Asn Phe Leu Ile Thr Asp
   305                 310                 315                 320
   Asp Glu Glu Tyr Ala Asp Asn Phe Gly Lys Ala Thr Trp Gly Glu Thr
                   325                 330                 335
   Lys Gly Pro Glu Arg Glu Met Asp Ala Ile Gln Ala Lys Glu Glu Met
               340                 345                 350
   Lys Lys Ala Arg Glu Glu Glu Glu Glu Glu Leu Leu Ser Gly Lys Ile
           355                 360                 365
   Asn Arg His Glu His Tyr Phe Asn Gln Phe His Arg Arg Asn Glu Leu
   370                 375                 380
<210> 11
   <211> 1659
   <212> DNA
   <213> Homo sapiens
<220>
   <221> CDS
   <222> (28) ... (1560)
<400> 11
   agcagtacag gcagaagctg gcggctc atg gct tcg tgc cca tgg ggt cag gaa      54
                                 Met Ala Ser Cys Pro Trp Gly Gln Glu
                                 1               5
   cag gga gcg agg agc ccc tcg gag gag cct cca gag gag gaa atc ccc      102
   Gln Gly Ala Arg Ser Pro Ser Glu Glu Pro Pro Glu Glu Glu Ile Pro
   10                  15                  20                  25
   aag gag gat ggg atc ttg gtg ctg agc cgc cac acc ctg ggc ctg gcc      150
   Lys Glu Asp Gly Ile Leu Val Leu Ser Arg His Thr Leu Gly Leu Ala
                   30                  35                  40
   ctg cgg gag cac cct gcc ctg ctg gtg gaa ttc tat gcc ccg tgg tgt      198
   Leu Arg Glu His Pro Ala Leu Leu Val Glu Phe Tyr Ala Pro Trp Cys
               45                  50                  55
   ggg cac tgc cag gcc ctg gcc ccc gag tac agc aag gca gct gcc gtg      246
   Gly His Cys Gln Ala Leu Ala Pro Glu Tyr Ser Lys Ala Ala Ala Val
           60                  65                  70
   ctc gcg gcc gag tca atg gtg gtc acg ctg gcc aag gtg gat ggg ccc      294
   Leu Ala Ala Glu Ser Met Val Val Thr Leu Ala Lys Val Asp Gly Pro
       75                  80                  85
   gcg cag cgc gag ctg gct gag gag ttt ggt gtg acg gag tac cct acg      342
   Ala Gln Arg Glu Leu Ala Glu Glu Phe Gly Val Thr Glu Tyr Pro Thr
   90                  95                  100                 105
   ctc aag ttc ttc cgc aat ggg aac cgc acg cac ccc gag gag tac aca      390
   Leu Lys Phe Phe Arg Asn Gly Asn Arg Thr His Pro Glu Glu Tyr Thr
                   110                 115                 120
   gga cca cgg gac gct gag ggc att gcc gag tgg ctg cga cgg cgg gtg      438
   Gly Pro Arg Asp Ala Glu Gly Ile Ala Glu Trp Leu Arg Arg Arg Val
               125                 130                 135
   ggg ccc agt gcc atg cgg ctg gag gat gag gcg gcc gcc cag gcg ctg      486
   Gly Pro Ser Ala Met Arg Leu Glu Asp Glu Ala Ala Ala Gln Ala Leu
           140                 145                 150
   atc ggt ggc cgg gac cta gtg gtc att ggc ttc ttc cag gac ctg cag      534
   Ile Gly Gly Arg Asp Leu Val Val Ile Gly Phe Phe Gln Asp Leu Gln
       155                 160                 165
   gac gag gac gtg gcc acc ttc ttg gcc ttg gcc cag gac gcc ctg gac      582
   Asp Glu Asp Val Ala Thr Phe Leu Ala Leu Ala Gln Asp Ala Leu Asp
   170                 175                 180                 185
   atg acc ttt ggc ctc aca gac cgg ccg cgg ctc ttt cag cag ttt ggc      630
   Met Thr Phe Gly Leu Thr Asp Arg Pro Arg Leu Phe Gln Gln Phe Gly
                   190                 195                 200
   ctc acc aag gac act gtg gtt ctc ttc aag aag ttt gat gag ggg cgg       678
   Leu Thr Lys Asp Thr Val Val Leu Phe Lys Lys Phe Asp Glu Gly Arg
               205                 210                 215
   gca gac ttc ccc gtg gac gag gag ctt ggc ctg gac ctg ggg gat ctg       726
   Ala Asp Phe Pro Val Asp Glu Glu Leu Gly Leu Asp Leu Gly Asp Leu
           220                 225                 230
   tcg cgc ttc ctg gtc aca cac agc atg cgc ctg gtc acg gag ttc aac       774
   Ser Arg Phe Leu Val Thr His Ser Met Arg Leu Val Thr Glu Phe Asn
       235                 240                 245
   agc cag acg tct gcc aag atc ttc gcg gcc agg atc ctc aac cac ctg       822
   Ser Gln Thr Ser Ala Lys Ile Phe Ala Ala Arg Ile Leu Asn His Leu
   250                 255                 260                 265
   ctg ctg ttt gtc aac cag acg ctg gct gcg cac cgg gag ctc cta gcg       870
   Leu Leu Phe Val Asn Gln Thr Leu Ala Ala His Arg Glu Leu Leu Ala
                   270                 275                 280
   ggc ttt ggg gag gca gct ccc cgc ttc cgg ggg cag gtg ctg ttc gtg       918
   Gly Phe Gly Glu Ala Ala Pro Arg Phe Arg Gly Gln Val Leu Phe Val
               285                 290                 295
   gtg gtg gac gtg gcg gcc gac aat gag cac gtg ctg cag tac ttt gga       966
   Val Val Asp Val Ala Ala Asp Asn Glu His Val Leu Gln Tyr Phe Gly
           300                 305                 310
   ctc aag gct gag gca gcc ccc act ctg cgc ttg gtc aac ctt gaa acc      1014
   Leu Lys Ala Glu Ala Ala Pro Thr Leu Arg Leu Val Asn Leu Glu Thr
       315                 320                 325
   act aag aag tat gcg cct gtg gat ggg ggc cct gtc acc gca gcg tcc      1062
   Thr Lys Lys Tyr Ala Pro Val Asp Gly Gly Pro Val Thr Ala Ala Ser
   330                 335                 340                 345
   atc act gct ttc tgc cat gca gtc ctc aac ggc caa gtc aag ccc tat      1110
   Ile Thr Ala Phe Cys His Ala Val Leu Asn Gly Gln Val Lys Pro Tyr
                   350                 355                 360
   ctc ctg agc cag gag ata ccc cct gat tgg gat cag cgg cca gtt aag      1158
   Leu Leu Ser Gln Glu Ile Pro Pro Asp Trp Asp Gln Arg Pro Val Lys
               365                 370                 375
   acc ctc gtg ggc aag aat ttt gag cag gtg gct ttt gac gaa acc aag      1206
   Thr Leu Val Gly Lys Asn Phe Glu Gln Val Ala Phe Asp Glu Thr Lys
           380                 385                 390
   aat gtg ttt gtc aag ttc tat gcc ccg tgg tgc acc cac tgc aag gag      1254
   Asn Val Phe Val Lys Phe Tyr Ala Pro Trp Cys Thr His Cys Lys Glu
       395                 400                 405
   atg gcc cct gcc tgg gag gca ttg gct gag aag tac caa gac cac gag      1302
   Met Ala Pro Ala Trp Glu Ala Leu Ala Glu Lys Tyr Gln Asp His Glu
   410                 415                 420                 425
   gac atc atc att gct gag ctg gat gcc acg gcc aac gag ctg gat gcc      1350
   Asp Ile Ile Ile Ala Glu Leu Asp Ala Thr Ala Asn Glu Leu Asp Ala
                   430                 435                 440
   ttc gct gtg cac ggc ttc cct act ctc aag tac ttc cca gca ggg cca      1398
   Phe Ala Val His Gly Phe Pro Thr Leu Lys Tyr Phe Pro Ala Gly Pro
               445                 450                 455
   ggt cgg aag gtg att gaa tac aaa agc acc agg gac ctg gag act ttc      1446
   Gly Arg Lys Val Ile Glu Tyr Lys Ser Thr Arg Asp Leu Glu Thr Phe
           460                 465                 470
   tcc aag ttc ctg gac aac ggg ggc gtg ctg ccc acg gag gag tcc ccg      1494
   Ser Lys Phe Leu Asp Asn Gly Gly Val Leu Pro Thr Glu Glu Ser Pro
       475                 480                 485
   gag gag cca gca gcc ccg ttc ccg gag cca ccg gcc aac tcc act atg      1542
   Glu Glu Pro Ala Ala Pro Phe Pro Glu Pro Pro Ala Asn Ser Thr Met
   490                 495                 500                 505
   ggg tcc aag gag gaa ctg tagctgcccc cgtgtcaccc ccgccatcac             1590
   Gly Ser Lys Glu Glu Leu
                   510
   tgctggacag gagccacccc cttgggtacc agagggagct gtgcattgtg aataaagagt    1650
   gagcttggt                                                            1659
<210> 12
   <211> 511
   <212> PRT
   <213> Homo sapiens
<400> 12
   Met Ala Ser Cys Pro Trp Gly Gln Glu Gln Gly Ala Arg Ser Pro Ser
    1               5                  10                  15
   Glu Glu Pro Pro Glu Glu Glu Ile Pro Lys Glu Asp Gly Ile Leu Val
               20                  25                  30
   Leu Ser Arg His Thr Leu Gly Leu Ala Leu Arg Glu His Pro Ala Leu
           35                  40                  45
   Leu Val Glu Phe Tyr Ala Pro Trp Cys Gly His Cys Gln Ala Leu Ala
       50                  55                  60
   Pro Glu Tyr Ser Lys Ala Ala Ala Val Leu Ala Ala Glu Ser Met Val
   65                  70                  75                  80
   Val Thr Leu Ala Lys Val Asp Gly Pro Ala Gln Arg Glu Leu Ala Glu
                   85                  90                  95
   Glu Phe Gly Val Thr Glu Tyr Pro Thr Leu Lys Phe Phe Arg Asn Gly
               100                 105                 110
   Asn Arg Thr His Pro Glu Glu Tyr Thr Gly Pro Arg Asp Ala Glu Gly
           115                 120                 125
   Ile Ala Glu Trp Leu Arg Arg Arg Val Gly Pro Ser Ala Met Arg Leu
       130                 135                 140
   Glu Asp Glu Ala Ala Ala Gln Ala Leu Ile Gly Gly Arg Asp Leu Val
   145                 150                 155                 160
   Val Ile Gly Phe Phe Gln Asp Leu Gln Asp Glu Asp Val Ala Thr Phe
                   165                 170                 175
   Leu Ala Leu Ala Gln Asp Ala Leu Asp Met Thr Phe Gly Leu Thr Asp
               180                 185                 190
   Arg Pro Arg Leu Phe Gln Gln Phe Gly Leu Thr Lys Asp Thr Val Val
           195                 200                 205
   Leu Phe Lys Lys Phe Asp Glu Gly Arg Ala Asp Phe Pro Val Asp Glu
       210                 215                 220
   Glu Leu Gly Leu Asp Leu Gly Asp Leu Ser Arg Phe Leu Val Thr His
   225                 230                 235                 240
   Ser Met Arg Leu Val Thr Glu Phe Asn Ser Gln Thr Ser Ala Lys Ile
                   245                 250                 255
   Phe Ala Ala Arg Ile Leu Asn His Leu Leu Leu Phe Val Asn Gln Thr
               260                 265                 270
   Leu Ala Ala His Arg Glu Leu Leu Ala Gly Phe Gly Glu Ala Ala Pro
           275                 280                 285
   Arg Phe Arg Gly Gln Val Leu Phe Val Val Val Asp Val Ala Ala Asp
       290                 295                 300
   Asn Glu His Val Leu Gln Tyr Phe Gly Leu Lys Ala Glu Ala Ala Pro
   305                 310                 315                 320
   Thr Leu Arg Leu Val Asn Leu Glu Thr Thr Lys Lys Tyr Ala Pro Val
                   325                 330                 335
   Asp Gly Gly Pro Val Thr Ala Ala Ser Ile Thr Ala Phe Cys His Ala
               340                 345                 350
   Val Leu Asn Gly Gln Val Lys Pro Tyr Leu Leu Ser Gln Glu Ile Pro
           355                 360                 365
   Pro Asp Trp Asp Gln Arg Pro Val Lys Thr Leu Val Gly Lys Asn Phe
       370                 375                 380
   Glu Gln Val Ala Phe Asp Glu Thr Lys Asn Val Phe Val Lys Phe Tyr
   385                 390                 395                 400
   Ala Pro Trp Cys Thr His Cys Lys Glu Met Ala Pro Ala Trp Glu Ala
                   405                 410                 415
   Leu Ala Glu Lys Tyr Gln Asp His Glu Asp Ile Ile Ile Ala Glu Leu
               420                 425                 430
   Asp Ala Thr Ala Asn Glu Leu Asp Ala Phe Ala Val His Gly Phe Pro
           435                 440                 445
   Thr Leu Lys Tyr Phe Pro Ala Gly Pro Gly Arg Lys Val Ile Glu Tyr
       450                 455                 460
   Lys Ser Thr Arg Asp Leu Glu Thr Phe Ser Lys Phe Leu Asp Asn Gly
   465                 470                 475                 480
   Gly Val Leu Pro Thr Glu Glu Ser Pro Glu Glu Pro Ala Ala Pro Phe
                   485                 490                 495
   Pro Glu Pro Pro Ala Asn Ser Thr Met Gly Ser Lys Glu Glu Leu
               500                 505                 510
<210> 13
   <211> 2865
   <212> DNA
   <213> Homo sapiens
<220>
   <221> CDS
   <222> (46)...(1980)
<400> 13
   ccagcggccg ccgacgctag gaggccgcgc tccgcccccg ctacc atg agg ccc cgg      57
                                                     Met Arg Pro Arg
                                                     1
   aaa gcc ttc ctg ctc ctg ctg ctc ttg ggg ctg gtg cag ctg ctg gcc       105
   Lys Ala Phe Leu Leu Leu Leu Leu Leu Gly Leu Val Gln Leu Leu Ala
   5                   10                  15                  20
   gtg gcg ggt gcc gag ggc ccg gac gag gat tct tct aac aga gaa aat       153
   Val Ala Gly Ala Glu Gly Pro Asp Glu Asp Ser Ser Asn Arg Glu Asn
                   25                  30                  35
   gcc att gag gat gaa gag gag gag gag gag gaa gat gat gat gag gaa       201
   Ala Ile Glu Asp Glu Glu Glu Glu Glu Glu Glu Asp Asp Asp Glu Glu
               40                  45                  50
   gaa gac gac ttg gaa gtt aag gaa gaa aat gga gtc ttg gtc cta aat      249
   Glu Asp Asp Leu Glu Val Lys Glu Glu Asn Gly Val Leu Val Leu Asn
           55                  60                  65
   gat gca aac ttt gat aat ttt gtg gct gac aaa gac aca gtg ctg ctg      297
   Asp Ala Asn Phe Asp Asn Phe Val Ala Asp Lys Asp Thr Val Leu Leu
       70                  75                  80
   gag ttt tat gct cca tgg tgt gga cat tgc aag cag ttt gct ccg gaa      345
   Glu Phe Tyr Ala Pro Trp Cys Gly His Cys Lys Gln Phe Ala Pro Glu
   85                  90                  95                  100
   tat gaa aaa att gcc aac ata tta aag gat aaa gat cct ccc att cct      393
   Tyr Glu Lys Ile Ala Asn Ile Leu Lys Asp Lys Asp Pro Pro Ile Pro
                   105                 110                 115
   gtt gcc aag atc gat gca acc tca gcg tct gtg ctg gcc agc agg ttt      441
   Val Ala Lys Ile Asp Ala Thr Ser Ala Ser Val Leu Ala Ser Arg Phe
               120                 125                 130
   gat gtg agt ggc tac ccc acc atc aag atc ctt aag aag ggg cag gct      489
   Asp Val Ser Gly Tyr Pro Thr Ile Lys Ile Leu Lys Lys Gly Gln Ala
           135                 140                 145
   gta gac tac gag ggc tcc aga acc cag gaa gaa att gtt gcc aag gtc      537
   Val Asp Tyr Glu Gly Ser Arg Thr Gln Glu Glu Ile Val Ala Lys Val
       150                 155                 160
   aga gaa gtc tcc cag ccc gac tgg acg cct cca cca gaa gtc acg ctt      585
   Arg Glu Val Ser Gln Pro Asp Trp Thr Pro Pro Pro Glu Val Thr Leu
   165                 170                 175                 180
   gtg ttg acc aaa gag aac ttt gat gaa gtt gtg aat gat gca gat atc      633
   Val Leu Thr Lys Glu Asn Phe Asp Glu Val Val Asn Asp Ala Asp Ile
                   185                 190                 195
   att ctg gtg gag ttt tat gcc cca tgg tgt gga cac tgc aag aaa ctt      681
   Ile Leu Val Glu Phe Tyr Ala Pro Trp Cys Gly His Cys Lys Lys Leu
               200                 205                 210
   gcc ccc gag tat gag aag gcc gcc aag gag ctc agc aag cgt tct cct      729
   Ala Pro Glu Tyr Glu Lys Ala Ala Lys Glu Leu Ser Lys Arg Ser Pro
           215                 220                 225
   cca att ccc ctg gca aag gtc gac gcc acc gca gaa aca gac ctg gcc      777
   Pro Ile Pro Leu Ala Lys Val Asp Ala Thr Ala Glu Thr Asp Leu Ala
       230                 235                 240
   aag agg ttt gat gtc tct ggc tat ccc acc ctg aaa att ttc cgc aaa      825
   Lys Arg Phe Asp Val Ser Gly Tyr Pro Thr Leu Lys Ile Phe Arg Lys
   245                 250                 255                 260
   gga agg cct tat gac tac aac ggc cca cga gaa aaa tat gga atc gtt      873
   Gly Arg Pro Tyr Asp Tyr Asn Gly Pro Arg Glu Lys Tyr Gly Ile Val
                   265                 270                 275
   gat tac atg atc gag cag tcc ggg cct ccc tcc aag gag att ctg acc      921
   Asp Tyr Met Ile Glu Gln Ser Gly Pro Pro Ser Lys Glu Ile Leu Thr
               280                 285                 290
   ctg aag cag gtc cag gag ttc ctg aag gat gga gac gat gtc atc atc      969
   Leu Lys Gln Val Gln Glu Phe Leu Lys Asp Gly Asp Asp Val Ile Ile
           295                 300                 305
   atc ggg gtc ttt aag ggg gag agt gac cca gcc tac cag caa tac cag      1017
   Ile Gly Val Phe Lys Gly Glu Ser Asp Pro Ala Tyr Gln Gln Tyr Gln
       310                 315                 320
   gat gcc gct aac aac ctg aga gaa gat tac aaa ttt cac cac act ttc      1065
   Asp Ala Ala Asn Asn Leu Arg Glu Asp Tyr Lys Phe His His Thr Phe
   325                 330                 335                 340
   agc aca gaa ata gca aag ttc ttg aaa gtc tcc cag ggg cag ttg gtt      1113
   Ser Thr Glu Ile Ala Lys Phe Leu Lys Val Ser Gln Gly Gln Leu Val
                   345                 350                 355
   gta atg cag cct gag aaa ttc cag tcc aag tat gag ccc cgg agc cac      1161
   Val Met Gln Pro Glu Lys Phe Gln Ser Lys Tyr Glu Pro Arg Ser His
               360                 365                 370
   atg atg gac gtc cag ggc tcc acc cag gac tcg gcc atc aag gac ttc      1209
   Met Met Asp Val Gln Gly Ser Thr Gln Asp Ser Ala Ile Lys Asp Phe
           375                 380                 385
   gtg ctg aag tac gcc ctg ccc ctg gtt ggc cac cgc aag gtg tca aac      1257
   Val Leu Lys Tyr Ala Leu Pro Leu Val Gly His Arg Lys Val Ser Asn
       390                 395                 400
   gat gct aag cgc tac acc agg cgc ccc ctg gtg gtc gtc tac tac agt      1305
   Asp Ala Lys Arg Tyr Thr Arg Arg Pro Leu Val Val Val Tyr Tyr Ser
   405                 410                 415                 420
   gtg gac ttc agc ttt gat tac aga gct gca act cag ttt tgg cgg agc      1353
   Val Asp Phe Ser Phe Asp Tyr Arg Ala Ala Thr Gln Phe Trp Arg Ser
                   425                 430                 435
   aaa gtc cta gag gtg gcc aag gac ttc cct gag tac acc ttt gcc att      1401
   Lys Val Leu Glu Val Ala Lys Asp Phe Pro Glu Tyr Thr Phe Ala Ile
               440                 445                 450
   gcg gac gaa gag gac tat gct ggg gag gtg aag gac ctg ggg ctc agc      1449
   Ala Asp Glu Glu Asp Tyr Ala Gly Glu Val Lys Asp Leu Gly Leu Ser
           455                 460                 465
   gag agt ggg gag gat gtc aat gcc gcc atc ctg gac gag agt ggg aag      1497
   Glu Ser Gly Glu Asp Val Asn Ala Ala Ile Leu Asp Glu Ser Gly Lys
       470                 475                 480
   aag ttc gcc atg gag cca gag gag ttt gac tct gac acc ctc cgc gag      1545
   Lys Phe Ala Met Glu Pro Glu Glu Phe Asp Ser Asp Thr Leu Arg Glu
   485                 490                 495                 500
   ttt gtc act gct ttc aaa aaa gga aaa ctg aag cca gtc atc aaa tcc      1593
   Phe Val Thr Ala Phe Lys Lys Gly Lys Leu Lys Pro Val Ile Lys Ser
                   505                 510                 515
   cag cca gtg ccc aag aac aac aag gga ccc gtc aag gtc gtg gtg gga      1641
   Gln Pro Val Pro Lys Asn Asn Lys Gly Pro Val Lys Val Val Val Gly
               520                 525                 530
   aag acc ttt gac tcc att gtg atg gac ccc aag aag gac gtc ctc atc      1689
   Lys Thr Phe Asp Ser Ile Val Met Asp Pro Lys Lys Asp Val Leu Ile
           535                 540                 545
   gag ttc tac gcg cca tgg tgc ggg cac tgc aag cag cta gag ccc gtg      1737
   Glu Phe Tyr Ala Pro Trp Cys Gly His Cys Lys Gln Leu Glu Pro Val
       550                 555                 560
   tac aac agc ctg gcc aag aag tac aag ggc caa aag ggc ctg gtc atc      1785
   Tyr Asn Ser Leu Ala Lys Lys Tyr Lys Gly Gln Lys Gly Leu Val Ile
   565                 570                 575                 580
   gcc aag atg gac gcc act gcc aac gac gtc ccc agc gac cgc tat aag      1833
   Ala Lys Met Asp Ala Thr Ala Asn Asp Val Pro Ser Asp Arg Tyr Lys
                   585                 590                 595
   gtg gag ggc ttc ccc acc atc tac ttc gcc ccc agt ggg gac aaa aag      1881
   Val Glu Gly Phe Pro Thr Ile Tyr Phe Ala Pro Ser Gly Asp Lys Lys
               600                 605                 610
   aac cca gtt aaa ttt gag ggt gga gac aga gat ctg gag cat ttg agc      1929
   Asn Pro Val Lys Phe Glu Gly Gly Asp Arg Asp Leu Glu His Leu Ser
           615                 620                 625
   aag ttt ata gaa gaa cat gcc aca aaa ctg agc agg acc aag gaa gag      1977
   Lys Phe Ile Glu Glu His Ala Thr Lys Leu Ser Arg Thr Lys Glu Glu
       630                 635                 640
   ctt tgaaggcctg aggtctgcgg aaggtgggag gaggcagacg ccctgcgtgg          2030
   Leu
   645
   cccatggtcg gggcgtccac cggaggccgg caacaaacga cagtatctcg gattcctttt   2090
   tttttttttt taatttttta tactttgttg tttcacttca tgctctgaat actgaataac   2150
   catgaatgac tgaatagttt agtccagatt tttacagagg atacatctat ttttatcatt   2210
   atttggggtt tgaaaaattt ttttttacac cttctaattt ctttatttct caaagcagat   2270
   aattcttctg tgtgaaaatg ttttcttttt ttaatttaag gtttaaaatt ccttttccaa   2330
   atcatgttga ttttgctctt taaaaaaaaa aaaaaaaaaa aaaaaaaaaa aaaaaaaaga   2390
   agggctggga ccaaccgggt gagatccaca agtctctgga tgtggctgaa ggcaaataca   2450
   caattgaagt actttctgtt ttgaagtgct ttcccttttg aatctggttt gaaacatgca   2510
   gcttctgtct ctagcccaag gaaagaccaa aacataggga aataaaagca tttatctttg   2570
   tcttggaagt aattgttgaa gttgtgcagt tgatcagtgc acagttagct gcaatgttta   2630
   tagaaattga ttgttaaacc aaatttacac tggcatgtgt ggtgtagttt ctaaaaggca   2690
   cttcacattt gaaatttttc ttaccttaga aagtttctag tgatctaaat gtctagtttt   2750
   gtattctttt gtgtgtgttc actgtttctc agtattacca cttgaataat tctctgtaca   2810
   ggggggtttg tgctatacac tgggatgtct aattgcagca ataaagcctt tcttt         2865
<210> 14
   <211> 645
   <212> PRT
   <213> Homo sapiens
<400> 14
   Met Arg Pro Arg Lys Ala Phe Leu Leu Leu Leu Leu Leu Gly Leu Val
    1               5                  10                  15
   Gln Leu Leu Ala Val Ala Gly Ala Glu Gly Pro Asp Glu Asp Ser Ser
               20                  25                  30
   Asn Arg Glu Asn Ala Ile Glu Asp Glu Glu Glu Glu Glu Glu Glu Asp
           35                  40                  45
   Asp Asp Glu Glu Glu Asp Asp Leu Glu Val Lys Glu Glu Asn Gly Val
       50                  55                  60
   Leu Val Leu Asn Asp Ala Asn Phe Asp Asn Phe Val Ala Asp Lys Asp
   65                  70                  75                  80
   Thr Val Leu Leu Glu Phe Tyr Ala Pro Trp Cys Gly His Cys Lys Gln
                   85                  90                  95
   Phe Ala Pro Glu Tyr Glu Lys Ile Ala Asn Ile Leu Lys Asp Lys Asp
               100                 105                 110
   Pro Pro Ile Pro Val Ala Lys Ile Asp Ala Thr Ser Ala Ser Val Leu
           115                 120                 125
   Ala Ser Arg Phe Asp Val Ser Gly Tyr Pro Thr Ile Lys Ile Leu Lys
       130                 135                 140
   Lys Gly Gln Ala Val Asp Tyr Glu Gly Ser Arg Thr Gln Glu Glu Ile
   145                 150                 155                 160
   Val Ala Lys Val Arg Glu Val Ser Gln Pro Asp Trp Thr Pro Pro Pro
                   165                 170                 175
   Glu Val Thr Leu Val Leu Thr Lys Glu Asn Phe Asp Glu Val Val Asn
               180                 185                 190
   Asp Ala Asp Ile Ile Leu Val Glu Phe Tyr Ala Pro Trp Cys Gly His
           195                 200                 205
   Cys Lys Lys Leu Ala Pro Glu Tyr Glu Lys Ala Ala Lys Glu Leu Ser
       210                 215                 220
   Lys Arg Ser Pro Pro Ile Pro Leu Ala Lys Val Asp Ala Thr Ala Glu
   225                 230                 235                 240
   Thr Asp Leu Ala Lys Arg Phe Asp Val Ser Gly Tyr Pro Thr Leu Lys
                   245                 250                 255
   Ile Phe Arg Lys Gly Arg Pro Tyr Asp Tyr Asn Gly Pro Arg Glu Lys
               260                 265                 270
   Tyr Gly Ile Val Asp Tyr Met Ile Glu Gln Ser Gly Pro Pro Ser Lys
           275                 280                 285
   Glu Ile Leu Thr Leu Lys Gln Val Gln Glu Phe Leu Lys Asp Gly Asp
       290                 295                 300
   Asp Val Ile Ile Ile Gly Val Phe Lys Gly Glu Ser Asp Pro Ala Tyr
   305                 310                 315                 320
   Gln Gln Tyr Gln Asp Ala Ala Asn Asn Leu Arg Glu Asp Tyr Lys Phe
                   325                 330                 335
   His His Thr Phe Ser Thr Glu Ile Ala Lys Phe Leu Lys Val Ser Gln
               340                 345                 350
   Gly Gln Leu Val Val Met Gln Pro Glu Lys Phe Gln Ser Lys Tyr Glu
           355                 360                 365
   Pro Arg Ser His Met Met Asp Val Gln Gly Ser Thr Gln Asp Ser Ala
       370                 375                 380
   Ile Lys Asp Phe Val Leu Lys Tyr Ala Leu Pro Leu Val Gly His Arg
   385                 390                 395                 400
   Lys Val Ser Asn Asp Ala Lys Arg Tyr Thr Arg Arg Pro Leu Val Val
                   405                 410                 415
   Val Tyr Tyr Ser Val Asp Phe Ser Phe Asp Tyr Arg Ala Ala Thr Gln
               420                 425                 430
   Phe Trp Arg Ser Lys Val Leu Glu Val Ala Lys Asp Phe Pro Glu Tyr
           435                 440                 445
   Thr Phe Ala Ile Ala Asp Glu Glu Asp Tyr Ala Gly Glu Val Lys Asp
       450                 455                 460
   Leu Gly Leu Ser Glu Ser Gly Glu Asp Val Asn Ala Ala Ile Leu Asp
   465                 470                 475                 480
   Glu Ser Gly Lys Lys Phe Ala Met Glu Pro Glu Glu Phe Asp Ser Asp
                   485                 490                 495
   Thr Leu Arg Glu Phe Val Thr Ala Phe Lys Lys Gly Lys Leu Lys Pro
               500                 505                 510
   Val Ile Lys Ser Gln Pro Val Pro Lys Asn Asn Lys Gly Pro Val Lys
           515                 520                 525
   Val Val Val Gly Lys Thr Phe Asp Ser Ile Val Met Asp Pro Lys Lys
       530                 535                 540
   Asp Val Leu Ile Glu Phe Tyr Ala Pro Trp Cys Gly His Cys Lys Gln
   545                 550                 555                 560
   Leu Glu Pro Val Tyr Asn Ser Leu Ala Lys Lys Tyr Lys Gly Gln Lys
                   565                     570                     575
   Gly Leu Val Ile Ala Lys Met Asp Ala Thr Ala Asn Asp Val Pro Ser
               580                 585                     590
   Asp Arg Tyr Lys Val Glu Gly Phe Pro Thr Ile Tyr Phe Ala Pro Ser
           595                 600                     605
   Gly Asp Lys Lys Asn Pro Val Lys Phe Glu Gly Gly Asp Arg Asp Leu
       610                 615                     620
   Glu His Leu Ser Lys Phe Ile Glu Glu His Ala Thr Lys Leu Ser Arg
   625                 630                     635                 640
   Thr Lys Glu Glu Leu
                   645
<210> 15
   <211> 2412
   <212> DNA
   <213> Homo sapiens
<220>
   <221> CDS
   <222> (1)...(2409)
<400> 15
   atg agg gcc ctg tgg gtg ctg ggc ctc tgc tgc gtc ctg ctg acc ttc        48
   Met Arg Ala Leu Trp Val Leu Gly Leu Cys Cys Val Leu Leu Thr Phe
    1               5                  10                  15
   ggg tcg gtc aga gct gac gat gaa gtt gat gtg gat ggt aca gta gaa        96
   Gly Ser Val Arg Ala Asp Asp Glu Val Asp Val Asp Gly Thr Val Glu
               20                  25                  30
   gag gat ctg ggt aaa agt aga gaa gga tca agg acg gat gat gaa gta       144
   Glu Asp Leu Gly Lys Ser Arg Glu Gly Ser Arg Thr Asp Asp Glu Val
           35                  40                  45
   gta cag aga gag gaa gaa gct att cag ttg gat gga tta aat gca tca       192
   Val Gln Arg Glu Glu Glu Ala Ile Gln Leu Asp Gly Leu Asn Ala Ser
       50                  55                  60
   caa ata aga gaa ctt aga gag aag tcg gaa aag ttt gcc ttc caa gcc       240
   Gln Ile Arg Glu Leu Arg Glu Lys Ser Glu Lys Phe Ala Phe Gln Ala
   65                  70                  75                  80
   gaa gtt aac aga atg atg aaa ctt atc atc aat tca ttg tat aaa aat       288
   Glu Val Asn Arg Met Met Lys Leu Ile Ile Asn Ser Leu Tyr Lys Asn
                   85                  90                  95
   aaa gag att ttc ctg aga gaa ctg att tca aat gct tct gat gct tta       336
   Lys Glu Ile Phe Leu Arg Glu Leu Ile Ser Asn Ala Ser Asp Ala Leu
               100                 105                 110
   gat aag ata agg cta ata tca ctg act gat gaa aat gct ctt tct gga       384
   Asp Lys Ile Arg Leu Ile Ser Leu Thr Asp Glu Asn Ala Leu Ser Gly
           115                 120                 125
   aat gag gaa cta aca gtc aaa att aag tgt gat aag gag aag aac ctg       432
   Asn Glu Glu Leu Thr Val Lys Ile Lys Cys Asp Lys Glu Lys Asn Leu
       130                 135                 140
   ctg cat gtc aca gac acc ggt gta gga atg acc aga gaa gag ttg gtt       480
   Leu His Val Thr Asp Thr Gly Val Gly Met Thr Arg Glu Glu Leu Val
   145                 150                 155                 160
   aaa aac ctt ggt acc ata gcc aaa tct ggg aca agc gag ttt tta aac       528
   Lys Asn Leu Gly Thr Ile Ala Lys Ser Gly Thr Ser Glu Phe Leu Asn
                   165                 170                 175
   aaa atg act gaa gca cag gaa gat ggc cag tca act tct gaa ttg att       576
   Lys Met Thr Glu Ala Gln Glu Asp Gly Gln Ser Thr Ser Glu Leu Ile
               180                 185                 190
   ggc cag ttt ggt gtc ggt ttc tat tcc gcc ttc ctt gta gca gat aag       624
   Gly Gln Phe Gly Val Gly Phe Tyr Ser Ala Phe Leu Val Ala Asp Lys
           195                 200                 205
   gtt att gtc act tca aaa cac aac aac gat acc cag cac atc tgg gag       672
   Val Ile Val Thr Ser Lys His Asn Asn Asp Thr Gln His Ile Trp Glu
       210                 215                 220
   tct gac tcc aat gaa ttt tct gta att gct gac cca aga gga aac act       720
   Ser Asp Ser Asn Glu Phe Ser Val Ile Ala Asp Pro Arg Gly Asn Thr
   225                 230                 235                 240
   cta gga cgg gga acg aca att acc ctt gtc tta aaa gaa gaa gca tct       768
   Leu Gly Arg Gly Thr Thr Ile Thr Leu Val Leu Lys Glu Glu Ala Ser
                   245                 250                 255
   gat tac ctt gaa ttg gat aca att aaa aat ctc gtc aaa aaa tat tca       816
   Asp Tyr Leu Glu Leu Asp Thr Ile Lys Asn Leu Val Lys Lys Tyr Ser
               260                 265                 270
   cag ttc ata aac ttt cct att tat gta tgg agc agc aag act gaa act       864
   Gln Phe Ile Asn Phe Pro Ile Tyr Val Trp Ser Ser Lys Thr Glu Thr
           275                 280                 285
   gtt gag gag ccc atg gag gaa gaa gaa gca gcc aaa gaa gag aaa gaa       912
   Val Glu Glu Pro Met Glu Glu Glu Glu Ala Ala Lys Glu Glu Lys Glu
       290                 295                 300
   gaa tct gat gat gaa gct gca gta gag gaa gaa gaa gaa gaa aag aaa       960
   Glu Ser Asp Asp Glu Ala Ala Val Glu Glu Glu Glu Glu Glu Lys Lys
   305                 310                 315                 320
   cca aag act aaa aaa gtt gaa aaa act gtc tgg gac tgg gaa ctt atg      1008
   Pro Lys Thr Lys Lys Val Glu Lys Thr Val Trp Asp Trp Glu Leu Met
                   325                 330                 335
   aat gat atc aaa cca ata tgg cag aga cca tca aaa gaa gta gaa gaa      1056
   Asn Asp Ile Lys Pro Ile Trp Gln Arg Pro Ser Lys Glu Val Glu Glu
               340                 345                 350
   gat gaa tac aaa gct ttc tac aaa tca ttt tca aag gaa agt gat gac      1104
   Asp Glu Tyr Lys Ala Phe Tyr Lys Ser Phe Ser Lys Glu Ser Asp Asp
           355                 360                 365
   ccc atg gct tat att cac ttt act gct gaa ggg gaa gtt acc ttc aaa      1152
   Pro Met Ala Tyr Ile His Phe Thr Ala Glu Gly Glu Val Thr Phe Lys
       370                 375                 380
   tca att tta ttt gta ccc aca tct gct cca cgt ggt ctg ttt gac gaa      1200
   Ser Ile Leu Phe Val Pro Thr Ser Ala Pro Arg Gly Leu Phe Asp Glu
   385                 390                 395                 400
   tat gga tct aaa aag agc gat tac att aag ctc tat gtg cgc cgt gta      1248
   Tyr Gly Ser Lys Lys Ser Asp Tyr Ile Lys Leu Tyr Val Arg Arg Val
                   405                 410                 415
   ttc atc aca gac gac ttc cat gat atg atg cct aaa tac ctc aat ttt      1296
   Phe Ile Thr Asp Asp Phe His Asp Met Met Pro Lys Tyr Leu Asn Phe
               420                 425                 430
   gtc aag ggt gtg gtg gac tca gat gat ctc ccc ttg aat gtt tcc cgc      1344
   Val Lys Gly Val Val Asp Ser Asp Asp Leu Pro Leu Asn Val Ser Arg
           435                 440                 445
   gag act ctt cag caa cat aaa ctg ctt aag gtg att agg aag aag ctt      1392
   Glu Thr Leu Gln Gln His Lys Leu Leu Lys Val Ile Arg Lys Lys Leu
       450                 455                 460
   gtt cgt aaa acg ctg gac atg atc aag aag att gct gat gat aaa tac      1440
   Val Arg Lys Thr Leu Asp Met Ile Lys Lys Ile Ala Asp Asp Lys Tyr
   465                 470                 475                 480
   aat gat act ttt tgg aaa gaa ttt ggt acc aac atc aag ctt ggt gtg      1488
   Asn Asp Thr Phe Trp Lys Glu Phe Gly Thr Asn Ile Lys Leu Gly Val
                   485                 490                 495
   att gaa gac cac tcg aat cga aca cgt ctt gct aaa ctt ctt agg ttc      1536
   Ile Glu Asp His Ser Asn Arg Thr Arg Leu Ala Lys Leu Leu Arg Phe
               500                 505                 510
   cag tct tct cat cat cca act gac att act agc cta gac cag tat gtg      1584
   Gln Ser Ser His His Pro Thr Asp Ile Thr Ser Leu Asp Gln Tyr Val
           515                 520                 525
   gaa aga atg aag gaa aaa caa gac aaa atc tac ttc atg gct ggg tcc      1632
   Glu Arg Met Lys Glu Lys Gln Asp Lys Ile Tyr Phe Met Ala Gly Ser
       530                 535                 540
   agc aga aaa gag gct gaa tct tct cca ttt gtt gag cga ctt ctg aaa      1680
   Ser Arg Lys Glu Ala Glu Ser Ser Pro Phe Val Glu Arg Leu Leu Lys
   545                 550                 555                 560
   aag ggc tat gaa gtt att tac ctc aca gaa cct gtg gat gaa tac tgt      1728
   Lys Gly Tyr Glu Val Ile Tyr Leu Thr Glu Pro Val Asp Glu Tyr Cys
                   565                 570                 575
   att cag gcc ctt ccc gaa ttt gat ggg aag agg ttc cag aat gtt gcc      1776
   Ile Gln Ala Leu Pro Glu Phe Asp Gly Lys Arg Phe Gln Asn Val Ala
               580                 585                 590
   aag gaa gga gtg aag ttc gat gaa agt gag aaa act aag gag agt cgt      1824
   Lys Glu Gly Val Lys Phe Asp Glu Ser Glu Lys Thr Lys Glu Ser Arg
           595                 600                 605
   gaa gca gtt gag aaa gaa ttt gag cct ctg ctg aat tgg atg aaa gat      1872
   Glu Ala Val Glu Lys Glu Phe Glu Pro Leu Leu Asn Trp Met Lys Asp
       610                 615                 620
   aaa gcc ctt aag gac aag att gaa aag gct gtg gtg tct cag cgc ctg      1920
   Lys Ala Leu Lys Asp Lys Ile Glu Lys Ala Val Val Ser Gln Arg Leu
   625                 630                 635                 640
   aca gaa tct ccg tgt gct ttg gtg gcc agc cag tac gga tgg tct ggc      1968
   Thr Glu Ser Pro Cys Ala Leu Val Ala Ser Gln Tyr Gly Trp Ser Gly
                   645                 650                 655
   aac atg gag aga atc atg aaa gca caa gcg tac caa acg ggc aag gac      2016
   Asn Met Glu Arg Ile Met Lys Ala Gln Ala Tyr Gln Thr Gly Lys Asp
               660                 665                 670
   atc tct aca aat tac tat gcg agt cag aag aaa aca ttt gaa att aat      2064
   Ile Ser Thr Asn Tyr Tyr Ala Ser Gln Lys Lys Thr Phe Glu Ile Asn
           675                 680                 685
   ccc aga cac ccg ctg atc aga gac atg ctt cga cga att aag gaa gat      2112
   Pro Arg His Pro Leu Ile Arg Asp Met Leu Arg Arg Ile Lys Glu Asp
       690                 695                 700
   gaa gat gat aaa aca gtt ttg gat ctt gct gtg gtt ttg ttt gaa aca      2160
   Glu Asp Asp Lys Thr Val Leu Asp Leu Ala Val Val Leu Phe Glu Thr
   705                 710                 715                 720
   gca acg ctt cgg tca ggg tat ctt tta cca gac act aaa gca tat gga      2208
   Ala Thr Leu Arg Ser Gly Tyr Leu Leu Pro Asp Thr Lys Ala Tyr Gly
                   725                 730                 735
   gat aga ata gaa aga atg ctt cgc ctc agt ttg aac att gac cct gat      2256
   Asp Arg Ile Glu Arg Met Leu Arg Leu Ser Leu Asn Ile Asp Pro Asp
               740                 745                 750
   gca aag gtg gaa gaa gag cct gaa gaa gaa cct gaa gag aca gca gaa      2304
   Ala Lys Val Glu Glu Glu Pro Glu Glu Glu Pro Glu Glu Thr Ala Glu
           755                 760                 765
   gac aca aca gaa gac aca gag caa gac gaa gat gaa gaa atg gat gtg      2352
   Asp Thr Thr Glu Asp Thr Glu Gln Asp Glu Asp Glu Glu Met Asp Val
       770                 775                 780
   gga aca gat gaa gaa gaa gaa aca gca aag gaa tct aca gct gaa aaa      2400
   Gly Thr Asp Glu Glu Glu Glu Thr Ala Lys Glu Ser Thr Ala Glu Lys
   785                 790                 795                 800
   gat gaa ttg taa                                                      2412
   Asp Glu Leu
<210> 16
   <211> 803
   <212> PRT
   <213> Homo sapiens
<400> 16
   Met Arg Ala Leu Trp Val Leu Gly Leu Cys Cys Val Leu Leu Thr Phe
    1               5                  10                  15
   Gly Ser Val Arg Ala Asp Asp Glu Val Asp Val Asp Gly Thr Val Glu
               20                  25                  30
   Glu Asp Leu Gly Lys Ser Arg Glu Gly Ser Arg Thr Asp Asp Glu Val
           35                  40                  45
   Val Gln Arg Glu Glu Glu Ala Ile Gln Leu Asp Gly Leu Asn Ala Ser
       50                  55                  60
   Gln Ile Arg Glu Leu Arg Glu Lys Ser Glu Lys Phe Ala Phe Gln Ala
   65                  70                  75                  80
   Glu Val Asn Arg Met Met Lys Leu Ile Ile Asn Ser Leu Tyr Lys Asn
                   85                      90                      95
   Lys Glu Ile Phe Leu Arg Glu Leu Ile Ser Asn Ala Ser Asp Ala Leu
               100                     105                     110
   Asp Lys Ile Arg Leu Ile Ser Leu Thr Asp Glu Asn Ala Leu Ser Gly
           115                     120                     125
   Asn Glu Glu Leu Thr Val Lys Ile Lys Cys Asp Lys Glu Lys Asn Leu
       130                     135                     140
   Leu His Val Thr Asp Thr Gly Val Gly Met Thr Arg Glu Glu Leu Val
   145                     150                     155                     160
   Lys Asn Leu Gly Thr Ile Ala Lys Ser Gly Thr Ser Glu Phe Leu Asn
                   165                     170                     175
   Lys Met Thr Glu Ala Gln Glu Asp Gly Gln Ser Thr Ser Glu Leu Ile
               180                     185                     190
   Gly Gln Phe Gly Val Gly Phe Tyr Ser Ala Phe Leu Val Ala Asp Lys
           195                     200                     205
   Val Ile Val Thr Ser Lys His Asn Asn Asp Thr Gln His Ile Trp Glu
       210                     215                     220
   Ser Asp Ser Asn Glu Phe Ser Val Ile Ala Asp Pro Arg Gly Asn Thr
   225                     230                     235                     240
   Leu Gly Arg Gly Thr Thr Ile Thr Leu Val Leu Lys Glu Glu Ala Ser
                   245                     250                     255
   Asp Tyr Leu Glu Leu Asp Thr Ile Lys Asn Leu Val Lys Lys Tyr Ser
               260                     265                     270
   Gln Phe Ile Asn Phe Pro Ile Tyr Val Trp Ser Ser Lys Thr Glu Thr
           275                     280                     285
   Val Glu Glu Pro Met Glu Glu Glu Glu Ala Ala Lys Glu Glu Lys Glu
       290                     295                     300
   Glu Ser Asp Asp Glu Ala Ala Val Glu Glu Glu Glu Glu Glu Lys Lys
   305                     310                     315                     320
   Pro Lys Thr Lys Lys Val Glu Lys Thr Val Trp Asp Trp Glu Leu Met
                   325                     330                     335
   Asn Asp Ile Lys Pro Ile Trp Gln Arg Pro Ser Lys Glu Val Glu Glu
               340                     345                     350
   Asp Glu Tyr Lys Ala Phe Tyr Lys Ser Phe Ser Lys Glu Ser Asp Asp
           355                     360                     365
   Pro Met Ala Tyr Ile His Phe Thr Ala Glu Gly Glu Val Thr Phe Lys
       370                     375                     380
   Ser Ile Leu Phe Val Pro Thr Ser Ala Pro Arg Gly Leu Phe Asp Glu
   385                     390                     395                     400
   Tyr Gly Ser Lys Lys Ser Asp Tyr Ile Lys Leu Tyr Val Arg Arg Val
                   405                     410                     415
   Phe Ile Thr Asp Asp Phe His Asp Met Met Pro Lys Tyr Leu Asn Phe
               420                     425                     430
   Val Lys Gly Val Val Asp Ser Asp Asp Leu Pro Leu Asn Val Ser Arg
           435                     440                     445
   Glu Thr Leu Gln Gln His Lys Leu Leu Lys Val Ile Arg Lys Lys Leu
       450                     455                     460
   Val Arg Lys Thr Leu Asp Met Ile Lys Lys Ile Ala Asp Asp Lys Tyr
   465                     470                     475                     480
   Asn Asp Thr Phe Trp Lys Glu Phe Gly Thr Asn Ile Lys Leu Gly Val
                   485                     490                     495
   Ile Glu Asp His Ser Asn Arg Thr Arg Leu Ala Lys Leu Leu Arg Phe
               500                     505                     510
   Gln Ser Ser His His Pro Thr Asp Ile Thr Ser Leu Asp Gln Tyr Val
           515                     520                     525
   Glu Arg Met Lys Glu Lys Gln Asp Lys Ile Tyr Phe Met Ala Gly Ser
       530                     535                     540
   Ser Arg Lys Glu Ala Glu Ser Ser Pro Phe Val Glu Arg Leu Leu Lys
   545                     550                     555                     560
   Lys Gly Tyr Glu Val Ile Tyr Leu Thr Glu Pro Val Asp Glu Tyr Cys
                   565                     570                     575
   Ile Gln Ala Leu Pro Glu Phe Asp Gly Lys Arg Phe Gln Asn Val Ala
               580                 585                 590
   Lys Glu Gly Val Lys Phe Asp Glu Ser Glu Lys Thr Lys Glu Ser Arg
           595                 600                 605
   Glu Ala Val Glu Lys Glu Phe Glu Pro Leu Leu Asn Trp Met Lys Asp
       610                 615                 620
   Lys Ala Leu Lys Asp Lys Ile Glu Lys Ala Val Val Ser Gln Arg Leu
   625                 630                 635                 640
   Thr Glu Ser Pro Cys Ala Leu Val Ala Ser Gln Tyr Gly Trp Ser Gly
                   645                 650                 655
   Asn Met Glu Arg Ile Met Lys Ala Gln Ala Tyr Gln Thr Gly Lys Asp
               660                 665                 670
   Ile Ser Thr Asn Tyr Tyr Ala Ser Gln Lys Lys Thr Phe Glu Ile Asn
           675                 680                 685
   Pro Arg His Pro Leu Ile Arg Asp Met Leu Arg Arg Ile Lys Glu Asp
       690                 695                 700
   Glu Asp Asp Lys Thr Val Leu Asp Leu Ala Val Val Leu Phe Glu Thr
   705                 710                 715                 720
   Ala Thr Leu Arg Ser Gly Tyr Leu Leu Pro Asp Thr Lys Ala Tyr Gly
                   725                 730                 735
   Asp Arg Ile Glu Arg Met Leu Arg Leu Ser Leu Asn Ile Asp Pro Asp
               740                 745                 750
   Ala Lys Val Glu Glu Glu Pro Glu Glu Glu Pro Glu Glu Thr Ala Glu
           755                 760                 765
   Asp Thr Thr Glu Asp Thr Glu Gln Asp Glu Asp Glu Glu Met Asp Val
       770                 775                 780
   Gly Thr Asp Glu Glu Glu Glu Thr Ala Lys Glu Ser Thr Ala Glu Lys
   785                 790                 795                 800
   Asp Glu Leu
<210> 17
   <211> 21
   <212> DNA
   <213> Homo sapiens
<400> 17
   aacaactgca tgggtaacct t                                            21

## Description

### TECHNICAL FIELD

This invention relates to methods and compositions for modulating apoptosis by selectively targeting glucose regulated proteins (GRPs) and more particularly to modulating the activity and/or interaction of GRP78 and procaspase.

### BACKGROUND

Resistance to chemotherapy remains a major obstacle for the treatment of cancer. The complexity of drug resistance in human cancer strongly suggests the involvement of multiple pathways. One mechanism, both intrinsic and acquired, is the result of genetic alterations within cancer cells. Another mechanism may result from environmental conditions that occur naturally in solid tumors. Because of poor vascularization, solid tumors usually contain regions undergoing glucose starvation and hypoxia, resulting in acidosis and alterations in cell metabolism. These pockets of hypoxia and nutrient deprivation occur in well differentiated, slow growing, non-metastatic tumors, as well as in rapidly growing, aggressive anaplastic malignancies.

Stress conditions in cell culture, such as glucose starvation, commonly cause the glucose-regulated stress response which, is part of a general cellular defense mechanism referred to as the unfolded protein response (UPR). One characteristic of the UPR is the induction of the endoplasmic reticulum (ER) resident stress proteins referred to as the glucose-regulated proteins (GRPs). The GRPs are ca²⁺-binding chaperone proteins with protective properties. The best characterized GRP is GRP78, a 78-kDa protein also referred to as BiP. As a protein chaperone, GRP78 is known to form complexes with heterologous proteins that are processed through the ER.

Glucose Regulated Proteins, or GRPs (GRP74, GRP78, GRP94, GRP170, ERp72, PDI, calreticulin, and GRP58 (alias ERp57)) are ER molecular chaperones that assist in protein folding and assembly. GRP78, GRP94, ERp72 and calreticulin are also Ca2+ binding proteins. GRP78 and GRP94 share sequence homology with heat shock proteins. The GRP family of proteins is coordinately induced by glucose starvation, anoxia, alterations in intracellular calcium and, exposure to inhibitors of glycosylation as well as by PDT-mediated oxidative stress (Gomer, et al., Cancer Res. 51:6574-79, 1991; and Li, et al., J. Cell Physiol. 153:575-82, 1992). The 78,000 GRP (i.e., GRP78) is identical in sequence to the immunoglobulin heavy chain binding protein and both GRP78 and GRP94 are localized in the ER.

Many of the cytotoxic drugs, including topoisomerase inhibitors such as etoposide, initiate programmed cell death. DNA damaging agents such as etoposide can trigger cell death through the p53-mediated caspase cell death signaling cascade, resulting in cytochrome c release and the activation of caspase-3. Caspases-3, -6, and -7 are members of the apoptotic executing group of caspases with caspase-7 structurally and functionally most similar to caspase-3. Active caspase-7 has been shown to be associated with the mitochondria and the ER membranes, whereas caspase-3 remains cytosolic. Although these observations suggest that similar apoptotic executioner's function in different cellular compartments and act on distinct substrates, there is limited information on the contribution of organelles such as the ER in the apoptotic process.

Accordingly, there exists a need in the art to identify key interactions between proteins involved in the apoptotic pathway and to regulate those interactions.

### SUMMARY

Overexpression and antisense approaches in cell systems show that GRP78 can protect cells against cell death caused by disturbance of ER homeostasis. Whereas GRP78 overexpression could limit damage in normal tissues and organs exposed to ER stress, the anti-apoptotic function of GRP78 also predicts that its natural induction in neoplastic cells could lead to cancer progression and drug resistance. In a variety of cancer cell lines, solid tumors, and human cancer biopsies, the level of GRP78 is elevated, correlating with malignancy. Using human cancer and other cell lines, a large number of stress induction studies show that a glucose-regulated stress response results in the induction of GRP78 and other coordinately regulated GRP genes correlating with cellular drug resistance. Nonetheless, the direct role of GRPs in conferring drug resistance has not been proven. This is because of the inherent problems associated with using stress inducers or deficiencies in certain cell functions to induce the GRPs, because the inducing conditions can exert other unknown pleiotropic effects, possibly affecting multiple cellular pathways. Furthermore, the mechanisms for the protective function of the ER localized GRPs in drug resistance are not understood.

Methods and compositions for modulating apoptosis by regulating the physical and functional interactions of glucose responsive protein 78 (GRP78) with cytosolic components of a cell that mediate apoptosis are provided. The methods and compositions are particularly well suited to identifying agents that can be used in conjunction with apoptosis-inducing therapeutic compounds to treat cell proliferative disorders. Thus, the disclosure relates to the preparation of pharmaceutical compositions for treating, preventing, and/or delaying a disease in a subject, such as, for example, a cell proliferative disorder.

The invention provides a method of modulating apoptosis in a cell in vitro, the method comprising contacting a glucose regulated protein 78(GRP78) with a peptide which inhibits the interaction of the GRP78 with a procaspase-7 that mediates apoptosis, wherein the peptide is a soluble domain of GRP78 and interacts with the ATP-binding domain of the GRP78 protein.

The details of one or more embodiments of the invention are set forth in the accompanying drawings and the description below. Other features, objects, and advantages of the invention will be apparent from the description and drawings, and from the claims.

### DESCRIPTION OF DRAWINGS

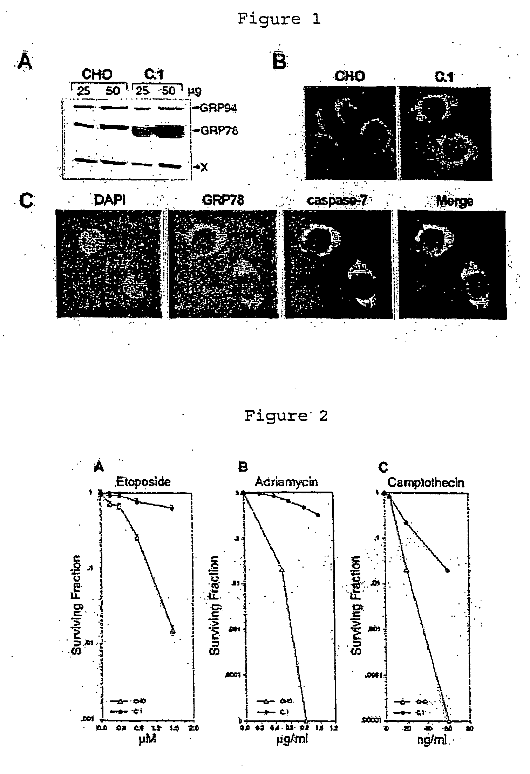

**Figure 1****, Panel A** depicts a comparison of GRP78 protein levels in parental CHO cell line and its derivative C.1. Total protein lysates (25 and 50 µg/lane) were separated on 8% SDS-PAGE, and the levels of GRP78, GRP94, and protein X were determined by immunoblotting with an anti-KDEL antibody.

**Figure 1****, Panel B** depicts GRP78 localization by immunofluorescence. Subcellular distribution of GRP78 is primarily perinuclear, indicative of ER localization.

**Figure 1****, Panel C** depicts co-localization of GRP78 and caspase-7 in the ER. Green fluorescence indicates caspase-7, red indicates GRP78, and purple staining represents the nucleus. In the merged images, yellow staining indicates co-localization.

**Figure 2****, Panel A** depicts clonogenic survival assays for CHO and C.1 cells subjected to various concentrations of etoposide for 6 h.

**Figure 2****, Panel B** depicts clonogenic survival assays for CHO and C.1 cells subjected to various concentrations of adriamycin for 1 h.

**Figure 2****, Panel C** depicts clonogenic survival assays for CHO and C.1 cells subjected to various concentrations of camptothecin for 24 h.

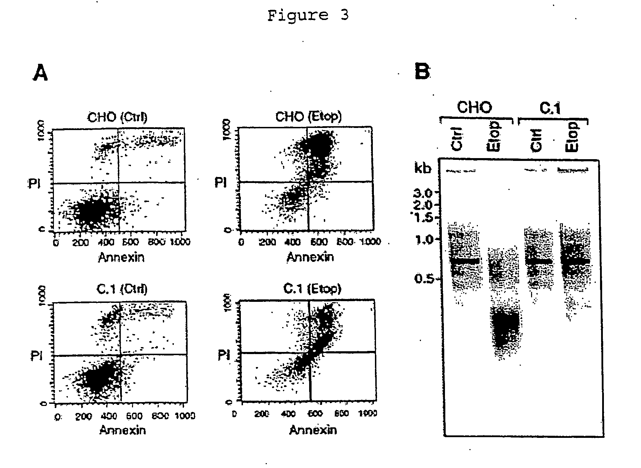

**Figure 3****, Panel A** depicts GRP78 overexpression protecting cells from etoposide-induced apoptosis. C.1 and CHO cells were either non-treated (Ctrl) or treated with 30 µM etoposide (Etop) for 6 h. Viable cells are those with low annexin or no annexin and PI staining (lower left panel). Early stage apoptotic cells are represented by high annexin and low PI staining (lower right panel), later stage apoptotic cells represented by high annexin and high PI staining (upper right panel), and necrosis is represented by cells with high PI and low annexin staining (upper left panel).

**Figure 3****, Panel B** depicts DNA fragmentation pattern of CHO and C.1 cells following etoposide treatment.

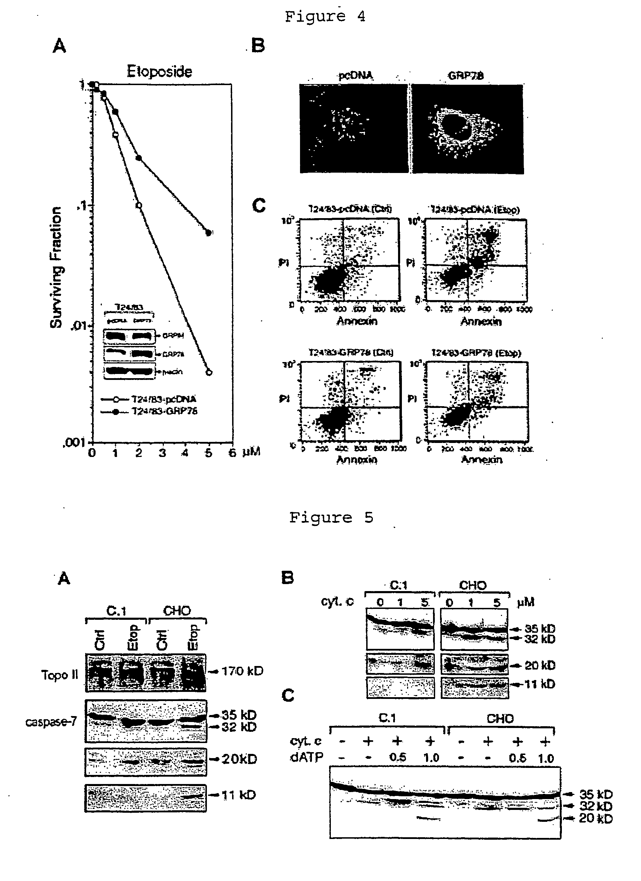

**Figure 4****, Panel A** depicts overexpression of GRP78 confers etoposide resistance to human bladder carcinoma T24/83 cells. T24/83 cell lines stably transfected with the empty vector pcDNA (T24/83-pcDNA) or vector expressing wild-type GRP78 (T24/83-GRP78) were established. Immunoblot analysis of GRP78 protein level for the two cell lines is shown (inset).

**Figure 4****, Panel B** depicts immunofluorescence imaging of GRP78 expression in T24/83 cells. GRP78 localization is perinuclear.

**Figure 4****, Panel C** depicts the effect of overexpression of GRP78 on etoposide-induced apoptosis. T24/83-pcDNA and T24/83-GRP78 cells were either non-treated (Ctrl) or treated with etoposide (Etop).

**Figure 5****, Panel A** depicts the effect of GRP78 overexpression on topoisomerase II expression and caspase-7 activation. Total protein lysates were prepared from non-treated (Ctrl) or etoposide-treated (Etop) CHO and C.1 cells.

**Figure 5****, Panel B** depicts overexpression of GRP78 as inhibiting *in vitro* activation of caspase-7.

**Figure 5****, Panel C** depicts cytoplasmic extracts prepared from CHO and C.1 cells and incubated with (+) or without (-) 10 µM cytochrome c and the various amounts of dATP (mM) as indicated.

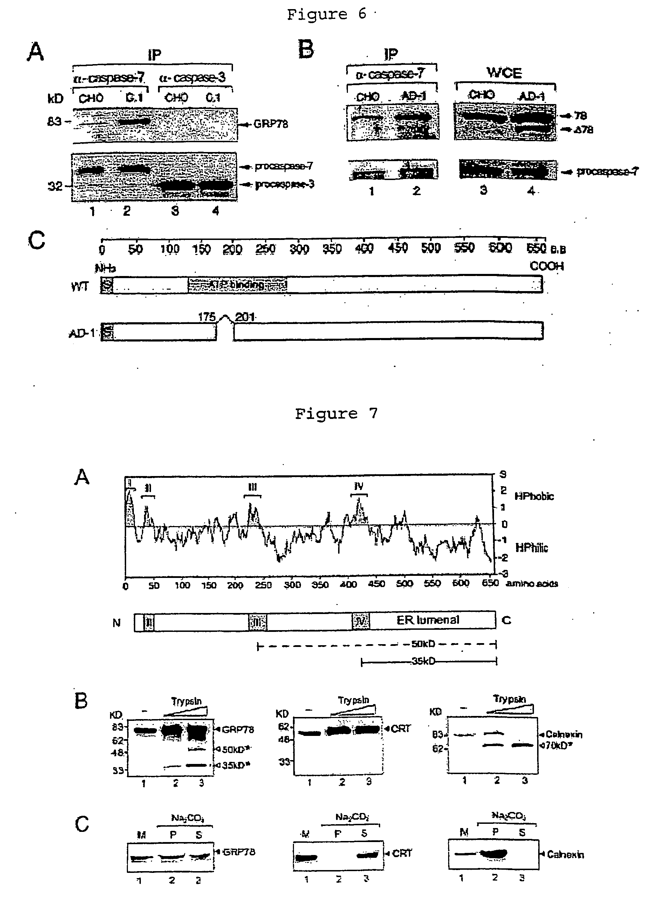

**Figure 6****, Panel A** depicts cell lysates from CHO and C.1 cells immunoprecipitated with anti-caspase-7 (lanes 1 and 2) or anti-caspase-3 (lanes 3 and 4) antibodies.

**Figure 6****, Panel B** depicts cell lysates in extraction buffer from CHO and AD-1 cells were immunoprecipitated with anti-caspase-7 antibody (lanes 1 and 2). Whole cell extracts (WCE) from CHO and AD-1 cells were immunoblotted in parallel (lanes 3 and 4). The positions of GRP78, procaspase-7, and the deletion mutant form of GRP78 (Δ78) are indicated.

**Figure 6****, Panel C** depicts a schematic drawing of wild-type (WT) GRP78 and AD-1 showing the location of the signal sequence (S), the ATP-binding domain, and the AD-1 deletion spanning amino acids 175 to 201.

**Figure 7****, Panel A** depicts a hydropathicity plot of GRP78 as generated using the Kyte-Doolittle method with a window size of 17. Four putative hydrophobic domains (I-IV) are identified. Represented below is a schematic drawing of the mature GRP78 protein with the hydrophobic domains IV and III as putative transmembrane domains generating carboxyl 35-and 50-kDa trypsin resistant fragments.

**Figure 7****, Panel B** depicts limited trypsin digestion. Isolated microsomes from C.1 cells were either non-treated (lane 1) or subjected to trypsin digestion at the concentration of 0.01% (lane 2) or 0.05% (lane 2). At the end of the reaction, the amount of GRP78 was detected by Western blotting using the rabbit polyclonal anti-GRP78 antibody recognizing the carboxyl terminus (StressGen, Victoria, Canada) (left panel). The full-length GRP78 band is indicated by a closed arrow, and the 35- and 50-kDa proteolytic products are indicated by open arrows highlighted with a star. The same membrane was stripped and re-probed with a rabbit polyclonal anti-calreticulin (CRT) antibody (middle panel) or a rabbit polyclonal anti-calnexin antibody recognizing the amino terminus of calnexin (right panel). The full-length CRT and calnexin are indicated by closed arrows, and the 70-kDa proteolytic product of calnexin is indicated by an open arrow highlighted by a star.

**Figure 7****, Panel C** depicts sodium carbonate extraction. The microsome (M) fraction was either non-treated (lane 1) or treated with 100 mM sodium carbonate and separated into pellet (P) and supernatant (S) fractions (lanes 2 and 3, respectively). The protein samples from each fraction were separated by 10% SDS-PAGE and subjected to Western blotting with rabbit anti-GRP78 antibody (left panel), rabbit anti-calreticulin antibody (middle panel), and rabbit anti-calnexin antibody (right panel).

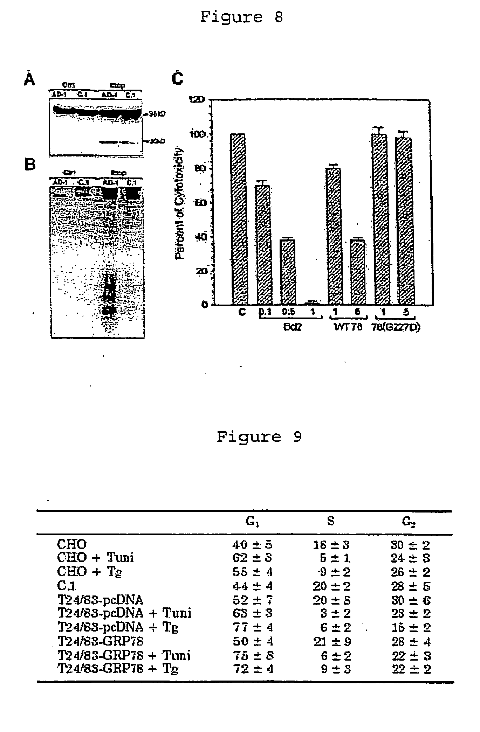

**Figure 8****, Panel A** depicts cytoplasmic extracts (50 µg/lane) prepared from non-treated (Ctrl) and etoposide-treated (Etop) AD-1 and C.1 cells as separated on 10% SDS-PAGE and immunoblotted using anticaspase-7 antibody.

**Figure 8****, Panel B** depicts the DNA fragmentation pattern of C.1 and AD-1 cells following etoposide treatment.

**Figure 8****, Panel C** depicts cell death assays indicating that the ATP-binding domain of GRP78 is necessary for protection against etoposide-induced cell death. The S.D. is shown.

**Figure 9** depicts the relative cell cycle distribution of GRP-78. The percentage of cells in G1, G2, and S phase was determined.

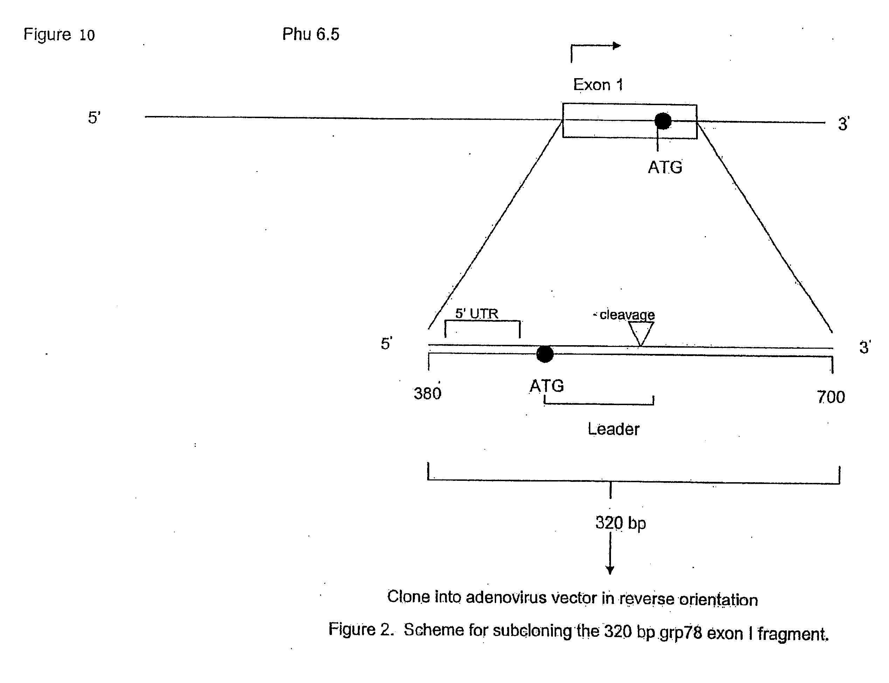

**Figure 10** is a schematic showing the subcloning of the 320 bp grp78 exon 1 fragment.

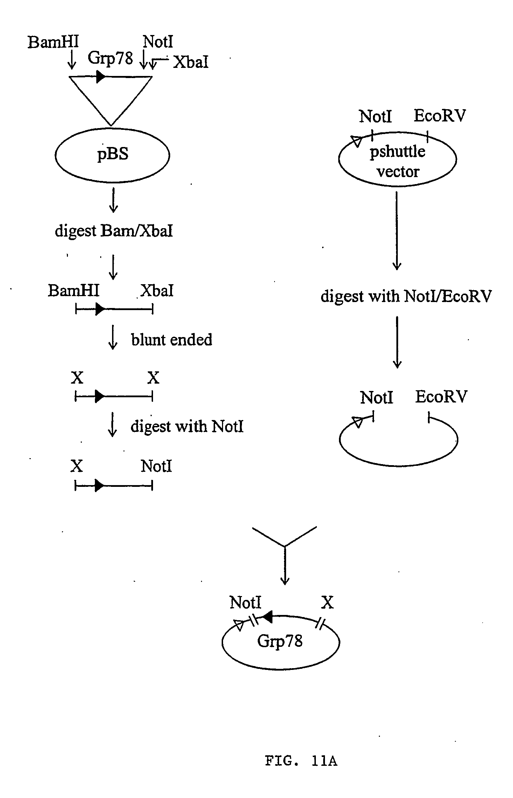

**Figure 11A-C** shows the schemes used for subcloning. (A) shows the scheme for the subcloning of full-length His-tagged GRP78 into pShuttle CMV adenovirus; (B) shows the scheme for the subcloning of full-length His-Tagged GRP78 Full-length antisense (AS); and (C) shows the scheme used for the subcloning of GRP78 (320BP) antisense (AS) into PShuttle CMV adenovirus.

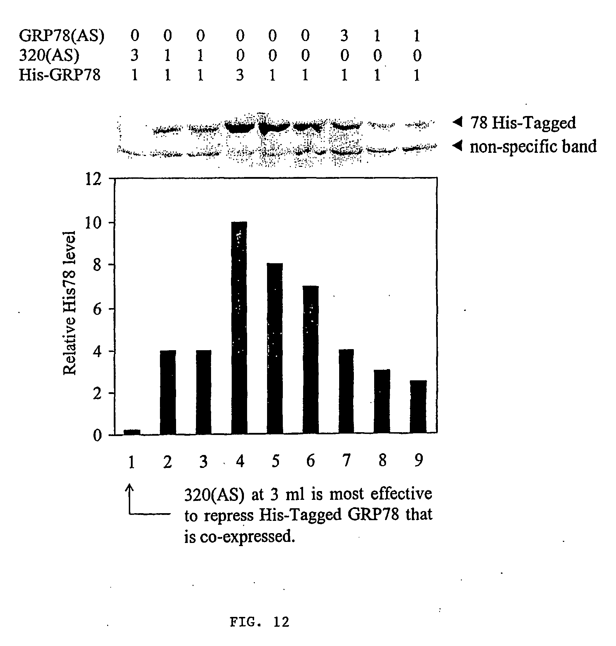

**Figure 12** shows the expression of His-tagged GRP78, GRP78(AS) and 320(AS) in adenovirus infected 293 T cells.

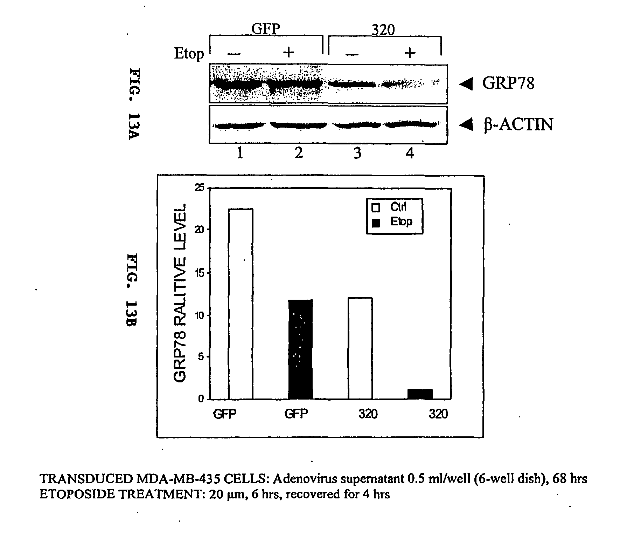

**Figure 13A** and **B** shows the results of transduced MDA-MB-435 cells treated with etoposide in the presence and absence of the 320(AS).

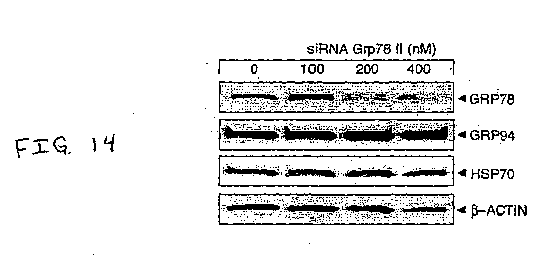

**Figure 14** shows a Western blot of 293T cells. The concentrations of siRNA Grp78 II used were indicated on top.

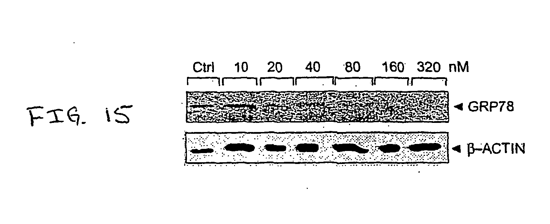

**Figure 15** shows a Western blot of MDA-MB-435 cells. The concentrations of siRNA Grp78 II used were indicated on top.

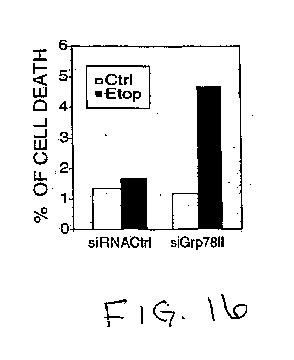

**Figure 16** shows suppression of GRP78 by siRNA oligonucleotides results in enhanced sensitivity to etoposide-mediated cell death in breast cancer cells.

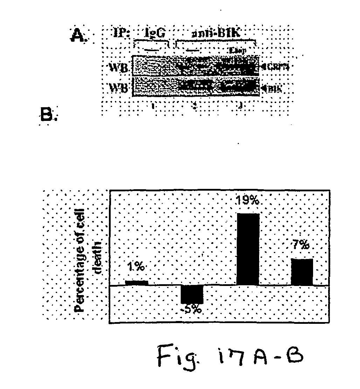

**Figure 17A-B** show the physical and functional interaction between GRP BIK. (A) 293T cells were either not treated (lanes 1, 2) or treated with 50 uM etoposide for 24 hours (lane 3). Cells were harvested and cells lysate immunoprecipitated with either goat IgG (lane 1) or goat anti-BlK antibody (lanes 2, 3). Western blots with anti-BlK antibody and anti-GRP78 antibody show co-immunoprecipitation of endogenous GRP78 with Blk. (B) 293T cells were transiently transfected with either pcDNA, CMV promoter driven expression vector for His-tagged GRP78, expression vector for Flag-tagged Glk-b5TM alone or in combination as indicated. After 48 hours, cell death was determined by trypan blue exclusion assay. The level of apoptosis observed in cells transfected with pcDNA3 was set as 1. The results showed overexpression of GRP78 protects 293T cells from death induced by transient transfection of ER-targeted Blk-b5tM.

### DETAILED DESCRIPTION

Within the microenvironment of a solid tumor, unique stress conditions can lead to induction of GRPs. The data and invention described herein indicate that GRPs act as anti-apoptotic proteins. The GRP-mediated protection involves GRP interaction with effectors of apoptosis, leading to the blockage of cell death induced by drug treatment.

Thus, in accordance with the invention, GRPs (*e.g*., GRP74, GRP78 and GRP94) represent rational targets for chemotherapeutics, immunotherapeutics, antisense, ribozymes, siRNA and vaccines relevant to the treatment of cell proliferative diseases such as cancer. In view of their function as molecular chaperones, the GRPs (*e.g*., GRP78 and 94) further represent rational targets for the development of therapeutics for tissue injury and stress, such as can occur in ischemic injuries including, but not limited to, organ (kidney, heart, lung, liver) transplantation, cerebral stroke, and myocardial infarct. Methods and compositions for modulating apoptosis are provided.

The invention provides methods useful to modulate apoptosis in a cell, tissue and/or subject. "Apoptosis" refers to programmed cell death which occurs by an active, physiological process. Apoptosis plays an important role in developmental processes, including morphogenesis, maturation of the immune system, and tissue homeostasis whereby cell numbers are limited in tissues that are continually renewed by cell division. Apoptosis is an important cellular safeguard against tumorigenesis. An apoptotic cell or a cell going through "programmed cell death" exhibits one or more characteristics associated with timed or targeted cell death. Characteristics include inhibition of cell survival, growth, death or differentiation, protein/nucleic acid cleavage/fragmentation, chromatin condensation, membrane fragmentation, changes in expression or activity of one or more proteins that promote apoptosis or that inhibit apoptosis.

"Modulating" apoptosis means increasing, stimulating or inducing, or decreasing, inhibiting, blocking or preventing (*e.g.*, prophylaxis) one or more characteristics of programmed cell death as described herein or known in the art. For example, the methods and compositions of the disclosure include agents (*e.g*., antisense molecules, ribozymes, polypeptides, small molecules, and the like) that increase, stimulate or induce apoptosis by inhibiting the activity or production of GRPs. The disclosure also includes agents and methods that increase the activity or production of GRPs to inhibit apoptosis in tissues or cells subject to damage due to ischemia and the like.

GRP78 binds transiently to nascent, secretory and transmembrane proteins and binds permanently to abnormally folded or processed proteins in the ER. GRP78 is thought to have a protective function during and after cellular stress when protein processing in the ER is perturbed. GRP78 has been proposed as a possible target for several antitumor agents, principally radicicol and geldanamycin (Scheibel and Buckner, Biochem Pharm 56:675-82, 1998).

A potential yet heretofore uncharacterized protective role of GRP94 in ischemia is supported by the observation that expression of GRP94 is enhanced in hippocampus after transient forebrain ischemia of a duration known to result in neuronal death (Yagita et al., J Neurochem 72:1544-1551, 1999). GRP94 is similarly induced following acute kidney ischemia (Kuznetsov, Proc Natl Acad Sci USA 93:8584-8589, 1996). By comparison, heat-shock proteins, including HSP90, are over-expressed during the oxidative stress of reperfusion that generally follows ischemia. For example, the higher levels of GRP78 and GRP94 in the brains of immature rats when compared to those of adult animals account for the higher resistance of immature rats to seizure. In addition, specific induction of these GRPs in the dentate gyrus region of the adult rat brain following seizure is associated with a neuroprotective effect. For early-onset familial Alzheimer's disease (FAD), overexpression of GRP78 in neuroblastoma cells expressing a mutant presenilin-1 (PS1) protein was reported to restore resistance to ER stress.

Expression of GRP78 also prevents the aggregation and facilitates the proteasomal degradation of mutant prion proteins, which are implicated in neurodegenerative disorders such as prion diseases and transmissible spongiform encephalopathies. Induction of GRP78 has also been observed in endothelial cells damaged by reductive stress that was caused by hyperhomocysteinaemia, which, with both genetic and environmental components, is a common risk factor for thrombotic vascular events such as premature arteriosclerosis, stroke, myocardial infarction, and thrombosis. Therefore, the induction of GRP could be an adaptive response evolved in mammals to protect endothelial cells against stress-induced cell death.

Although apoptosis is mediated by diverse signals and complex interactions of cellular gene products, the results of these interactions ultimately feed into a cell death pathway that is evolutionarily conserved between humans and invertebrates. The pathway, itself, is a cascade of proteolytic events analogous to that of the blood coagulation cascade.

Several gene families and products that are involved in the apoptotic pathway have been identified. Key to the apoptotic program is a family of cysteine proteases termed caspases. The human caspase family includes Ced-3, human ICE (interleukin-1beta converting enzyme) (caspase-1), ICH-1 (caspase-2), CPP32 (caspase-3), ICErelII (caspase-4), ICErelII (caspase-5), Mch2 (caspase-6), ICE-LAP3 (caspase-7), Mch5 (caspase-8), ICE-LAP6 (caspase-9), Mch4 (caspase-10), caspase 11-14 and others.

It has been demonstrated that caspases are required for apoptosis to occur. Moreover, caspases appear to be necessary for the accurate and limited proteolytic events that are the hallmark of classic apoptosis (see Salvesen and Dixit, Cell 91:443-446, 1997). During apoptosis, an initiator caspase zymogen is activated by autocatalytic cleavage, which then activates the effector caspases by cleaving their inactive zymogen (Salvesen and Dixit, Proc. Natl. Acad. Sci. USA 96:10964-10967, 1999; Srinivasula et al., Mol. Cell. 1:949-957, 1998). The effectors are responsible for proteolytic cleavage of a number of cellular proteins leading to the characteristic morphological changes and DNA fragmentation that are often associated with apoptosis (reviewed in Cohen, Biochem. J. 326:1-16, 1997; Henkart, Immunity 4:195-201, 1996; Martin and Green, Cell 82:349-352, 1995; Nicholson and Thomberry, TIBS 257:299-306, 1997; Porter et al., BioEssays 19:501-507, 1997; Salvesen and Dixit, Cell 91:443-446, 1997).

Among the executor caspases, caspase-7 has been reported to be associated with the ER. Upon induction of apoptosis, procaspase-7 (35 kDa) is first converted into a 32-kDa intermediate, which is further processed into active 20-and 11-kDa subunits. Western blotting data provided herein indicates that treating CHO cells with etoposide results in activation of caspase-7, giving rise to the 32-kDa intermediate form (Figure 5, Panel A). Upon longer exposure of the autoradiogram, the active 20- and 11-kDa forms were evident in the etoposide-treated cells. When GRP78 was overexpressed, a low level of caspase-7 activation was detected in both the non-treated and etoposide-treated cells. These data indicate that GRP78 can suppress caspase-7 activation *in vivo* thereby inhibiting apoptosis. In addition, upon addition of cytochrome c, caspase-7 activation was higher in CHO cells than C.1 cells, as evidenced by the increase in the active 20- and 11-kDa forms in the CHO samples compared with the C.1 samples. In the presence of both cytochrome c and dATP, the suppressive effect of the C.1 samples was reversed. At 1 mM dATP, both cell lines showed equivalent amounts of the 32- and 20-kDa forms, indicating that dATP releases procaspase-7 from GRP78, resulting in its activation.

The invention demonstrates that over-expression in tissue culture systems of GRP78 can protect cells against cell death. The invention also demonstrates that inhibitors of expression (e.g., antisense and RNAi) or inhibitors of GRP activity in tissue culture systems can induce apoptosis. Thus, the protective function of the GRPs is useful and beneficial in situations involving tissue or organ damage. This same protective function is detrimental in cancer by preventing apoptosis of cancer cells.

As demonstrated herein, GRP upregulation and/or overexpression is useful in limiting damage in organs exposed to stress. However, the anti-apoptotic function of GRPs also indicates that their induction in neoplastic cells and cell proliferative disorders could lead to cancer progression and drug resistance. In a variety of cancer cell lines, solid tumors and human cancer biopsies, the levels of GRP78 and GRP94 are elevated, correlating with malignancy. In addition, induction of GRP78 has been shown to protect cancer cells from immune surveillance, whereas suppressing the stress-mediated induction of GRP78 enhanced apoptosis, inhibited tumor growth and increased the cytotoxicity of chronic hypoxic cells.

Thus, the invention provides methods and compositions useful for targeted suppression of GRP expression or function in cancer cells as a novel approach to cancer therapy. For example, Genistein, which suppresses both the GRP and the heat shock responses, inhibits the growth of carcinogen-induced tumors in rats and in human leukemia cells transplanted into mice. In another example, GRP94 has been' shown to associate with and stabilize p185/erbB2 (also referred to HER-2/neu), which is commonly over-expressed in breast carcinomas and is associated with poor prognosis. Treatment of breast cancer cells with geldanamycin, an antiproliferative agent, enables the degradation of p185 in the breast cancer cells by disrupting the GRP94-p185 complex.

Pre-induction of GRP in a variety of human cancer cell lines confers resistance to inhibitors of topoisomerase II (*e.g*., etoposide) but increases sensitivity to DNA crosslinking agents such as cisplatin. Direct suppression of GRP94 levels by antisense knockdown strategies results in enhanced sensitivity to etoposide-induced cell death.

Accordingly, the invention provides methods useful in reducing the anti-apoptotic effect of GRPs, increase sensitivity of cancer cells to chemotherapeutic agents, and promote apoptosis of neoplastic cells. The methods of the invention inhibit the production or activity of GRPs in neoplastic cells (*e.g*., cancer cells) and tissues.

As will be discussed below, the invention provides the first evidence that a population of GRPs is integrally-associated with the membrane of the endoplasmic reticulum. These GRPs interact with a cytosolic component to mediate apoptosis. A "cytosolic component that mediates apoptosis", as used herein, is any polypeptide, or group of polypeptides that cooperate in the initiation or facilitation of apoptosis. For example, the interaction between GRP78 and caspase-7 and/or the interaction between GRP94 and p185/erbB2 is involved in GRPs ability to modulate apoptosis. The interaction can be, for example, with caspase-7 individually, or as part of a group of other polypeptides involved in the apoptosis pathway.

The invention further provides the first evidence that complex formation between endogenous GRP78 and caspase-7 occurs in association with the endoplasmic reticulum. While the data provided herein indicates that GRP78 and caspase-7 interact, the invention is not limited to a direct interaction between the two proteins. It is understood that the invention encompasses a cytosolic component that is a complex of polypeptides, including caspase-7, or caspase-7 individually. By preventing the interaction of GRP78 with caspase-7, the agent modulates apoptosis by promoting apoptosis. Alternatively, by promoting the interaction of GRP78 with caspase-7, an agent would modulate apoptosis by inhibiting apoptosis.

As used herein, the term "interact" includes any detectable interactions between molecules. The term "interact" is also meant to include "binding" interactions between molecules. Interactions can, for example, be protein-protein, protein-nucleic acid, and nucleic acid-nucleic acid in nature including hydrogen-bond interactions, covalent-bond interactions and the like.

An "agent", as used herein, can be any molecule including, for example, a polypeptide, an antibody, a nucleic acid (*e.g*., an antisense, ribozyme, siRNA or the like) or a small molecule. An agent can be a "therapeutic agent" useful for treating disorders associated with cell proliferation including anti-neoplastic agents and anti-inflammatory agents.

The invention provides apoptotic agents (*e.g*., GRP antagonists) comprising agents that inhibit the anti-apoptotic affect of GRPs (*e.g*., GRP78).

In embodiments where apoptosis is desired, the agent that directly reduces expression/activity of GRP can be a nucleic acid that reduces expression of GRP. In embodiments where anti-apoptotic activity is desired, the nucleic acid can be a sense nucleic acid that encodes a GRP protein (*e.g*., introduction into a cell can increase the cells GRP activity).

The coding strand sequences of GRPs are known. For example, Table 1 provides the coding sequences of some GRPs and related molecular chaperones. Other sequence will be readily apparent and available through GenBank.

**Table 1**

| GRP | GenBank/NCBI Accession No. |
|---|---|
| GRP58 | NM_005313 (SEQ ID NO:5 and 6) |
| GRP78 | BCO20235 and P11021 (SEQ ID NO:1 and 2) |
| GRP94 | BC066656 and AAH66656 (SEQ ID NO:15 and 16) |
| Calreticulin (CALR) | NM_004343 and BT007448 (SEQ ID NO:7 and 8) |
| calreticulin 3 (CALR3) PDI | NM_145046 (SEQ ID NO:9 and 10) E03087 and NM_006849 (pancreatic) (SEQ ID NO:11 and 12) |
| ERp72 | HUMERP72H (SEQ ID NO:13 and 14) |

Each of the accession numbers and their content is incorporated herein by reference. Given the coding strand sequences encoding, for example, GRP78, antisense nucleic acids can be designed according to the rules of Watson and Crick base pairing. The antisense nucleic acid molecule can be complementary to the entire coding region of a GRP polynucleotide (*e.g*., GRP78 mRNA), or can be an oligonucleotide, which is antisense to only a portion of the coding or noncoding region of a GRP. For example, the antisense oligonucleotide can be complementary to the region surrounding the transcriptional or translation start site of GRP mRNA. An antisense oligonucleotide can be, for example, about 10, 20, 25, 50, 100, 150, 200, 250, 300, 350, 400 or more nucleotides in length. An antisense nucleic acid molecule can be constructed using chemical synthesis and enzymatic ligation reactions using procedures known in the art.

[0066] Soluble domains of a GRP may interact with a transmembrane domain of a GRP to prevent incorporation of the GRP into a membrane of a cell. For example, as described herein, GRP78 comprises a cytosolic domain and transmembrane domains. The cytosolic domain (*e.g*., a soluble domain) interacts with cytosolic proteins that induce apoptotis. By inhibiting the interaction of the GRP with the cytosolic proteins that induce apoptosis, the anti-apoptotic effect of the GRP can be inhibited. Furthermore, GRPs (e.g., GRP78 comprises hydrophobic transmembrane domain(s). For example, hydrophobic transmembrane domain III (amino acids 210-260 of SEQ ID NO:2) and/or domain IV (amino acids 400-450 of SEQ ID NO:2) of the protein of GRP78 are useful targets. Polypeptide agents that regulate the ability of a GRP polypeptide to integrate in to the membrane are candidates for modulating apoptosis. For example, a polypeptide agent that inhibits the ability of GRP78 to integrate into the membrane will also be capable of promoting apoptosis because it will prevent GRP78 from interacting with cytosolic components that are required to promote apoptosis. Thus, variants and fragments of a GRP protein (e.g., fragments, analogs and derivatives of native GRP proteins) may also be used in methods of the invention to inhibit anti-apoptotic activity of a GRP (e.g., a GRP antagonist).

As discussed above, the topology of GRP78 indicates that part of GRP78 is exposed to the cytosol, allowing it to interact with cytosolic components. Thus, in another embodiment, the invention provides a method of modulating apoptosis by contacting GRP78 with an agent that inhibits or prevents the ability of the protein to integrate in to the membrane of the endoplasmic reticulum.

The invention provides methods that are useful to promote apoptosis in a tissue or cell comprising contacting the tissue or cell with an agent that inhibits the anti-apoptotic activity of a GRP (*e.g*., GRP78). The methods are useful in treating neoplastic disorders including cancer and tumor growth. The methods can be used alone or in combination with other neoplastic/cancer therapies. For example, the methods and compositions of the invention can be used in combination with chemotherapeutic drugs such as, but not limited to, 5-fluorouracil (5FU), cytosine arabinoside, cyclophosphamide, cisplatin, carboplatin, doxyrubicin, etoposide, taxol, and alkylating agents. Furthermore, combinations of nucleic acid inhibitors may be used (e.g., a combination of SEQ ID NO:3 and SEQ ID NO:4).

In another aspect, variants and fragments of a GRP protein (e.g., fragments, analogs and derivatives of native GRP proteins) may also be used in methods of the invention. Such variants include, *e.g*., a polypeptide encoded by a naturally occurring allelic variant of a native GRP polynucleotide, a polypeptide encoded by an alternative splice form of a native GRP polynucleotide, a polypeptide encoded by a homolog of a native GRP polynucleotide, and a polypeptide encoded by a non-naturally occurring variant of a native GRP polynucleotide.

GRP protein variants have a polypeptide sequence that differs from a native GRP protein in one or more amino acids. The peptide sequence of such variants can feature a deletion, addition, or substitution of one or more amino acids of a native GRP polypeptide. Amino acid insertions are typically of about 1 to 4 contiguous amino acids, and deletions are preferably of about 1 to 10 contiguous amino acids. In some applications, variant GRP proteins substantially maintain a native GRP protein functional activity (*e.g*., ability to mediate anti-apoptotic activity, bind caspase-7 and the like; are agonists). Such functional variants are useful in treating disorders associated with apoptosis, *e.g*., ischemia and the like, where it is desirable to reduce apoptosis. For other applications, variant GRP proteins lack or feature a significant reduction in a GRP protein functional activity. Where it is desired to retain a functional activity of native GRP protein, a GRP protein variant can be made by expressing nucleic acid molecules that feature silent or conservative changes. Variant GRP proteins with substantial changes in functional activity can be made by expressing nucleic acid molecules that feature less than conservative changes.

GRP protein fragments corresponding to one or more particular motifs and/or domains or to arbitrary sizes, for example, at least 5, 10, 25, 50, 75, 100, 125, 150, or 175 amino acids in length may be utilized in methods of the invention. In addition, fragments can be chemically synthesized using techniques known in the art such as conventional solid phase f-Moc or t-Boc chemistry. For example, a GRP protein used in the methods of the invention may be arbitrarily divided into fragments of desired length with no overlap of the fragments, or divided into overlapping fragments of a desired length. The fragments can be produced (recombinantly or by chemical synthesis) and tested to identify those fragments, which can function as either agonists or antagonists of a native GRP protein.

Methods of the invention may also involve recombinant forms of the GRP proteins. Recombinant polypeptides, in addition to native GRP protein, are encoded by a nucleic acid that has at least 85% sequence identity (*e.g*., 85, 86, 87, 88, 89, 90, 91, 92, 93, 94, 95, 96, 97, 98, 99, 100%) with a native GRP nucleic acid sequence. In a one embodiment, variant GRP proteins lack one or more functional activities of a native GRP protein.

GRP protein variants can be generated through various techniques known in the art. For example, GRP protein variants can be made by mutagenesis, such as by introducing discrete point mutation(s), or by truncation. Mutation can give rise to a GRP protein variant having substantially the same, or merely a subset of the functional activity of a native GRP protein. Alternatively, antagonistic forms of the protein can be generated which are able to inhibit the function of the naturally occurring form of the protein, such as by competitively binding to another molecule that interacts with GRP protein (*e.g*., interferes with the interaction of GRP78 and caspase-7). In addition, agonistic forms of the protein may be generated that constitutively express one or more GRP functional activities. Other variants of GRP proteins that can be generated include those that are resistant to proteolytic cleavage, as for example, due to mutations that alter protease target sequences. Whether a change in the amino acid sequence of a peptide results in a GRP protein variant having one or more functional activities of a native GRP protein can be readily determined by testing the variant for a native GRP protein functional activity.

Nucleic acid molecules encoding GRP fusion proteins may be used in methods of the invention. Such nucleic acids can be made by preparing a construct (*e.g*., an expression vector) that expresses a GRP fusion protein when introduced into a suitable host. For example, such a construct can be made by ligating a first polynucleotide encoding a GRP protein fused in frame with a second polynucleotide encoding another protein such that expression of the construct in a suitable expression system yields a fusion protein.

As another example, GRP protein variants can be generated from a degenerate oligonucleotide sequence. Chemical synthesis of a degenerate polynucleotide sequence can be carried out in an automatic DNA synthesizer, and the synthetic polynucleotide then ligated into an appropriate expression vector. The purpose of a degenerate set of polynucleotides is to provide, in one mixture, all of the sequences encoding the desired set of potential GRP protein sequences. The synthesis of degenerate oligonucleotides is well known in the art (see for example, Narang, Tetrahedron 39:3, 1983; Itakura et al., Recombinant DNA, Proc 3rd Cleveland Sympos. Macromolecules, ed. A G Walton, Amsterdam: Elsevier pp 273-289, 1981; Itakura et al., Annu. Rev. Biochem. 53:323, 1984; Itakura et al., Science 198:1056, 1984; Ike et al., Nucleic Acid Res. 11:477, 1983. Such techniques have been employed in the directed evolution of other proteins (see, for example, Scott et al., Science 249:386-390, 1990; Roberts et al., Proc. Natl. Acad. Sci. USA 89:2429-2433, 1992; Devlin et al., Science 249: 404-406, 1990; Cwirla et al., Proc. Natl. Acad. Sci. USA 87: 6378-6382, 1990; as well as U.S. Pat. Nos. 5,223,409; 5,198,346; and 5,096,815).

Similarly, a library of coding sequence fragments can be provided for a GRP clone in order to generate a variegated population of GRP polypeptide fragments for screening and subsequent selection of fragments having one or more GRP agonist (*e.g*., anti-apoptotic) or antagonist (*e.g*., apoptotic) activities. A variety of techniques are known in the art for generating such libraries, including chemical synthesis. In one embodiment, a library of coding sequence fragments can be generated by (i) treating a double-stranded PCR fragment of a GRP polynucleotide coding sequence with a nuclease under conditions wherein nicking occurs only about once per molecule; (ii) denaturing the double-stranded DNA; (iii) renaturing the DNA to form double-stranded DNA which can include sense/antisense pairs from different nicked products; (iv) removing single-stranded portions from reformed duplexes by treatment with S1 nuclease; and (v) ligating the resulting fragment library into an expression vector. By this exemplary method, an expression library can be derived which codes for N-terminal, C-terminal and internal fragments of various sizes.

A wide range of techniques are known in the art for screening products of combinatorial libraries made by point mutations or truncation, and for screening cDNA libraries for products having a certain property. Such techniques will be generally adaptable for rapid screening of the gene libraries generated by the combinatorial mutagenesis of GRP polynucleotide variants. The most widely used techniques for screening large libraries typically involve cloning the library into replicable expression vectors, transforming appropriate cells with the resulting library of vectors, and expressing the combinatorial genes under conditions in which detection of a desired activity facilitates relatively easy isolation of the vector encoding the gene whose product was detected. One screening technique useful to measure anti-apoptotic and apoptotic effects includes determining cell survival in the presence of etoposide. Recombinant products that inhibit (*e.g*., are antagonistic of) native GRP function will show an increase in cell-death in the presence of etoposide, whereas products that promote (*e.g*., are agonists of) GRP function will show a reduced cell-death compared to controls. Thus, the invention provides methods of mutagenizing and screening gene products to determine their agonistic and/or antagonistic effect on GRP activity. Agents having agonistic effects are useful for treating ischemia and related disorders that cause unwanted cell death. Agents that have antagonistic effects are useful to treat diseases and disorders having unwanted cell growth (e.g., cell proliferative disorders associated with cancer and the like).

Methods of the invention may utilize mimetics, *e.g*. peptide or non-peptide agents, that are able to disrupt binding of a GRP protein to other proteins or molecules with which a native GRP protein interacts (*e.g*., caspase-7). Thus, the mutagenic techniques described herein can also be used to map which determinants of GRP protein participate in the intermolecular interactions involved in, for example, binding of a GRP protein to other proteins which function to carry out apoptosis. Although the invention described thus far has focused on methods and compositions useful to promote apoptosis by inhibiting anti-apoptotic GRPs, the invention also includes methods and compositions that promote anti-apoptotic activity (e.g., in response ischemic injury and the like) .

The invention also provides methods which promote anti-apoptotic activity of GRPs (*e.g*., GRP78 and 94). The compositions and methods of this aspect of the invention are useful to treat tissue damage or potential damage to cells or tissues resulting from, for example, stroke, heart attack, hypoxia, hypoglycemia, brain or spinal cord ischemia, or brain or spinal cord trauma. The methods use agents (including, *e.g*., small molecules, polypeptides, peptides, and nucleic acids) that promote GRP activity, GRP expression, GRP production, and/or GRP association with polypeptides resulting in an inhibition of apoptosis.

The invention provides methods for modulating GRP expression and/or activity in a cell. Numerous agents for modulating expression/activity of intracellular proteins such as GRP in a cell are known. Any of these suitable for the particular system being used may be employed. Typical agents for promoting (*e.g*., agonistic) activity of GRPs include mutant/variant GRP polypeptides or fragments, nucleic acids encoding a functional GRP polypeptide or variant, and small organic or inorganic molecules.

Examples of proteins that can modulate GRP expression and/or activity in a cell include native GRP proteins (*e.g*., to upregulate activity) or variants thereof that can compete with a native GRP protein for binding ligands such as a caspase (*e.g*., to downregulate apoptosis). Such protein variants can be generated through various techniques known in the art as described herein. For example, GRP protein variants can be made by mutagenesis, such as by introducing discrete point mutation(s), or by truncation (*e.g*., of the transmembrane region). Mutation can give rise to a GRP variant or fragment having substantially the same, improved, or merely a subset of the functional activity of a native GRP protein. Agonistic (or superagonistic) forms of the protein may be generated that constitutively express one or more GRP functional activities. Other variants of GRP polypeptides that can be generated include those that are resistant to proteolytic cleavage, as for example, due to mutations which alter protease target sequences. Whether a change in the amino acid sequence of a peptide results in a GRP protein variant having one or more functional activities of a native GRP protein can be readily determined by testing the variant for a native GRP protein functional activity (*e.g*., modulating apoptosis).

The invention provides methods involving modulating levels of GRP in a cell *in vitro.*

Various techniques using viral vectors for the introduction of a GRP nucleic acid (*e.g*., an inhibitory nucleic acid such as an antisense molecule or a GRP variant) into a cell may be utilized in the methods of the invention. Viral vectors for use in the invention are those that exhibit low toxicity to a host cell and induce production of therapeutically useful quantities of a GRP protein or antisense and/or RNAi nucleic acids in a tissue-specific manner. Viral vector methods and protocols that may be used in the invention are reviewed in Kay et al. Nature Medicine 7:33-40, 2001. The use of specific vectors, including those based on adenoviruses, adeno-associated viruses, herpes viruses, and retroviruses are described in more detail below.

The use of recombinant adenoviruses as gene therapy vectors is discussed in W. C. Russell, Journal of General Virology 81:2573-2604, 2000; and Bramson et al., Curr. Opin. Biotechnol. 6:590-595, 1995. Adenovirus vectors are useful in the invention because they (1) are capable of highly efficient gene expression in target cells and (2) can accommodate a relatively large amount of heterologous (non-viral) DNA. A typical form of recombinant adenovirus is a "helper-dependent" adenovirus vector. Such a vector features, for example, (1) the deletion of all or most viral-coding sequences (those sequences encoding viral proteins), (2) the viral inverted terminal repeats (ITRs) which are sequences required for viral DNA replication, (3) up to 28-32 kb of "exogenous" or "heterologous" sequences (*e.g*., sequences encoding a GRP protein, a GRP variant, an antisense molecule, or an RNAi molecule), and (4) the viral DNA packaging sequence which is required for packaging of the viral genomes into infectious capsids

Other viral vectors that might be used in the invention are adeno-associated virus (AAV)-based vectors. AAV-based vectors are advantageous because they exhibit high transduction efficiency of target cells and can integrate into the host genome in a site-specific manner. Use of recombinant AAV vectors is discussed in detail in Tal, J., J. Biomed. Sci. 7:279-291, 2000 and Monahan and Samulski, Gene Therapy 7:24-30, 2000. A typical AAV vector comprises a pair of AAV inverted terminal repeats (ITRs) which flank at least one cassette containing a tissue (*e.g*., heart)- or cell (*e.g*., cardiomyocyte)-specific promoter operably linked to a GRP nucleic acid. The DNA sequence of the AAV vector, including the ITRs, the promoter and GRP gene may be integrated into the host genome.

The use of herpes simplex virus (HSV)-based vectors is discussed in detail in Cotter and Robertson, Curr. Opin. Mol. Ther. 1:633-644, 1999. HSV vectors deleted of one or more immediate early genes (IE) are advantageous because they are generally non-cytotoxic, persist in a state similar to latency in the host cell, and afford efficient host cell transduction. Recombinant HSV vectors can incorporate approximately 30 kb of heterologous nucleic acid. A typical HSV vector is one that: (1) is engineered from HSV type I, (2) has its IE genes deleted, and (3) contains a tissue-specific promoter operably linked to a GRP nucleic acid (*e.g*., an antisense, RNAi, GRP variant). HSV amplicon vectors may also be useful in various methods of the invention. Typically, HSV amplicon vectors are approximately 15 kb in length, and possess a viral origin of replication and packaging sequences.

Retroviruses such as C-type retroviruses and lentiviruses are also useful in the invention. For example, retroviral vectors may be based on murine leukemia virus (MLV). See, *e.g*., Hu and Pathak, Pharmacol. Rev. 52:493-511, 2000 and Fong et al., Crit. Rev. Ther. Drug Carrier Syst. 17:1-60, 2000. MLV-based vectors may contain up to 8 kb of heterologous nucleic acids in place of the viral genes. The heterologous nucleic acids typically comprise a tissue-specific promoter and a GRP nucleic acid.

Additional retroviral vectors that might be used are replication-defective lentivirus-based vectors, including human immunodeficiency (HIV)-based vectors. See, *e.g*., Vigna and Naldini, J. Gene Med. 5:308-316, 2000 and Miyoshi et al., J. Virol. 72:8150-8157, 1998. Lentiviral vectors are advantageous in that they are capable of infecting both actively dividing and non-dividing cells. They are also highly efficient at transducing human epithelial cells. Lentiviral vectors for use in the invention may be derived from human and non-human (including SUV) lentiviruses. A typical lentiviral vector includes nucleic acid sequences required for vector propagation as well as a tissue-specific promoter operably linked to a GRP nucleic acid.

A lentiviral vector may be packaged into any suitable lentiviral capsid. The substitution of one particle protein with another from a different virus is referred to as "pseudotyping". The vector capsid may contain viral envelope proteins from other viruses, including murine leukemia virus (MLV) or vesicular stomatitis virus (HSV). The use of the VSV G-protein yields a high vector titer and results in greater stability of the vector virus particles.

Alphavirus-based vectors, such as those made from semliki forest virus (SFV) and sindbis virus (SIN), might also be used in the invention. Use of alphaviruses is described in Lundstrom, K., Intervirology 43:247-257, 2000 and Perri et al., Journal of Virology 74:9802-9807, 2000. Alphavirus vectors typically are constructed in a format known as a replicon. A replicon may contain (1) alphavirus genetic elements required for RNA replication, and (2) a heterologous nucleic acid such as one encoding a GRP nucleic acid.

Recombinant, replication-defective alphavirus vectors are advantageous because they are capable of high-level gene expression, and can infect a wide host cell range. Alphavirus replicons may be targeted to specific cell types by displaying on their virion surface a functional ligand or binding domain that would allow selective binding to target cells expressing a cognate binding partner. Alphavirus replicons may establish latency, and therefore long-term heterologous nucleic acid expression in a host cell. The replicons may also exhibit transient heterologous nucleic acid expression in the host cell. To increase tissue selectivity of the virus and reduce risk not only can such a virus have a targeted ligand on the virion surface, but also the heterologous nucleic acid (*e.g*., a GRP nucleic acid) can be operably linked to a tissue specific promoter.

In addition to viral vector-based methods, non-viral methods may also be used to introduce a GRP nucleic acid into a host cell. A review of non-viral methods of gene delivery is provided in Nishikawa and Huang, Human Gene Ther. 12:861-870, 2001. A non-viral gene delivery method according to the invention employs plasmid DNA to introduce a GRP nucleic acid into a cell. Plasmid-based gene delivery methods are generally known in the art and are described in references such as Ilan, Y., Curr. Opin. Mol. Ther. 1:116-120, 1999, Wolff, J. A., Neuromuscular Disord. 7:314-318, 1997 and Arztl, Z., Fortbild Qualitatssich 92:681-683, 1998.

Methods involving physical techniques for introducing a GRP nucleic acid into a host cell can be adapted for use in the invention. For example, the particle bombardment method of gene transfer utilizes an Accell device (gene gun) to accelerate DNA-coated microscopic gold particles into a target tissue, *e.g.*, a cancer tissue. See, *e.g*., Yang et al., Mol. Med. Today 2:476-481 1996 and Davidson et al., Rev. Wound Repair Regen. 6:452-459, 2000. As another example, cell electropermeabilization (also termed cell electroporation) may be employed to deliver GRP nucleic acids into cells. See, *e.g*., Preat, V., Ann. Pharm. Fr. 59:239-244 2001.

Synthetic gene transfer molecules can be designed to form multimolecular aggregates with plasmid DNA. These aggregates can be designed to bind to a target cell surface in a manner that triggers endocytosis and endosomal membrane disruption. Cationic amphiphiles, including lipopolyamines and cationic lipids, may be used to provide receptor-independent GRP nucleic acid transfer into target cells. In addition, preformed cationic liposomes or cationic lipids may be mixed with plasmid DNA to generate cell-transfecting complexes. Methods involving cationic lipid formulations are reviewed in Felgner et al., Ann. N.Y. Acad. Sci. 772:126-139, 1995 and Lasic and Templeton, Adv. Drug Delivery Rev. 20:221-266, 1996. For gene delivery, DNA may also be coupled to an amphipathic cationic peptide (Fominaya et al., J. Gene Med. 2:455-464, 2000).

DNA microencapsulation may be used to facilitate delivery of a GRP nucleic acid. Microencapsulated gene delivery vehicles may be constructed from low viscosity polymer solutions that are forced to phase invert into fragmented spherical polymer particles when added to appropriate nonsolvents. Methods involving microparticles are discussed in Hsu et al., J. Drug Target 7:313-323, 1999 and Capan et al., Pharm. Res. 16:509-513, 1999.

Protein transduction offers an alternative to gene therapy for the delivery of therapeutic proteins into target cells, and methods involving protein transduction are within the scope of the invention. Protein transduction is the internalization of proteins into a host cell from the external environment. The internalization process relies on a protein or peptide which is able to penetrate the cell membrane. To confer this ability on a normally non-transducing protein, the non-transducing protein can be fused to a transduction-mediating protein such as the antennapedia peptide, the HIV TAT protein transduction domain, or the herpes simplex virus VP22 protein. See Ford et al., Gene Ther. 8:1-4, 2001.

There are two major approaches to getting the nucleic acid (optionally contained in a vector) into a subject's cells; *in vivo* and *ex vivo.* For *in vivo* delivery the nucleic acid is injected directly into the subject, usually at the site where the nucleic acid is needed. For *ex vivo* treatment, the subject's cells are removed, the nucleic acid is introduced into these isolated cells and the modified cells are administered to the subject either directly or, for example, encapsulated within porous membranes which are implanted into the subject (see, *e.g*., U.S. Pat. Nos. 4,892,538 and 5,283,187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, viral vectors and the like. A commonly used vector for *ex vivo* and in vivo delivery is a viral vector as discussed above.

Host cells can be transfected or transformed with expression or cloning vectors described herein and cultured in conventional nutrient media modified as appropriate for inducing promoters, selecting transformants, or amplifying the nucleic acids encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook *et al*.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook *et al*., *supra,* or electroporation is generally used for prokaryotes. Infection with Agrobacterium tumefaciens is used for transformation of certain plant cells, as described by Shaw et al., Gene, 23:315 (1983) and WO 89/05859 published 29 Jun. 1989. For mammalian cells without such cell walls, the calcium phosphate precipitation method of Graham and van der Eb, Virology, 52:456-457 (1978) can be employed. General aspects of mammalian cell host system transfections have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, see Keown et al., Methods in Enzymology, 185:527-537 (1990) and Mansour et al., Nature, 336:348-352 (1988) .

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacter, Erwinia, Klebsiella, Proteus, Salmonella, *e.g*., Salmonella typhimurium, Serratia, *e.g*., Serratia marcescans, and Shigella, as well as Bacilli such as B. subtilis and B. licheniformis (*e.g*., B. licheniformis 41P disclosed in DD 266,710 published 12 Apr. 1989), Pseudomonas such as P. aeruginosa, and Streptomyces. These examples are illustrative rather than limiting.

In addition to prokaryotes, eukaryotic microbes such as filamentous fungi or yeast are suitable cloning or expression hosts. *Saccharomyces cerevisiae* is a commonly used lower eukaryotic host microorganism. Others include *Schizosaccharomyces* pombe, Kluyveromyces hosts such as, *e.g*., *K. lactis, K. fragilis, K. bulgaricus, K. wickeramii, K. waltii, K. drosophilarum, K. thermotolerans,* and *K. marxianus;* yarrowia; *Pichia pastoris; Candida; Trichoderma reesia; Neurospora crassa; Schwanniomyces* such as *Schwanniomyces occidentalis;* and filamentous fungi such as, *e.g., Neurospora, Penicillium, Tolypocladium,* and *Aspergillus* hosts such as *A*. *nidulans* and *A. niger*. Methylotropic yeasts are suitable herein and include, but are not limited to, yeast capable of growth on methanol selected from the genera consisting of Hansenula, Candida, Kloeckera, Pichia, Saccharomyces, Torulopsis, and Rhodotorula. A list of specific species that are exemplary of this class of yeasts may be found in C. Anthony, The Biochemistry of Methylotrophs, 269 (1982).

Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59,1977); baby hamster kidney cells (BHK, ATCC CCL 10); Chinese hamster ovary cells/-DHFR (CHO, Urlaub et al., Proc. Natl. Acad. Sci. USA 77:4216, 1980); mouse sertoli cells (TM4, Mather, Biol. Reprod. 23:243-251, 1980); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI culls (Mather et al., Annals N.Y. Acad. Sci. 383:44-68, 1982); MRC 5 cells; FS4 cells; and a human hepatoma line (Hep G2).

Host cells are transformed with the above-described GRP nucleic acid expression or cloning vectors and cultured in conventional nutrient media modified as appropriate for inducing promoters, selecting transformants, or amplifying the nucleic acids encoding the desired sequences.

The GRP nucleic acids (*e.g*., antisense, RNAi, ribozymes, variants, coding sequences and the like) may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

Expression and cloning vectors will typically contain a selection gene, also termed a selectable marker. Typical selection genes encode proteins that (a) confer resistance to antibiotics or other toxins, *e.g*., ampicillin, neomycin, methotrexate, or tetracycline, (b) complement auxotrophic deficiencies, or (c) supply critical nutrients not available from complex media, *e.g*., the gene encoding D-alanine racemase for Bacilli.

An example of suitable selectable markers for mammalian cells are those that enable the identification of cells competent to take up the BDB oligopeptide-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216, 1980. A suitable selection gene for use in yeast is the trp1 gene present in the yeast plasmid YRp7 (Stinchcomb et al., Nature, 282:39, 1979; Kingsman et al., Gene, 7:141, 1979; Tschemper et al., Gene, 10:157, 1980). The trp1 gene provides a selection marker for a mutant strain of yeast lacking the ability to grow in tryptophan.

A variety of well-known techniques can be used to identify polypeptides which specifically bind to, for example, GRP78 and/or caspase-7, and regulate their interactions. Exemplary techniques include mobility shift DNA-binding assays, methylation and uracil interference assays, DNase and hydroxy radical footprinting analysis, fluorescence polarization, and UV crosslinking or chemical cross-linkers. For a general overview, see, *e.g*., Ausubel (chapter 12, DNA-Protein Interactions). Furthermore, biological assays the measure the agonistic and antagonistic effects of such agents are also provided.

The invention provides methods for treating a subject having a cell proliferative disorder. The subject can be any mammal, and is preferably a human. The contacting can be in vivo or ex vivo. Methods of administering pharmaceutical compositions are known in the art and include, for example, systemic administration, topical administration, intraperitoneal administration, intra-muscular administration, as well as administration directly at the site of a tumor or cell-proliferative disorder and other routes of administration known in the art.

The pharmaceutical compositions according to the invention may be administered locally or systemically. By "therapeutically effective dose" is meant the quantity of a compound according to the invention necessary to prevent, to cure or at least partially arrest the symptoms of the disease and its complications. Amounts effective for this use will, of course, depend on the severity of the disease and the weight and general state of the subject. Typically, dosages used in vitro may provide useful guidance in the amounts useful for in situ administration of the pharmaceutical composition, and animal models may be used to determine effective dosages for treatment of particular disorders. Various considerations are described, e.g., in Langer, Science, 249: 1527, (1990); Gilman et al. (eds.) (1990), each of which is herein incorporated by reference.

As used herein, "administering a therapeutically effective amount" is intended to include methods of giving or applying a pharmaceutical composition of the invention to a subject that allow the composition to perform its intended therapeutic function. The therapeutically effective amounts will vary according to factors such as the degree of infection in a subject, the age, sex, and weight of the individual. Dosage regimen can be adjusted to provide the optimum therapeutic response. For example, several divided doses can be administered daily or the dose can be proportionally reduced as indicated by the exigencies of the therapeutic situation.

As used herein, a "pharmaceutically acceptable carrier" is intended to include solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the pharmaceutical composition, use thereof in the therapeutic compositions and methods of treatment is contemplated. Supplementary active compounds can also be incorporated into the compositions.

The principal pharmaceutical composition is compounded for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

Further, methods of the invention can be performed alone or in conjunction with standard medical treatments currently available for treating a cell proliferative disorder. For example, when a tumor is being treated, it may be preferable to remove the majority of a tumor surgically or by radiation prior to introducing a construct of the invention in to the cells comprising the tumor.

The terms "protein", "peptide" and "polypeptide" as used herein, describe any chain of amino acids, regardless of length or post-translational modification (for example, glycosylation or phosphorylation). Thus, the terms can be used interchangeably herein to refer to a polymer of amino acid residues. The terms also apply to amino acid polymers in which one or more amino acid residue is an artificial chemical mimetic of a corresponding naturally occurring amino acid. Thus, the term "polypeptide" includes full-length, naturally occurring proteins as well as recombinantly or synthetically produced polypeptides that correspond to a full-length naturally occurring protein or to particular domains or portions of a naturally occurring protein. The term also encompasses mature proteins which have an added amino-terminal methionine to facilitate expression in prokaryotic cells.

Polypeptides and peptides can be chemically synthesized using known techniques or produced using known molecular biology techniques. Polypeptides and proteins are encoded in the genome of an organism by nucleic acids in discrete functional units sometimes referred to as "genes". Nucleic acid molecules, however, can be removed and isolated from their naturally occurring environment and engineered and manipulated using molecular biology techniques. The term "isolated" means altered "by the hand of man" from its natural state; i.e., if it occurs in nature, it has been changed or removed from its original environment, or both. For example, a naturally occurring nucleic acid molecule or a polypeptide naturally present in a living animal in its natural state is not "isolated", but the same nucleic acid or polypeptide separated from the coexisting materials of its natural state is "isolated", as the term is employed herein.

"Polynucleotide" or "nucleic acid molecule" refers to a polymeric form of nucleotides at least 10 bases in length. By "isolated nucleic acid" is meant a polynucleotide that is not immediately contiguous with either of the coding sequences with which it is immediately contiguous (one on the 5' end and one on the 3' end) in the naturally occurring genome of the organism from which it is derived. The term therefore includes, for example, a recombinant DNA which is incorporated into a vector; into an automatically replicating plasmid or virus; or into the genomic DNA of a prokaryote or eukaryote, or which exists as a separate molecule (*e.g*., a cDNA) independent of other sequences. The nucleic acid molecules of the invention may comprise ribonucleotides, deoxyribonucleotides, or modified forms of either nucleotide. The term includes single and double stranded forms.

The term nucleic acid molecule(s) or polynucleotide(s) generally refers to any polyribonucleotide or polydeoxyribonucleotide, which may be unmodified RNA or DNA or modified RNA or DNA. Thus, for instance, polynucleotides as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single-and double-stranded regions.

In addition, a polynucleotide or nucleic acid molecule as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide.

As used herein, the term polynucleotide includes DNAs or RNAs as described above that contain one or more modified bases. Thus, DNAs or RNAs with backbones modified for stability or for other reasons are "nucleic acid molecules" as that term is intended herein.

Nucleic acid molecules comprising an antisense molecule, a siRNA molecule, or encoding a GRP polypeptide and the like, as disclosed herein, can be operatively linked to expression control element(s). "Operatively linked" refers to a juxtaposition wherein the components so described are in a relationship permitting them to function in their intended manner. An expression control element(s) operatively linked to a nucleic acid molecule of the invention is ligated such that transcription of the nucleic acid molecule is achieved under conditions compatible with the expression control element(s). As used herein, the term "expression control element(s)" refers to control domain that regulate the expression of a nucleic acid molecule to which it is operatively linked. Expression control element(s) are operatively linked to a nucleic acid molecules when the expression control element(s) control and regulate the transcription and, as appropriate, translation of the nucleic acid molecule. Thus, expression control element(s) can include appropriate promoters, enhancers, transcription terminators, a start condon (i.e., ATG) in front of a protein-encoding nucleic acid, splicing signals for introns, maintenance of the correct reading frame of that gene to permit proper translation of the mRNA, and stop condons. The term "control element(s)" is intended to include, at a minimum, components whose presence can influence expression, and can also include additional components whose presence is advantageous, for example, leader sequences and fusion partner sequences. Expression control element(s) can include a promoter.

By "promoter" is meant a minimal nucleic acid domain sufficient to direct transcription. Also included in the invention are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters, are included in the invention (see *e.g*., Bitter et al., Methods in Enzymology 153:516-544, 1987). For example, when cloning in bacterial systems, inducible promoters such as pL of bacteriophage-γ, plac, ptrp, ptac (ptrp-lac hybrid promoter) and the like may be used. When cloning in mammalian cell systems, promoters derived from the genome of mammalian cells (*e.g*., metallothionein promoter) or from mammalian viruses (*e.g*., the retrovirus long terminal repeat; the adenovirus late promoter; the vaccinia virus 7.5K promoter) may be used. Promoters produced by recombinant DNA or synthetic techniques may also be used to provide for transcription of the nucleic acids of the invention.

A nucleic acid molecule may be designed to selectively hybridize to a target polynucleotide or oligonucleotide under desired conditions. The phrase "selectively (or specifically) hybridizes to" refers to the binding, duplexing, or hybridizing of a molecule only to a particular nucleotide sequence under stringent hybridization conditions when that sequence is present in a complex mixture.

The phrase "astringent hybridization conditions" refers to conditions under which a nucleic acid molecule will hybridize to its target complementary sequence, typically in a complex mixture of nucleic acids, but to no other sequences. In the context of the invention, stringent conditions comprises hybridization and washing under which nucleotide sequences at least 60% homologous to each other typically remain hybridized to each other. Typically, the conditions are such that sequences at least about 65%-70% or 75% or more homologous to each other typically remain hybridized to each other.

Generally, stringent conditions are selected to be about 5 to 10 °C lower than the thermal melting point (Tₘ) for the specific sequence at a defined ionic strength pH. The Tₘ is the temperature (under defined ionic strength, pH, and nucleic acid concentration) at which 50% of the nucleic acid molecules complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30 °C for short probes (for example, 10 to 50 nucleotides) and at least about 60 °C. for long probes (for example, greater than 50 nucleotides). Stringent conditions also may be achieved with the addition of destabilizing agents, for example, formamide.

Exemplary highly stringent hybridization conditions can be as following, for example: 50% formamide, 5xSSC and 1% SDS, incubating at 42 °C, or 5xSSC and 1% SDS, incubating at 65 °C, with wash in 0.2xSSC and 0.1% SDS at 65 °C. Alternative conditions include, for example, conditions at least as stringent as hybridization at 68 °C for 20 hours, followed by washing in 2xSSC, 0.1% SDS, twice for 30 minutes at 55 °C and three times for 15 minutes at 60 °C. Another alternative set of conditions is hybridization in 6xSSC at about 45 °C, followed by one or more washes in 0.2xSSC, 0.1% SDS at 50-65 °C. Exemplary moderately stringent hybridization conditions include a hybridization in a buffer of 40% formamide, 1 M NaCl, 1% SDS at 37 °C, and a wash in 1xSSC at 45 °C.

"Treating" or "treatment" or "alleviation" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for a neoplastic disorder/cancer if, after receiving a therapeutic amount of a GRP antagonist the subject shows observable and/or measurable reduction in or absence of one or more of the following: reduction in the number of cancer cells or absence of the cancer cells; reduction in the tumor size; inhibition (i.e., slow to some extent and preferably stop) of cancer cell infiltration into peripheral organs including the spread of cancer into soft tissue and bone; inhibition (i.e., slow to some extent and preferably stop) of tumor metastasis; inhibition, to some extent, of tumor growth; and/or relief to some extent, one or more of the symptoms associated with the specific cancer; reduced morbidity and mortality, and improvement in quality of life issues.

The phrase "non-dividing" cell refers to a cell that does not go through mitosis. Non-dividing cells may be blocked at any point in the cell cycle, (*e.g*., G0/G1 G1/S, G2/M), as long as the cell is not actively dividing. Examples of pre-existing non-dividing cells in the body include neuronal, muscle, liver, skin, heart, lung, and bone marrow cells, and their derivatives.

By "dividing" cell is meant a cell that undergoes active mitosis, or meiosis. Such dividing cells include stem cells, skin cells (*e.g*., fibroblasts and keratinocytes), gametes, and other dividing cells known in the art. Of particular interest and encompassed by the term dividing cell are cells having cell proliferative disorders, such as neoplastic cells. The term "cell proliferative disorder" refers to a condition characterized by an abnormal number of cells. The condition can include both hypertrophic (the continual multiplication of cells resulting in an overgrowth of a cell population within a tissue) and hypotrophic (a lack or deficiency of cells within a tissue) cell growth or an excessive influx or migration of cells into an area of a body. The cell populations are not necessarily transformed, tumorigenic or malignant cells, but can include normal cells as well.

Cell proliferative disorders include disorders associated with an overgrowth of connective tissues, such as various fibrotic conditions, including scleroderma, arthritis and liver cirrhosis. Cell proliferative disorders include neoplastic disorders such as head and neck carcinomas. Head and neck carcinomas would include, for example, carcinoma of the mouth, esophagus, throat, larynx, thyroid gland, tongue, lips, salivary glands, nose, paranasal sinuses, nasopharynx, superior nasal vault and sinus tumors, esthesioneuroblastoma, squamous call cancer, malignant melanoma, sinonasal undifferentiated carcinoma (SNUC) or blood neoplasia. Also included are carcinoma's of the regional lymph nodes including cervical lymph nodes, prelaryngeal lymph nodes, pulmonary juxtaesophageal lymph nodes and submandibular lymph nodes (Harrison's Principles of Internal Medicine (eds., Isselbacher, et al., McGraw-Hill, Inc., 13th Edition, pp1850-1853, 1994). Other cancer types, include, but are not limited to, lung cancer, colon-rectum cancer, breast cancer, prostate cancer, urinary tract cancer, uterine cancer lymphoma, oral cancer, pancreatic cancer, leukemia, melanoma, stomach cancer and ovarian cancer.

Methods involving conventional molecular biology techniques are described herein. Such techniques are generally known in the art and are described in detail in methodology treatises such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Various techniques using polymerase chain reaction (PCR) are described, *e.g*., in Innis et al., PCR Protocols: A Guide to Methods and Applications, Academic Press: San Diego, 1990. PCR-primer pairs can be derived from known sequences by known techniques such as using computer programs intended for that purpose (*e.g*., Primer, Version 0.5, 81991, Whitehead Institute for Biomedical Research, Cambridge, Mass.). Methods for chemical synthesis of nucleic acids are discussed, for example, in Beaucage and Carruthers, Tetra. Letts. 22:1859-1862, 1981, and Matteucci et al., J. Am. Chem. Soc. 103:3185, 1981. Chemical synthesis of nucleic acids can be performed, for example, on commercial automated oligonucleotide synthesizers. Immunological methods (*e.g*., preparation of antigen-specific antibodies, immunoprecipitation, and immunoblotting) are described, *e.g*., in Current Protocols in Immunology, ed. Coligan et al., John Wiley & Sons, New York, 1991; and Methods of Immunological Analysis, ed. Masseyeff et al., John Wiley & Sons, New York, 1992. Conventional methods of gene transfer and gene therapy can also be adapted for use in the invention. See, *e.g*., Gene Therapy: Principles and Applications, ed. T. Blackenstein, Springer Verlag, 1999; Gene Therapy Protocols (Methods in Molecular Medicine), ed. P. D. Robbins, Humana Press, 1997; and Retro-vectors for Human Gene Therapy, ed. C. P. Hodgson, Springer Verlag, 1996.

A few diseases and disorders (e.g., ischemia and neoplastic disorders) have been mentioned. However, those of skill in the art will recognize that a variety of diseases and degenerative disorders involve aberrant or disregulated apoptosis, resulting in inappropriate or premature cell death or inappropriate cell proliferation. For example, inhibition of cell death may contribute to disease in the immune system by allowing the persistence of self-reactive B and T cells, which leads to autoimmune disease. Furthermore, the infection by certain viruses may depend on suppression of host cell death by anti-apoptotic viral gene products and inhibition of apoptosis can alter the course (lytic vs. latent) of viral infection.

### EXAMPLES

The invention is based, in part, on the discovery that GRPs (*e.g*., GRP78) confers resistance to topoisomerase inhibitors through protection against drug-induced apoptosis. As shown in Figure 1, panel A, quantitation of the immunoblots of whole cell extracts showed 5-fold higher GRP78 level in C.1 cells compared with the parental CHO cells, whereas the level of GRP94, also an ER-localized chaperone protein, and a 45-kDa unidentified protein (X) recognizable by the anti-KDEL antibody was relatively constant in both cell lines. In situ immunofluorescence imaging using anti-GRP78 antibody further revealed that in both CHO and C.1 cells, the majority of GRP78 was concentrated in the perinuclear region, consistent with its location in the ER (Figure 1, Panel B). The intensity of the immunofluorescent signal for GRP78 was greater in the majority of C.1 cells compared with CHO cells.

In examining the distribution of GRP78 and caspase-7 *in situ* using immunofluorescence, caspase-7 exhibits a perinuclear pattern indicative of ER localization (Figure 1, Panel C). Confocal microscopy further revealed caspase-7 is in close proximity with a subfraction of GRP78. The co-localization of GRP78 and caspase-7 was primarily detected in the perinuclear/ER region.

The physical interaction of endogenous caspase-7 with GRP78 was further confirmed using whole cell extracts prepared from CHO and C.1 cells. In agreement with the co-localization results obtained from confocal microscopy, procaspase-7 forms a complex with a high level of GRP78 in C.1 cells (Figure 6, Panel A, lane 2). For CHO cells, GRP78 was detected as a faint band in the anti-caspase-7 immunoprecipitate using an anti-KDEL antibody (Figure 6, Panel A, lane 1), and the signal for GRP78 was very enhanced when an anti-hamster GRP78 antibody was used for the Western blots (Figure 6, Panel B, lane 1). Using anti-caspase-3 as the immunoprecipitating antibody, GRP78 was not detected associated with procaspase-3 in Western blots (Figure 6, Panel A, lanes 3 and 4). Thus, endogenous GRP78 constitutively associates with procaspase-7. While the data provided herein indicates that GRP78 and caspase-7 interact, the invention is not limited to a direct interaction between the two proteins. It is understood that the invention encompasses a cytosolic component that is a complex of polypeptides, including caspase-7, or caspase-7 individually. By preventing the interaction of GRP78 with caspase, the agent modulates apoptosis by promoting apoptosis. Alternatively, by promoting the interaction of GRP78 with caspase-7, an agent would modulate apoptosis by inhibiting apoptosis.

As shown in Figure 7, Panel A, at low dose of trypsin digestion, a resistant carboxyl band of about 35kDa was detected. At the higher dose of trypsin, the intensity of the 35-kDa band became stronger, and a minor band of around 50-kDa was also visible. The digestion pattern for the CHO cells was the same, with the resistant bands more prominent for C.1 cells correlating with GRP78 overexpression. The trypsin treatment did not digest ER proteins localized inside the ER lumen as confirmed by the calreticulin control (Figure 7, Panel B).

In addition, sodium carbonate extraction of the microsome membrane fractions indicates that GRP78 is located in both the membrane and lumenal (Figure 7, Panel C). These results show that GRP78 is not exclusively an ER lumen protein, rather a subpopulation exist as a transmembrane protein. This is consistent with domains III and IV serving as putative transmembrane domains, with carboxyl fragments locating inside the lumen of the ER rendering them resistant to trypsin digestion (Figure 7, Panel A). This topology further indicates that part of GRP78 is exposed to the cytosol, allowing it to interact with cytosolic components. Thus, in another embodiment, the invention provides a method of modulating apoptosis by contacting glucose regulated protein 78 (GRP78) with an agent that inhibits or prevents the ability of the protein to integrate in to the membrane of the endoplasmic reticulum. In yet another embodiment, the invention provides a method of identifying an agent that modulates the interaction of glucose regulated protein 78 (GRP78) with a membrane by providing a polypeptide that includes hydrophobic transmembrane domain III (amino acids 210-260 of SEQ ID NO:1 or 2) and/or domain IV (amino acids 400-450 of SEQ ID NO:1 or 2) of the protein of glucose regulated protein 78. The polypeptide can be contacted with an agent, and the effect of the agent on the interaction can be determined. Agents that regulate the ability of the polypeptide to integrate in to the membrane are candidates for modulating apoptosis. For example, an agent that inhibits the ability of GRP78 to integrate in to the membrane will also be capable of promoting apoptosis because it will prevent GRP78 from interacting with cytosolic components that are required to promote apoptosis.

It was further determined that the ATP-binding domain of GRP78 is necessary for the interaction with cytosolic components. The ATP binding domain resides in the amino portion of GRP78 (Figure 6, Panel C). The invention further encompasses the use of fragments of GRP78 containing the ATP-binding domain in the methods of the invention. For example, a polypeptide that includes amino acids 125-275 of SEQ ID NO:1 or 2, or amino acids 150-250 of SEQ ID NO:1 or 2, or amino acids 175-201 of SEQ ID NO:1 or 2 can be used in a method to identify an agent that regulates the interaction of GRP78 and cytosolic components.

CHO cell line AD-1 that stably expresses a deleted form of GRP78 (Figure 6, Panel C) was used to determine that the ATP-binding region of GRP78 is necessary for binding to caspase. The deletion spans residues 175 to 201 within the ATP binding domain, resulting in defective ATPase activity. Western blot analysis of whole cell extracts prepared from CHO and AD-1 cells confirmed expression of the deleted GRP78 form, in addition to endogenous wild-type GRP78, in AD-1 cells (Fig. 6B, lanes 3 and 4).

Immunoprecipitation using anti-caspase-7 antibody showed that whereas procaspase-7 is able to form a complex with wild-type GRP78, deletion of residues 175 to 201 abolished its ability to bind to procaspase-7 (Figure 6, Panel B, lanes 1 and 2. Upon etoposide treatment, AD-1 cells showed more caspase-7 activation *in vivo* and more extensive DNA fragmentation compared with C.1 cells that overexpress the wild-type protein (Figure 8, Panels A and B). Annexin labeling and clonogenic survival assays performed with AD-1 cells further showed no protection against etoposide treatment compared with the parental CHO cells.

A transient transfection cell death assay further indicated that inhibition of apoptosis by GRP78 was dependent upon the GRP78-caspase interaction. Cell viability was measured quantitatively by the retention of β-galactosidase activity in the cells after drug treatment. As shown in Figure 8, cells transfected with the expression vector for wild-type GRP78 conferred protection against etoposide treatment. To confirm that the ATP binding function of GRP78 is required for the protective effect of GRP78, the cells were transfected with vectors expressing either wild-type GRP78 or mutant GRP78 (G227D), which carries an amino acid substitution at position 227, destroying its ATP binding ability. As shown in Figure 8, Panel C, the protective effect was lost with the mutant form of GRP78.

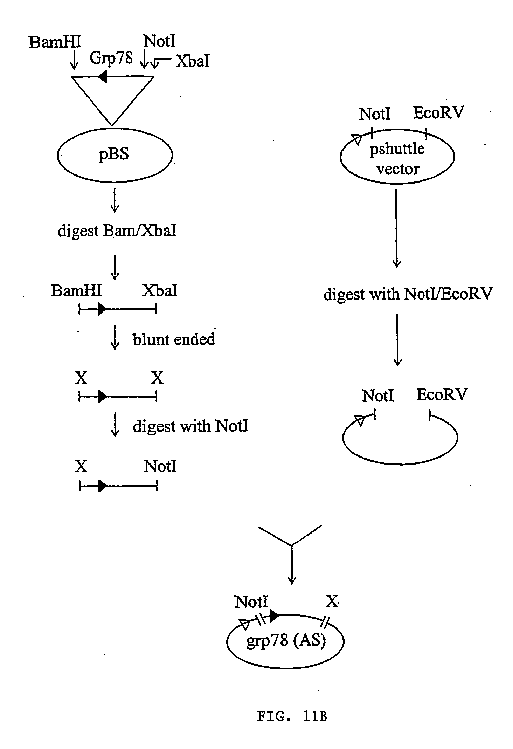

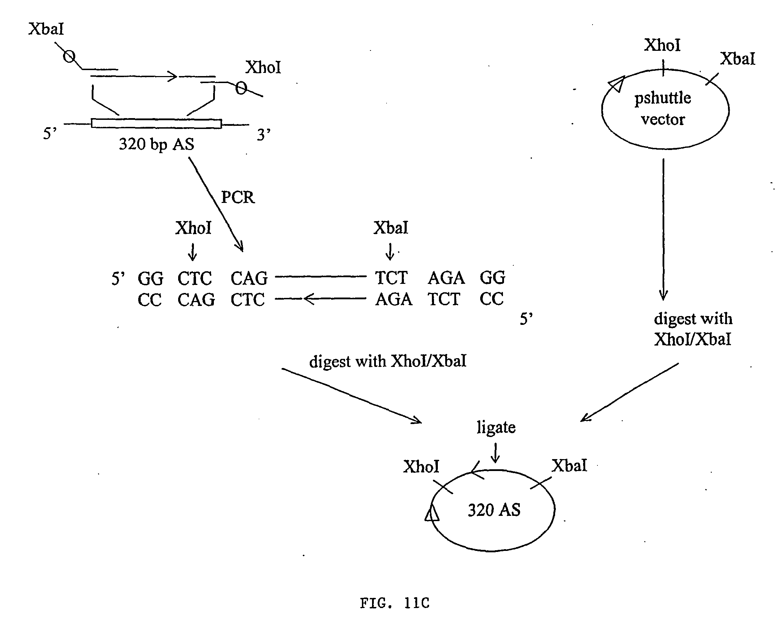

Experiments were performed to look at the effect of GRP78 on chemotherapeutics. A strong cellular promoter (CMV) was used to drive expression of GRP78 in the context of a well-characterized adenovirus vector called pShuttle-CMV. Both the sense and antisense orientation of GRP78 were constructed, to serve the function of overexpression or suppression of GRP78. Two versions of adenovirus with CMV promoter driving antisense (AS) Grp78 were constructed (see Figures 11A-C). The construction scheme for the full length AS construct is shown in Figure 11. An adenovirus construct expressing a partial length AS comprising a 320 bp fragment of the grp78 exon I was cloned in reverse orientation to the CMV promoter (see Fig. 11C). This shorter fragment targets the AUG start codon and may be more effective than the full length antisense molecule.

Overexpression of the His-tagged GRP78 in a human 293T tissue culture test system was performed. This cell line was used because it can be infected very efficiently with adenovirus. For this purpose, different doses of adenovirus expressing His-tagged GRP78 was infected into 293T cells. After 72 hr, the cell lysate was prepared and immunoblot was performed to detect the level of His-tagged GRP78, using antibody against the His-tagged which is specific for the adenovirus expressed protein. The results showed that His-tagged GRP78 was expressed in high levels in a dosage dependent manner (Figure 12, lanes 4, 5 and 6). This proves that the adenovirus construct for expressing full length GRP78 is successful. The next step is to repeat these experiments in MDA and MCF-7 cells. This step is more difficult because the cells are harder to culture and they are more difficult to infect.

As a proof of principle, the two antisense (AS) constructs were tested to determine their ability to suppress the His-tagged GRP78 in the human 293T tissue culture test system. For this purpose, different doses of adenovirus expressing either the full length AS or 320 bp AS was co-infected with adenovirus expressing His-tagged GRP78 into 293T cells. After 72 hr, the cell lysate was prepared and immunoblot was performed to detect the level of His-tagged GRP78, using antibody against the His-tagged which is specific for the adenovirus expressed protein. The results showed that both the full length AS (Figure 12, lanes 7, 8 and 9) and the 320 bp AS (Figure 12, lanes 1, 2 and 3) were able to suppress expression of the His-tagged GRP78 in a dosage dependent manner. This proves that the adenovirus constructs for expressing antisense GRP78 are successful. The most drastic reduction was obtained with the 320 bp AS (Figure 12, lane 1).

Next human breast cancer MDA-MB-435 cells were infected with the adenovirus expressing the 320 bp AS, in the presence or absence of treatment with the chemotherapy drug etoposide. The same cells were infected with the GFP negative control adenovirus. The results showed that in the mock-infected cells, etoposide treatment by itself reduced the amount of endogenous GRP78 by about 40% (Figure 13, lanes 1 and 2). Importantly, the 320 bp AS construct further reduced GRP78 level significantly, particularly in cells treated with etoposide, such that the final level was less than 10% (Figure 13, lanes 3 and 4). Thus, both the full length and the 320 bp version of the AS construct targeted against GRP78 blocked expression of GRP78 in a 293T test system. This was repeated in human breast cancer cells and showed that the AS adenovirus is able to suppress endogenous GRP78 expression.

Experiments were also performed to determine the anti-apoptotic effect conferred by overexpression of GRP78. This task was completed. GRP78 overexpression confers resistance to all four drugs (cisplatin, doxorubicin, etoposide and camptothecin). The results are summarized in Table 2.

**Table 2**

| | % Survival (Colony Assay) | |
|---|---|---|
| | Control | GRP78 Overexpression |
| Cisplatin (M) | | |
| 0 | 100 | 100 |
| 3.3 | 43 | 60 |
| 6.6 | 3.7 | 50 |
| 9.9 | 0 | 27 |
| 13.0 | 0 | 16 |
| Doxorubicin (µg/ml) | | |
| 0 | 1 | 100 |
| 0.2 | 80 | 95 |
| 0.4 | 42 | 84 |
| 0.6 | 2 | 68 |
| 0.8 | 0 | 48 |
| 1.0 | 0 | 32 |
| Etoposide (µM) | | |
| 0 | 100 | 100 |
| 0.2 | 71 | 93 |
| 0.4 | 67 | 93 |
| 0.8 | 26 | 77 |
| 1.6 | 1.5 | 63 |
| camptothecin (ng/ml) | | |
| 0 | 100 | 100 |
| 5 | 68 | 82 |
| 20 | 0.7 | 22 |
| 60 | 0 | 2 |

The antisense approach described above was replaced with an siRNA approach. The sequence of the Grp78 siRNA included:
5' AAGGTTACCCATGCAGTTGTT 3' (SEQ ID NO:4)
3' TTCCAATGGGTACGTCAACAA 5' (SEQ ID NO:17)
When blasted against the human genomic sequence, this sequence is unique and in principle, should not affect any other human gene. To prove this, experiments were performed to test the effect of this siRNA on the expression of GRP78 and related stress proteins GRP94 and HSP70 in human 293T cells. The level of β-actin was used as loading control. As shown in Figure 14, only GRP78 level is suppressed, confirming that the siRNA is specific for GRP78.

Experiments further showed that Grp78(II) siRNA at 80 nM or higher can significantly suppress GRP78 level in human breast cancer MDA-MB-435 cells (Figure 15). This translates to more death in the cells treated with Grp78(II)siRNA than control siRNA targeted against the unrelated green fluorescence protein (Figure 16).

One candidate target of GRP78 action is BIK, the BH3-only protein inducible in response to DNA damage that is located in the ER as well as the mitochondria. Remarkably, ER-targeted BIK can induce cytochrome c release, suggesting it can act at the ER site to initiate a parallel cell death pathway (Germain *et al*., 2002). To test whether GRP78, as a molecular chaperone, can either directly or indirectly block the action of pro-apoptotic molecules that control the release of cytochrome c from the mitochondria the following experiment was performed. Using 293T as a model system, BIK level was observed to increase following etoposide treatment and co-IP of GRP78 with endogenous BIK (Figure 17A). These results suggesting that GRP78 overexpression can protect cells from cell death mediated by ER-targeted BIK (Figure 17B). These findings imply that in cells treated with etoposide, there are both physical and functional interactions between BIK and GPP78. Thus, this can contribute in part to the protective effect of GRP78 towards etoposide-induced cell death.

Cell Culture Conditions - The CHO cells were maintained in α-minimum Eagle's medium with nucleosides supplemented with 10% dialyzed fetal calf serum and 1% penicillin/streptomycin/neomycin antibiotics. The C.1 and AD-1 cells were maintained in the above conditions in the presence of 0.1 µg/ml methotrexate but without added nucleosides. The establishment of stable T24/83 human transitional bladder carcinoma cell lines overexpressing human GRP78 or transfected with the empty expression vector (pcDNA3.1) has been described. The T24/83 cell lines were maintained in M199 medium supplemented with 10% fetal calf serum containing 1% penicillin/streptomycin/neomycin antibiotics and 200 µg/ml G418. The human acute T cell leukemia Jurkat cells were maintained in RPMI 1640 medium supplemented with 10% fetal calf serum containing 1% penicillin/streptomycin/neomycin antibiotics. All the cells were maintained at 37°C in a humidified atmosphere of 5% CO₂/95% air.

Reagents - Etoposide (Calbiochem) was dissolved in Me₂SO at a concentration of 30 mM and stored at -20 °C. Methotrexate (Sigma) was dissolved in a minimum amount of 1 M NaOH, diluted with water to 1µg/ml, and stored at -20°C. Doxorubicin (Bedford Laboratories, Bedford, OH) at 2 mg/ml and camptothecin (Amersham Biosciences) at 20mg/ml were supplied as isotonic solutions.

Cell Cycle Analysis - Following seeding, exponentially growing cells were trypsinized at different days and fixed in 70% ethanol. The fixed cells were treated with PBS containing 0.1% (v/v) Triton X-100, 0.2 mg/ml DNase-free RNase, and 20 µg/ml propidium iodide (PI) for 30 min at room temperature. The cell cycle distributions were analyzed by fluorescence-activated cell sorting (FACS) analysis (FACstar; BD Biosciences). The cell cycle distribution measurements were repeated three to four times.

Clonogenic Survival Assays - Four thousand cells were seeded into 10-cm-diameter dishes. Two days after seeding, cells were treated with etoposide for 6 h, doxorubicin for 1 h, or camptothecin for 24 h at different concentrations as indicated. After drug treatment, the cells were grown in fresh medium for 10 to 14 days. The colonies were washed with ice-cold PBS, fixed with methanol, and stained with 10% Giemsa staining solution. The surviving fraction was determined by dividing the number of the surviving colonies in the drug-treated cells by the number of colonies in the non-treated control groups.

Annexin V Staining and FACS Analysis - CHO, C.1, and T24/83 cells were trypsinized, washed twice with ice-cold PBS, pH 7.4, and resuspended in 1µ binding buffer (10 mM HEPES, pH 7.4, 140 mM NaCl, 2.5 mM CaCl₂ at a concentration of 1 x 10⁶ cells/ml. One hundred µl of cell suspension was transferred to 5-ml plastic tubes, and 5 µl of annexin V-fluorescein isothiocyanate (PharMingen) and 4 µl of 0.5 mg/ml PI were added. The cells were gently vortexed and incubated in the dark at room temperature for 20 min. Four hundred µl of binding buffer was added to each tube, and annexin V staining was analyzed by flow cytometry within 1 h. Cells negative for both PI and annexin V staining are live cells, annexin V positive staining cells are early apoptotic cells, and PI positive and annexin V positive staining cells are primarily cells in late stages of apoptosis.

Caspase-7 Activation Assays - The cells were either non-treated or treated with 100 µM etoposide for 6 h and harvested after 24 h. The cells were suspended in 5 volumes of a hypotonic buffer (5 mM Tris-HCl, pH 7.4, 5 mM KCl, 1.5 mM MgC12, 0.1 mM EGTA, pH 8.0, and 1 mM dithiothreitol) in the presence of 2 µg/ml leupeptin, pepstatin, and aprotinin protease inhibitors. After incubation on ice for 20 min, sucrose was added to a final concentration of 250 mM, and the cells were disrupted by douncing eight times in a 1-ml Wheaton Dounce homogenizer. The homogenate was centrifuged twice at 750 µ g for 10 min. The supernatant was clarified again at 16,000 x g for 15 min at 4 °C and designated as the cytoplasmic fraction. For *in vitro* caspase-7 activation assay, 150 µg of cell-free extract was incubated with various amounts of cytochrome c and dATP at 37 °C for 1 h. Equal amounts of total proteins were separated, and Western blotting was performed for caspase-7.

Western Blotting - The cell lysate was prepared in radioimmune precipitation assay buffer and subjected to immunoblot with antibodies against GRP78, GRP94, topoisomerase II, caspase-7, and α-actin as described. Nitrocellulose membranes containing the transferred proteins were blocked in Tris-buffered saline containing 5% non-fat dry milk and 0.1% Tween 20 for 1 h at room temperature and were probed with the respective primary antibodies. For GRP78, an anti-KDEL mouse monoclonal antibody (SPA-827), an anti-GRP78 rabbit polyclonal antibody directed against the carboxyl ten amino acids of rat GRP78 (SPA-826) (StressGen, Victoria, Canada), or an anti-hamster GRP78 rabbit polyclonal antibody at 1:3000, 1:2000, and 1:5000 dilution, respectively, was used. Dilutions for the other primary antibodies were as follows: anti-calnexin rabbit polyclonal antibody (SPA-865) (StressGen) at 1:2000, anti-calreticulin rabbit polyclonal antibody (SPA-600) (StressGen) at 1:3000, anti-β-actin mouse monoclonal antibody (Sigma) at 1:5000, anti-caspase-7 mouse monoclonal antibody (10-1-62) (BD Biosciences) at 1:1000, anti-caspase-3 rabbit polyclonal antibody (Cell Signaling, Beverly, MA) at 1:1000, and anti-topoisomerase II mouse monoclonal antibody (SWT3D1) (Oncogene, San Diego, CA) at 1:1000. Respective horseradish peroxidase-conjugated secondary antibodies were used, and the protein bands were visualized by the ECL method (Amersham Biosciences).

Transient Transfection Death Assay - Briefly, Jurkat cells were transiently transfected with either CMV-neo-Bcl2 or expression vectors for wild-type hamster GRP78 or a GRP78 ATP-binding site mutant G227D. After drug treatment, cell lysates were prepared and assayed for β-galactosidase activity remaining in the surviving cells. The percent cytotoxicity was calculated as described previously.

DNA Fragmentation Assays - The cells were either non-treated or treated with 100 µM etoposide for 12 h and harvested after 48 h. The DNA fragmentation assays were performed using an apoptosis DNA ladder kit (Roche Molecular Biochemicals) according to manufacturer's instructions.

Immunofluorescence Staining and Image Analysis - CHO and C.1 cells were grown to 60% confluence in chamber slides (Nalge Nunc International, Naperville, IL), washed twice with PBS, and fixed with 4% paraformaldehyde in PBS for 10 min. The cells were then washed with PBS and permeabilized in PBS containing 0.1% Triton X-100 and 5% bovine serum albumin for 30 min. For detection of GRP78, the cells were stained with a 1:1000 dilution of anti-GRP78 (C-20) goat polyclonal antibody (Santa Cruz Biotechnology, Inc., Santa Cruz, CA) and a 1:500 dilution of anti-goat Texas red-conjugated secondary antibody
(Vector Labs, Burlingame, CA). For detection of caspase-7, the cells were stained with anti-caspase-7 mouse monoclonal antibody (BD Biosciences) at a 1:500 dilution and a 1:500 dilution of anti-mouse fluorescein isothiocyanate-conjugated secondary antibody (Vector Labs). Cells were mounted in Vectashield with DAPI mounting medium (Vector Labs) and visualized on a Zeiss LSM 510 dual-photon confocal microscope. The T24/83 cells were incubated with the same anti-GRP78 polyclonal antibody as described for the CHO cells. Whole cell images were subsequently captured and analyzed using Northern exposure image analysis/archival software (Mississauga, Ontario, Canada).

Co-immunoprecipitation Assays - 2 x 10⁶ cells were lysed in 400 µl of extraction buffer (50 mM Tris-HCl, pH 7.5, 150 mM NaCl, 0.5% Nonidet P-40, and 0.5% deoxycholate, with protease inhibitor tablet (Roche Molecular Biochemicals)) and frozen and thawed three times. 500 µg of total protein extract from each sample was pretreated with 50 µl of protein A-Sepharose beads (Sigma) for 1 h at 4 °C prior to incubation with 5 µg of either anti-caspase-7 mouse monoclonal antibody or anticaspase-3 antibodies for 2 h. Following the incubation period, 50 µl of protein A-Sepharose beads was added, and the mixtures were rotated at 4 °C overnight. The beads were then washed five times with the extraction buffer. The immunoprecipitate was released from the washed beads by the addition of 30 µl of 1x SDS-PAGE sample loading buffer (50 mM Tris-HCl, pH 6.8, 100 mM dithiothreitol, 2% SDS, 0.1% bromphenol blue, 10% glycerol), followed by heating at 100 °C for 10 min. The supernatant obtained after centrifugation was resolved by SDS-PAGE and subjected to Western blot analysis to detect the co-immunoprecipitated proteins.

Isolation of Microsomes and Protease Digestion - The cells were trypsinized, and after washing with cold PBS, were lysed by incubation in 10 volumes of cold hypotonic buffer (10 mM Tris-HCl, pH 7.4), followed by Dounce homogenization. The lysate was immediately adjusted to 0.25 M sucrose, 1 mM MgCl2 and centrifuged at 1000 x g for 10 min at 4 °C to remove nuclei and cell debris. The supernatant was further centrifuged at 100,000 x g for 90 min. The pellet, representing microsomes, was rinsed briefly with cold water and resuspended in 50 mM Tris-HCl, pH 7.4, and used for proteolytic digestion and sodium-carbonated extraction studies.

Sodium Carbonate Extraction-For separation of ER membranes from lumenal proteins, the microsome pellet was resuspended in 50 volumes of 100 mM sodium carbonate, pH 11.5, and incubated on ice for 1 h. The suspension was then centrifuged for 1 h at 240,000 x g at 4 °C. The pellet, which represents ER membrane, was rinsed with cold water and resuspended in 1x SDS-PAGE sample loading buffer and analyzed by Western blot. Proteins present in the ER lumen were recovered from the supernatant by the addition of trichloroacetic acid to a final concentration of 10%. The pellet was washed three times with acetone, air-dried, solubilized in the 1x SDS-PAGE sample loading buffer, and analyzed by Western blot.

Limited Tryptic Digestion of Microsomal Proteins - For trypsin digestion reactions, the microsomes were incubated with trypsin (0.01% or 0.05%) for 30 min at room temperature. The proteolytic cleavage reactions were terminated by the addition of 1x SDS-PAGE sample loading buffer and boiling at 100 °C for 5 min. 10-20 µg of total protein from each reaction was analyzed by Western blot.

To examine directly whether specific overexpression of GRP78 can lead to the development of drug resistance, CHO and C.1 cells were exposed to various drugs, and cell survival was measured using clonogenic survival assays. Various dosages of etoposide (also referred to as VP16), adriamycin (also referred to as doxorubicin), and camptothecin were tested. Both etoposide and adriamycin are inhibitors of topoisomerase II, and camptothecin is a topoisomerase I inhibitor. The results for each of the drugs tested are shown in Figure 2. With all three drugs, C.1 cells overexpressing GRP78 conferred higher resistance than CHO cells. These data establish that specific overexpression of GRP78, in the absence of the UPR, is sufficient to render CHO cells more resistant to topoisomerase I and II inhibitors.

To determine whether GRP78 protects the cells from etoposide-induced apoptosis, CHO and C.1 cells were either nontreated or treated with etoposide and labeled with annexin V and PI. The apoptotic cells were identified by annexin V labeling. For CHO cells, the percentage of apoptotic cells increased 10-fold (from 9 to 90%) upon etoposide treatment; for C.1 cells, the increase was 4.7-fold (from 15 to 70%) (Fig. 3A). More extensive DNA fragmentation was also detected in etoposide-treated CHO but not C.1 cells (Figure, Panel B).

A pair of stably transfected human transitional bladder carcinoma T24/83 cell lines selected and cultured under identical conditions, were used to determine the effect of GRP overexpression on neoplastic cells. The cell line, referred to as T24/83-GRP78, overexpressed human GRP78, and the other line, referred to as T24/83-pcDNA, was stably transfected with the empty expression vector pcDNA (28). Immunoblot analysis followed by normalization against α-actin revealed a 3-fold increase in the level of GRP78 expression in the T24/83-GRP78 cells as compared with T24/83-pcDNA cells (Fig. 4A, inset). Overexpression of GRP78 in T24/83-GRP78 cells did not affect the expression level of ER chaperone proteins GRP94, protein disulfide isomerase and calreticulin, or heat shock protein HSP47 (Figure 4, Panel A). Whole cell imaging revealed much greater GRP78 immunofluorescence for the T24/83-GRP78 cells, confirming the results of the immunoblots (Figure 4, Panel B).

In agreement with the CHO cell lines, T24/83 cells overexpressing GRP78 exhibited more resistance to etoposide in clonogenic survival assays (Figure, 4, Panel A). Similar protection was observed for adriamycin and camptothecin. For T24/83-cDNA cells, etoposide treatment increased the percentage of annexin V-labeled cells 2.7-fold (from 7 to 19%), as compared with an increase of 1.4-fold (from 8 and 11%) for T24/83-GRP78 cells (Fig. 4C).

With the availability of the GRP78 overexpressing cell lines, the effect of GRP78 overexpression on topoisomerase II level in the absence of an UPR was determined. CHO and C.1 cells were either non-treated or treated with etoposide, and the level of topoisomerase II was determined by immunoblotting (Figure 5, Panel A). The data indicate that specific GRP78 overexpression has no effect on the topoisomerase II protein level.

Analysis of the cell cycle distribution of exponentially growing cells showed CHO and C.1 cells with similar G1, S, and G2 distribution profiles (Figure 9). In contrast, CHO cells treated with tunicamycin or thapsigargin, both standard UPR inducers, showed more cells in G1 and a dramatic reduction in S phase cells. A similar pattern was observed for exponentially growing T24/83 cells. In both the vector-transfected and GRP78 overexpressing cells, the percentage of G1, S, and G2 cells is similar. Cells treated with tunicamycin or thapsigargin showed a higher percentage of G1 cells and a lower percentage in S phase (Figure 9). Collectively, these results show that in contrast to the UPR, specific overexpression of GRP78 does not alter the cell cycle distribution.

A number of embodiments of the invention have been described.

## Claims

1. A method of modulating apoptosis in a cell in vitro, the method comprising contacting a glucose regulated protein 78(GRP78) with a peptide which inhibits the interaction of the GRP78 with a procaspase-7 that mediates apoptosis, wherein the peptide is a soluble domain of GRP78 and interacts with the ATP-binding domain of the GRP78 protein.

2. The method of claim 1, wherein the GRP is located in the endoplasmic reticulum.

3. The method of claim 1, wherein the modulating is by promoting apoptosis.

4. The method of claim 1, wherein the ATP-binding domain comprises amino acids 125-275 of SEQ ID NO:2.

5. The method of claim 5, wherein the peptide interacts with amino acids 150-250 of SEQ ID NO:2, and/or amino acids 175-201 of SEQ ID NO:2.

6. A method according to claim 1, wherein the modulating is promoting apoptosis and the cell is a neoplastic cell; or the method of claim 3 wherein the cell is a neoplastic cell.

## Patentansprüche

1. Verfahren zum Modulieren der Apoptose in einer Zelle in vitro, wobei das Verfahren das Inkontaktbringen eines Glucose-regulierten Proteins 78 (GRP78) mit einem Peptid umfasst, das die Interaktion des GRP78 mit einer Procaspase-7 inhibiert, welche die Apoptose vermittelt, worin das Peptid eine lösliche Domäne von GRP78 ist und mit der ATPbindenden Domäne des GRP78-Proteins interagiert.

2. Verfahren nach Anspruch 1, worin sich das GRP im endoplasmatischen Retikulum befindet.

3. Verfahren nach Anspruch 1, worin die Modulation durch das Fördern der Apoptose erfolgt.

4. Verfahren nach Anspruch 1, worin die ATP-bindende Domäne die Aminosäuren 125-275 von SEQ ID NO:2 umfasst.

5. Verfahren nach Anspruch 5, worin das Peptid mit den Aminosäuren 150-250 von SEQ ID NO:2 und/oder den Aminosäuren 175-201 von SEQ ID NO:2 interagiert.

6. Verfahren nach Anspruch 1, worin das Modulieren das Fördern der Apoptose beinhaltet und die Zelle eine neoplastische Zelle ist, oder das Verfahren nach Anspruch 3, worin die Zelle eine neoplastische Zelle ist.

## Revendications

1. Méthode *in vitro* de modulation de l'apoptose dans une cellule, ladite méthode consistant à faire entrer en contact une protéine 78 régulée par le glucose (GRP78) avec un peptide qui inhibe l'interaction entre la GRP78 et une procaspase 7 qui assure la médiation pour l'apoptose, dans laquelle le peptide est un domaine soluble de la GRP78 qui interagit avec le domaine de liaison de l'ATP de la protéine GRP78.

2. Méthode selon la revendication 1, dans laquelle la GRP se situe dans le réticulum endoplasmique.

3. Méthode selon la revendication 1, dans laquelle la modulation facilite l'apoptose.

4. Méthode selon la revendication 1, dans laquelle le domaine de liaison de l'ATP comprend les acides aminés 125 -275 de la SÉQ ID No. 2.

5. Méthode selon la revendication 5, dans laquelle le peptide interagit avec les acides aminés 150-250 de la SÉQ ID No. 2, et/ou les acides aminés 175-201 de la SÉQ ID No. 2.

6. Méthode selon la revendication 1, dans laquelle la modulation facilite l'apoptose et la cellule est une cellule néoplasique ; ou la méthode selon la revendication 3 dans laquelle la cellule est une cellule néoplasique.
